# EUROPEAN PATENT APPLICATION

(11) **EP 2 123 651 A1**
(43) Date of publication of application: **25.11.2009**
(21) Application number: 08703164.7
(22) Date of filing: 11.01.2008
(51) Int. Cl.: C07D 471/04, A61K 31/437, A61K 31/4545, A61K 31/497, A61K 31/501, A61K 31/506, A61K 31/55, A61P 29/00, A61P 37/00, A61P 37/02, A61P 43/00, C07D 471/14

(54) **CONDENSED PYRIDINE COMPOUND**

(30) Priority: 12.01.2007 JP 2007005236
(71) Applicant: Astellas Pharma Inc., Tokyo 103-8411 (JP)
(72) Inventor: SHIRAKAMI, Shohei, Tokyo 103-8411 (JP); INOUE, Takayuki, Tokyo 103-8411 (JP); MUKOYOSHI, Koichiro, Tokyo 103-8411 (JP); NAKAJIMA, Yutaka, Tokyo 103-8411 (JP); USUDA, Hiroyuki, Tokyo 103-8411 (JP); HAMAGUCHI, Hisao, Tokyo 103-8411 (JP); HIGASHI, Yasuyuki, Tokyo 103-8411 (JP); HATANAKA, Keiko, Tokyo 103-8411 (JP)
(74) Representative: Gille, Christian
(86) International application number: PCT/JP2008/050300
(87) International publication number: WO 2008/084861

(57) **Abstract**

The present invention provides a compound having excellent JAK3 inhibitory activity and being useful as an active ingredient of an agent for treating and/or preventing various immune diseases including autoimmune diseases, inflammatory diseases, and allergic diseases.

As a result of investigations with respect to novel condensed heterocyclic derivatives, the inventors have verified that a condensed pyridine compound has excellent JAK3 inhibitory activity, thereby completing the present invention.

More specifically, it has been verified that since the compound according to the present invention has inhibitory activity against JAK3, the compound is useful as an active ingredient of an agent for treating or preventing diseases caused by undesirable cytokine signal transduction (e.g., rejection during live organ/tissue transplantation, autoimmune diseases, asthma, atopic dermatitis, rheumatism, psoriasis and atherosclerotic disease), or diseases caused by abnormal cytokine signal transduction (e.g., cancer and leukemia).

## Description

### TECHNICAL FIELD

The present invention relates to a novel condensed pyridine compound, to a medicament containing the compound as an active ingredient and to diseases caused by undesiable and/or abnormal cytokine signal transduction.

### BACKGROUND ART

Janus kinase 3 (hereafter referred to as JAK3) is a Janus family of protein kinases. Although kinases in this family, other than JAK3, are expressed in a wide range of tissues, JAK3 is expressed locally in hematopoietic cells. This is not contradict the fact that JAK3 plays an important role in signal transduction via various receptors, such as interleukin (hereafter referred to as IL) -2, IL-4, IL-7, IL-9, IL-15, and IL-21 by noncovalent association with the common gamma chain (refer to nonpatent literature 1 and 2).

XSCID (X-linked severe combined immunodeficiency) patient populations have been identified with reduced levels of JAK3 protein or with genetic defects to gamma chain, suggesting that immunosupression should result from blocking signaling through the JAK3 pathway (refer to nonpatent literature 3 and 4). Animal experiments have suggested that JAK3 not only plays an important role in maturation of B- and T-lymphocytes but also in maintaining the function of T-cells. Hence, it is expected that diseases involving proliferative abnormality of T-cells, such as rejection during organ/tissue transplantation and autoimmune diseases, can be treated by controlling immune response through this mechanism.

On the other hand, a pyrrolopyridine derivative (patent literature 1) represented by formula (A) or (B) or an imidazopyridine derivative (refer to patent literature 2) is known as a compound having JAK3 inhibition activity. 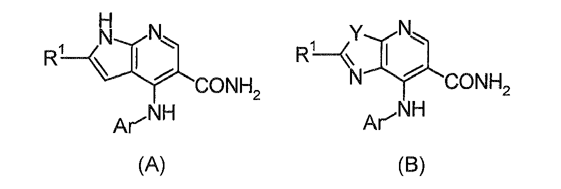 (For the symbols in the formulas, refer to the corresponding patent publications.)

Furthermore, a pyrrolopyrimidine derivative (refer to patent literature 3, 4, 5 and 6) represented by formula (C) is also known as a compound having JAK3 inhibition activity. 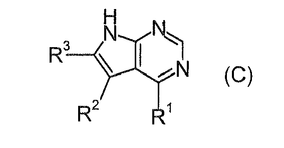 (For the symbols in the formula, refer to the corresponding patent publications.)

Moreover, a pyrrolopyridine derivativerepresented by formula (D) is also known as a compound having JAK3 inhibition activity (refer to patent literature 7). 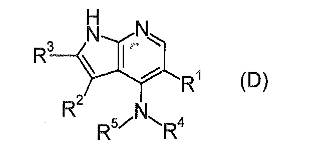 (For the symbols in the formula, refer to the corresponding patent publications.)

A pyrrolopyridine derivative represented by formula (E) is also known as a compound having JAK3 inhibition activity (refer to patent literature 8). 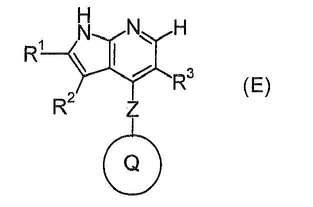 (For the symbols in the formula, refer to the corresponding patent publications.)

A condensed pyridine derivative represented by formula (F) having JAK3 inhibition activity is disclosed in patent literature 9, which was published after priority date of present application. 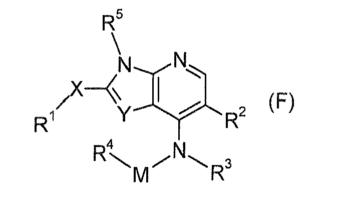 (For the symbols in the formula, refer to the corresponding patent publications.)

However, in any literature, the compound according to the present invention is not disclosed specifically.
[Nonpatent literature 1] J. J. O'shea et al., Cell, 2002, Vol . 109 (suppl.), S121,
[Nonpatent literature 2] K. Ozaki et al., Science, 2002, Vol. 298, p. 1630,
[Nonpatent literature 3] P. Macchi et al., Nature, 1995, Vol. 377, p. 65,
[Nonpatent literature 4] S. M. Russell et al., Science, 1995, Vol. 270, p. 797,
[Patent literature 1] WO 2004/099205,
[Patent literature 2] WO 2004/099204,
[Patent literature 3] WO 99/65908,
[Patent literature 4] WO 99/65909,
[Patent literature 5] WO 01/42246,
[Patent literature 6] WO 02/00661,
[Patent literature 7] WO 2006/069080,
[Patent literature 8] WO 2006/127587,
[Patent literature 9] WO 2007/007919.

### DISCLOSURE OF THE INVENTION

### (Problem to be Solved by the Invention)

As a result of intensive studies with an object of providing a useful pharmaceutical composition having JAK3 inhibition activity, the inventors have found that a novel condensed pyridine compound has an excellent JAK3 inhibition activity, thereby completed the present invention.

### (Means for solving problem)

More specifically, the present invention provides a novel condensed pyridine compound represented by the following formula (I) or pharmaceutically acceptable salts thereof, and a pharmaceutical composition containing the compound, a pharmaceutical composition serving as an agent for treating and/or preventing diseases caused by undesiable and/or abnormal cytokine signal transduction, and more particularly, autoimmune diseases, inflammatory diseases, and allergic diseases.

The condensed pyridine compound is a compound represented by the following formula (I): 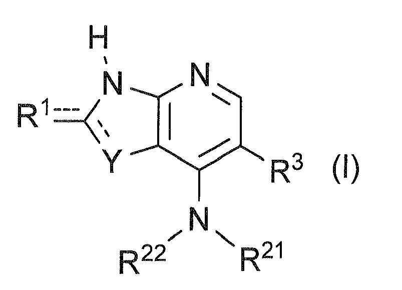 wherein
- R¹: is -H or =O;
- R³: is carbamoyl or oxadiazolyl, each of which may be substituted with C₁-C₆ alkyl;
- R²¹: is -H or may be bonded with R³ via a certain functional group to form divalent groups represented by the following formulas:

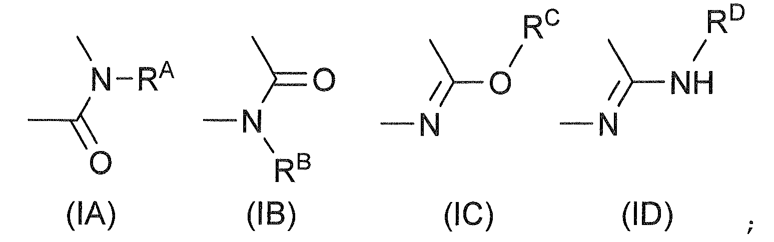

R^{A} is -H or C₁-C₆ alkyl;
R^{B} is -H or C₁-C₆ alkyl;
R^{C} is -H, C₁-C₆ alkyl or - (C₁-C₆ alkyl)-O-(C₁-C₆ alkyl);
R^{D} is -H, -C(=O)-(C₁-C₆ alkyl), -C(=O)-(C₁-C₆ alkyl)-O-(C₁-C₆ alkyl), -C (=O) C (=O) NH- (C₁-C₆ alkyl), -C(=O)C(=O)NH-(C₁-C₆ alkyl)-O-(C₁-C₆ alkyl) or -C(=O)O-(alkyl) ;
R²² is 5- to 7-membered nitrogen-containing heterocycloalkyl, C₃-C₉ cycloalkyl, benzopyranyl or benzyl,
each of which may be substituted with one or more identical or different group (s) selected from the group consisting of R^{V};
R^{V} is halogen, C₁-C₆ alkyl, -O-(C₁-C₆ alkyl), aryl, 5- to 6- membered nitrogen-containing heteroaryl, benzyl, carbamoyl, -(C=O)-(C₁-C₆ alkyl), -C(=O)-(5- to 6-membered heteroaryl), -C(=O)-(5- to 6-membered nitrogen-containing heterocycloalkyl), -C(=O)-carbamoyl, -C(=O)C(=O)-(5- to 6- membered nitrogen-containing heterocycloalkyl), -C(=O)O-(C₁-C₆ alkyl),
each of which may be substituted with one or more identical or different group (s) selected from the group consisting of R^{W};
R^{W} is halogen, -CN, -CF₃, -OH, =O, -NO₂, carbamoyl, oxime, C₁-C₆ alkyl, - (C₁-C₆ alkyl)-CN, -(C₁-C₆ alkyl)-OH, -C(=O)O-(C₁-C₆ alkyl), -O-(C₁-C₆ alkyl), -O- (C₁-C₆ alkyl)-CN, 2-pyrroridinone-1-yl or phenyl, wherein phenyl may be substituted with halogen;
Y is N, CH or CH₂; and
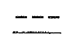 is a single bond or a double bond, wherein one is single bond, the other is double bond; or pharmaceutically acceptable salts thereof, or prodrug thereof.

The same shall be applied hereinafter.

### (Effect of the Invention)

The compound according to the present invention has JAK3 inhibition activity and is thus useful as an active ingredient of an agent for treating or preventing diseases caused by undesirable cytokine signal transduction (e.g., rejection during live organ/tissue transplantation, autoimmune diseases, asthma, atopic dermatitis, atherosclerotic disease, psoriasis, and rheumatism), or diseases caused by abnormal cytokine signal transduction (e.g., cancer and leukemia).

### (Best modes for carrying out the invention)

The present invention will be explained in more detail herein below.

The term "C₁-C₆ alkyl" in the specification is a C₁-C₆ straight or branched alkyl and may include (C₃-C₄)cycloalkyl-(C₁-C₂)alkyl, such as methyl, ethyl, n-propyl, isopropyl, n-butyl, isobutyl, t-butyl, n-pentyl, neopentyl, and n-hexyl, preferably methyl, ethyl, n-propyl, isopropyl, cyclopropylmethyl, isobutyl and cyclopropylmethyl, cyclopropylethyl, cyclobytylmethyl, and particularly preferably methyl and ethyl.

The term "alkyl" in the specification is a C₁-C₁₂ straight or branched alkyl, that is, an alkyl having 7 to 12 carbon atoms in addition to the C₁-C₆ alkyl described above.

The term "halogen" may include fluoro, chloro, bromo, and iodo, preferably fluoro, chloro, and bromo.

The term "C₃-C₉ cycloalkyl is a C₃-C₉ monovalent nonaromatic and nonbridged carbocyclic group, and may partially have unsaturated bonds. However, bridged cyclic hydrocarbons are excluded. Cycloalkyl may include, such as cyclopropyl, cyclobutyl, cyclohexyl, cycloheptyl, cyclooctyl, cyclobutenyl, cyclohexenyl, cyclooctadienyl and the like.

The term "5- to 7-membered nitrogen-containing heterocycloalkyl " is a monovalent group of a five- to seven-membered nonaromatic ring which may contain one or more nitrogen atom(s) and other hetero atom(s), and may include, such as pyrrolidinyl, piperazinyl, piperidinyl, homopiperidinyl, morpholinyl, thiomorpholinyl, imidazolidinyl, and pyrazolidinyl.

The term "aryl" is a monovalent group of an aromatic ring having carbon atoms and may include, such as phenyl and naphthyl.

The term "5- to 6- membered heteroaryl" is a monovalent group of a 5- to 6-membered aromatic heterocycle having one or more identical or different hetero atom(s) selected from the group consisting of nitrogen, oxygen and sulfur atoms. The "5-to 6- membered heteroaryl" may include, such as pyridyl, pyrazinyl, pyrimidynyl, pyridazinyl, pyrrolyl, pyrazolyl, imidazolyl, oxazolyl, thiazolyl, thienyl, furyl, oxadiazolyl and thiadiazolyl and the like.

The term "5- to 6- membered nitrogen-containing heteroaryl" is "5- to 6- membered heteroaryl" having at least one nitrogen atom as ring atom. "5- to 6- membered nitrogen-containing heteroaryl" may include, such as pyridyl, pyrazinyl, pyrimidynyl, pyridazinyl, pyrrolyl, pyrazolyl, imidazolyl, oxazolyl, thiazolyl, oxadiazolyl and thiadiazolyl and the like. One embodiement of suitable group in formula (I) is exemplified as follows:
- R¹ :: -H is preferred.
- R³:: Carbamoyl which may be substituted with C₁-C₆ alkyl is preferred.
- R²¹ :: -H, (IA) or (ID) are preferred.
- R^{A} :: -H is preferred.
- R^{B} :: -H is preferred.
- R^{C} :: -H is preferred.
- R^{D} :: -H or -C(=O)C(=O)NH-(C₂-C₆ alkyl) or -C(=O)C(=O)NH-(C₁-C₆ alkyl)-O-(C₁-C₆ alkyl) are preferred.
- R²² :: 5- to 7-membered nitrogen-containing heterocycloalkyl,
which may be substituted with one or more identical or different group(s) selected from the group consisting of R^{V},
are preferred.
- R^{V} :: Halogen, C₁-C₆ alkyl, -O-(C₁-C₆ alkyl), aryl, 5- to 6- membered nitrogen-containing heteroaryl and -C(=O)O-(C₁-C₆alkyl) are preferred.
- R^{W}:: Halogen, -CN, -CF₃, -OH, =O, -NO₂, carbamoyl, oxime, -(C₁-C₆ alkyl) -OH, -C (=O) O-(C₁-C₆ alkyl), -O- (C₁-C₆ alkyl) or -O-(C₁-C₆ alkyl) -CN are preferred.
- Y:: N and CH are preferred.

Another preferred embodiement of formula (I) is formula (II), (III) or (IV) shown below.

A compound of claim 1 having the following formula (II): 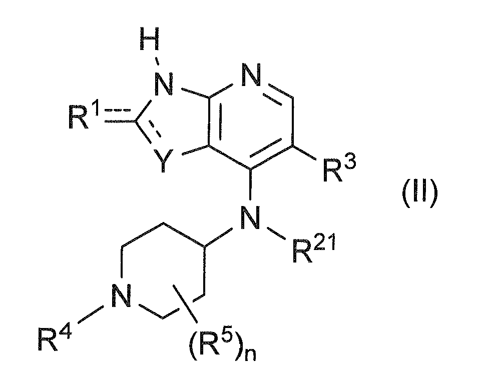 wherein
- R¹: is -H or =O;
- R³: is carbamoyl or oxadiazolyl,
each of which may be substituted with C₁-C₆ alkyl;
- R²¹: is -H or may be bonded with R³ via a certain functional group to form divalent groups represented by the following formula (IA):

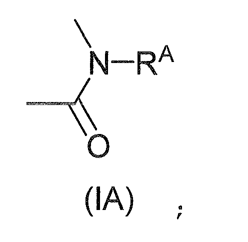

- R^{A}: is -H or C₁-C₆ alkyl;
- R⁴: is aryl, 5- to 6-membered heteroaryl, benzyl, carbamoyl, -C (=O) - (5- to 6- membered heteroaryl), -C(=O)-(5- to 6-membered nitrogen-containing heterocycloalkyl), -C(=O)-carbamoyl, -C(=O)C(=O)- (5- to 6- membered nitrogen-containing heterocycloalkyl), -C(=O)O-(C₁-C₆ alkyl),
each of which may be substituted with one or more identical or different group(s) selected from the group consisting of R⁴¹;
- R⁴¹: is halogen, -CN, -CF₃, -OH, =O, -NO₂, carbamoyl, oxime, C₁-C₆ alkyl, - (C₁-C₆ alkyl) -CN, -(C₁-C₆ alkyl)-OH, -C(=O)O-(C₁-C₆ alkyl), -O-(C₁-C₆ alkyl), -O-(C₁-C₆ alkyl)-CN, 2-pyrroridinone-1-yl or phenyl,
wherein phenyl may be substituted with halogen;
R⁵ is halogen or -O-(C₁-C₆ alkyl) ;
n is an integer from 1 to 6;
Y is N, CH or CH₂; and
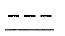 is a single bond or a double bond, wherein the one is single bond, the other is double bond; or pharmaceutically acceptable salts thereof, or prodrug thereof.

A compound of claim 1 having the following formula (III): 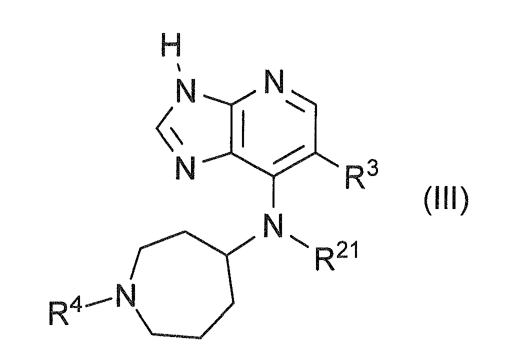 wherein
- R³: is carbamoyl or oxadiazolyl,
each of which may be substituted with C₁-C₆ alkyl;
- R²¹: is -H or may be bonded with R³ via a certain functional group to form divalent groups represented by the following formula (IA):

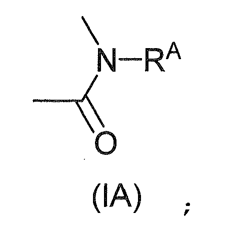

- R^{A}: is -H or C₁-C₆ alkyl;
- R⁴: is aryl, 5- to 6- membered heteroaryl, benzyl, carbamoyl, -(C=O)- (C₁-C₆ alkyl), -C(=O)-(5- to 6- membered heteroaryl), -C(=O)-(5- to 6- membered nitrogen-containing heterocycloalkyl), -C (=O) -carbamoyl, -C(=O)C(=O)-(5- to 6- membered nitrogen-containing heterocycloalkyl), -C(=O)O-(C₁-C₆ alkyl), each of which may be substituted with one or more identical or different group(s) selected from the group consisting of R⁴¹;
- R⁴¹: is halogen, -CN, -CF₃, -OH, =O, -NO₂, carbamoyl, oxime, C₁-C₆ alkyl, -(C₁-C₆ alkyl)-CN, -(C₁-C₆ alkyl)-OH, -C(=O)O-(C₁-C₆ alkyl), -O-(C₁-C₆ alkyl), -O-(C₁-C₆ alkyl)-CN, 2-pyrroridinone-1-yl or phenyl,
wherein phenyl may be substituted with halogen;
or pharmaceutically acceptable salts thereof, or prodrug thereof.

A compound of claim 1 having the following formula (IV): 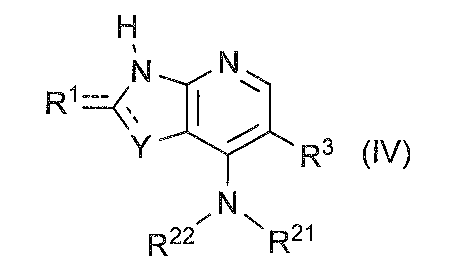 wherein
- R¹: is -H or =O;
- R²¹: is bonded with R³ via a certain functional group to form divalent groups represented by the following formula (IB), (IC) or (ID):

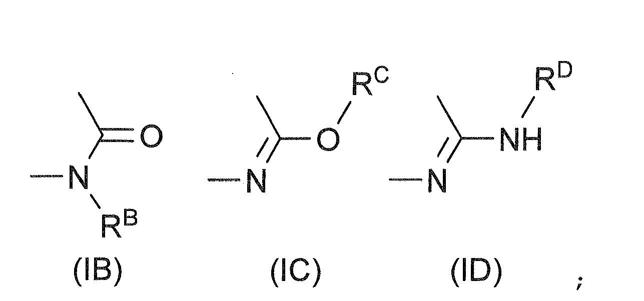

R^{B} is -H or C₁-C₆ alkyl;
R^{C} is -H, C₁-C₆ alkyl or (C₁-C₆ alkyl)-O-(C₁-C₆ alkyl) ;
R^{D} is -H, -C(=O)-(C₁-C₆ alkyl), -C(=O)-(C₁-C₆ alkyl)-O-(C₁-C₆ alkyl), -C(=O)C(=O)NH-(C₁-C₆ alkyl), -C(=O)C(=O)NH-(C₁-C₆ alkyl)-O-(C₁-C₆ alkyl) or -C(=O)O-(alkyl);
R²² is 5- to 7-membered nitrogen-containing heterocycloalkyl, C₃-C₉ cycloalkyl, or benzyl,
each of which may be substituted with one or more identical or different group(s) selected from the group consisting of R^{V1};
R^{V1} is halogen, C₁-C₆ alkyl, -O-(C₁-C₆ alkyl), aryl, 5- to 6- membered heteroaryl, -(C=O)- (C₁-C₆ alkyl), -(C=O)-(5- to 6- membered nitrogen-containing heterocycloalkyl), -(C=O)O-(C₁-C₆ alkyl), or carbamoyl, each of which may be substituted with one or more identical or different group(s) selected from the group consisting of R^{W1};
R^{W1} is halogen, -CN, -CF₃, -OH, =O, -NO₂, carbamoyl, oxime, C₁-C₆ alkyl, -(C₁-C₆ alkyl)-CN, -(C₁-C₆ alkyl)-OH, -C(=O)O-(C₁-C₆ alkyl), -O-(C₁-C₆ alkyl), -O-(C₁-C₆ alkyl)-CN, 2-pyrroridinone-1-yl or phenyl,
wherein phenyl may be substituted with halogen;
X is N, CH or CH₂; and
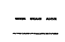 is a single bond or a double bond, wherein the one is single bond, the other is double bond; or pharmaceutically acceptable salts thereof, or prodrug thereof.

One embodiment of suitable groups in formula (II) is exemplified as follows:
- R¹:: -H is preferred.
- R³:: Carbamoyl which may be substituted with C₁-C₆ alkyl is preferred.
- R²¹:: -H or may be bonded with R³ via a certain functional group to form divalent groups represented by the formula (IA) are preferred.
- R^{A}:: -H is preferred.
- R⁴:: Aryl and 5- to 6-membered heteroaryl are preferred.
- R⁴¹:: Halogen, -CN, -CF₃, -OH, =O, -NO₂, carbamoyl, oxime, -(C₁-C₆alkyl)-CN, -(C₁-C₆alkyl)-OH, -C(=O)O-(C₁-C₆ alkyl), -O-(C₁-C₆ alkyl), -O-(C₁-C₆ alkyl)-CN and 2-pyrroridinone-1-yl are preferred.
- R⁵:: Halogen is more preferred.
- n:: An integer from 1 to 2 is more preferred.
- Y:: N and CH are more preferred.

More preferred compound(s) of formula (II) as follows: (from [1] to [3]).
[1]. A compound of [II], wherein:
   - R⁴: is aryl or 5- to 6-membered nitrogen-containing heteroaryl, or -C(=O)O-(C₁-C₆ alkyl), each of which may be substituted with one or more identical or different group(s) selected from the group consisting of R⁴¹;
   - R⁴¹: is halogen, -CN, -CF₃, -CH₂OH, -CONH₂, -C(=O)O- (C₁-C₆ alkyl) or -NO₂;
   or pharmaceutically acceptable salts thereof, or prodrug thereof.
[2]. A compound of [1], wherein:
   - R⁵ is: halogen;
   or pharmaceutically acceptable salts thereof, or prodrug thereof.
[3]. A compound of [2], wherein:
   - R³: is -CONH₂ or -C (=O)NH- (C₁-C₆ alkyl) ;
   - Y: is CH; and
   - R¹: is -H;
   or pharmaceutically acceptable salts thereof, or prodrug thereof.

Another preferred compound(s) of formula(II) is(are):
(1) rel-4-{[(3R,4S)-1-(6-Cyanopyridazin-3-yl)-3-fluoropiperidin-4-yl]amino}-1H-pyrrolo[2,3-b]pyridine-5-carboxamide,
(2) rel-4-{[(3R,4S)-1-(5-Cyanopyridin-2-yl)-3-fluoropiperidin-4-yl]amino}-1H-pyrrolo[2,3-b]pyridine-5-carboxamide,
(3) Ethyl rel-(3R,4S)-4-[(5-carbamoyl-1H-pyrrolo[2,3-b]pyridin-4-yl)amino]-3-methoxypiperidine-1-carboxylate,
(4) rel-4-{[(3R,4R)-1-(5-Cyanopyridin-2-yl)-3-fluoropiperidin-4-yl]amino}-1H-pyrrolo[2,3-b]pyridine-5-carboxamide,
(5) rel-4-{[(3R,4S)-1-(5-Cyanopyridin-2-yl)-3-methoxypiperidin-4-yl]amino}-1H-pyrrolo[2,3-b]pyridine-5-carboxamide,
(6) rel-4-{[(3R,4S)-1-(5-Cyano-1,3-thiazol-2-yl)-3-fluoropiperidin-4-yl]amino}-1H-pyrrolo[2,3-b]pyridine-5-carboxamide,
(7) rel-4-({(3R,4S)-3-Fluoro-1-[6-(trifluoromethyl)pyridazin-3-yl]piperidin-4-yl}amino)-1H-pyrrolo[2,3-b]pyridine-5-carboxamide,
(8) 4-{[1-(5-Cyanopyridin-2-yl)-3,3-difluoropiperidin-4-yl]amino}-1H-pyrrolo[2,3-b]pyridine-5-carboxamide,
(9) rel-6-[(3R,4S)-3-Fluoro-4-(2-oxo-3,6-dihydroimidazo[4,5-d]pyrrolo[2,3-b]pyridin-1(2H)-yl)piperidin-1-yl]nicotinonitrile,
(10) 4-{[(3S,4R)-1-(5-Cyanopyridin-2-yl)-3-fluoropiperidin-4-yl]amino}-1H-pyrrolo[2,3-b]pyridine-5-carboxamide,
(11) 4-{[(3S,4R)-1-(6-Cyanopyridazin-3-yl)-3-fluoropiperidin-4-yl]amino}-1H-pyrrolo[2,3-b]pyridine-5-carboxamide,
(12) 4-{[(3R,4S)-1-(6-Cyanopyridazin-3-yl)-3-fluoropiperidin-4-yl]amino}-1H-pyrrolo[2,3-b]pyridine-5-carboxamide,
(13) 4-{[(3R,4S)-1-(4-Cyanophenyl)-3-fluoropiperidin-4-yl]amino}-1H-pyrrolo[2,3-b]pyridine-5-carboxamide and
(14) rel-6-[(3R,4S)-3-Fluoro-4-(2-oxo-3,6-dihydroimidazo[4,5-d]pyrrolo[2,3-b]pyridin-1(2H)-yl)piperidin-1-yl]nicotinonitrile
or pharmaceutically acceptable salts thereof, or prodrug thereof.

One embodiment of suitable groups in formula (III) is exemplified as follows:
- R³:: Carbamoyl which may be substituted with C₁-C₆ alkyl is preferred.
- R²¹:: -H or may be bonded with R³ via a certain functional group to form divalent groups represented by the formula (IA) are preferred.
- R^{A}:: -H is preferred.
- R⁴:: Aryl or 5- to 6- membered heteroaryl are more preferred.
- R⁴¹:: Halogen, -CN, -CF₃, -OH, =O, -NO₂, carbamoyl, oxime, - (C₁-C₆ alkyl) -OH, -O-(C₁-C₆ alkyl)-CN are more preferred.

More preferred compound(s) of formula (III) as follows: ([41]).

### [4]. A compound of [III], wherein:

- R³: is -CONH₂ or -C(=O)NH-(C₁-C₆ alkyl) ; or pharmaceutically acceptable salts thereof, or prodrug thereof.

Another preferred compound of formula(III) is:
7-{[1-(5-Cyanopyridin-2-yl)azepan-4-yl]amino}-3H-imidazo[4,5-b]pyridine-6-carboxamide,
or pharmaceutically acceptable salts thereof, or prodrug thereof.

One embodiment of suitable groups in formula (IV) is exemplified as follows:
- R¹:: -H is preferred.
- R²¹:: (ID) is more preferred.
- R^{B}:: -H is more preferred.
- R^{C}:: -H is more preferred.
- R^{D}:: -H or -C(=O)C(=O)NH-(C₁-C₆ alkyl) are more preferred.
- R²²: 5- to 7-membered nitrogen-containing heterocycloalkyl or C₃-C₉ cycloalkyl are preferred.
- R^{V1}:: halogen, C₁-C₆ alkyl, -O-(C₁-C₆ alkyl), aryl or 5- to 6- membered heteroaryl are preferred.
- R^{W1}:: halogen, -CN, -CF₃, -OH, =O, -NO₂, carbamoyl, oxime, C₁-C₆ alkyl, -(C₁-C₆ alkyl) -CN, -(C₁-C₆ alkyl)-OH, -C (=O)O-(C₁-C₆ alkyl), -O-(C₁-C₆ alkyl), -O-(C₁-C₆ alkyl)-CN are preferred.
- X:: CH is more preferred.

More preferred compound(s) of formula (IV) as follows: ([5]).

### [5]. A compound of [IV], wherein

- Y: is CH;
- R¹ is: -H;
- R²¹: and R³ form a group of represented by the formula (ID);
- R^{D} is: -H or -C(=O)C(=O)NH-(C₁-C₆ alkyl) ; and
- R²²: is 5- to 7-membered nitrogen-containing heterocycloalkyl or C₃-C₉ cycloalkyl,
each of which may be substituted with one or more identical or different group(s) selected from the group consisting of R^{V1};
or pharmaceutically acceptable salts thereof, or prodrug thereof.

Another preferred compound of formula(IV) is:
1) rel-(2R,4R)-4-fluoro-1-{[(3S,4S)-4-methyl-3-(2-oxo-3,6-dihydroimidazo[4,5-d]pyrrolo[2,3-b]pyridin-1(2H)-yl)piperidin-1-yl]carbonyl}pyrrolidine-2-carbonitrile,
2)rel-3-[(3R,4R)-3-(3-aminopyrazolo[3,4-d]pyrrolo[2,3-b]pyridin-1(6H)-yl)-4-methylpiperidin-1-yl]-3-oxopropanenitrile,
3)rel-3-[(3R,4R)-4-methyl-3-(3-oxo-3,6-dihydropyrazolo[3,4-d]pyrrolo[2,3-b]pyridin-1(2H)-yl)piperidin-1-yl]-3-oxopropanenitrile,
4)rel-N-{1-[(3R,4R)-1-(cyanoacetyl)-4-methylpiperidin-3-yl]-1,6-dihydropyrazolo[3,4-d]pyrrolo[2,3-b]pyridin-3-yl}-N'-(cyclopropylmethyl)ethanediamide,
5)rel-6-[(3R,4R)-3-(3-aminopyrazolo[3,4-d]pyrrolo[2,3-b]pyridin-1(6H)-yl)-4-methylpiperidin-1-yl]pyridazine-3-carbonitrile,
6)rel-N-{1-[(3R,4R)-1-(5-cyanopyridin-2-yl)-4-methylpiperidin-3-yl]-1,6-dihydropyrazolo[3,4-d]pyrrolo[2,3-b]pyridin-3-yl}-N'-(cyclopropylmethyl)ethanediamide,
7)rel-N-(1-{(3R,4R)-1-[(cyanomethyl)(methyl)carbamoyl]-4-methylpiperidin-3-yl}-1,6-dihydropyrazolo[3,4-d]pyrrolo[2,3-b]pyridin-3-yl)-N'-isopropylethanediamide, and
8)rel-N-(1-{(3R,4R)-1-[(cyanomethyl)(methyl)carbamoyl]-4-methylpiperidin-3-yl}-1,6-dihydropyrazolo[3,4-d]pyrrolo[2,3-b]pyridin-3-yl)-N`-(cyclopropylmethyl)ethanediamide
   or pharmaceutically acceptable salts thereof, or prodrug thereof.

The compound according to the present invention may include geometric isomers and tautomeric isomers depending on the type of substituent. In addition, the compound according to the present invention may have asymmetric carbon atoms. All of these isomers, including separated isomers and mixtures thereof, are included within the scope of the present invention. Furthermore, labeled compounds, that is, compounds afforded by substituting one or more atom(s) of the compound according to the present invention with radioactive or nonradioactive isotopes are also included in the scope of the present invention.

Furthermore, the so-called pharmaceutically acceptable prodrug of the compound of the present invention is also included in the scope of the present invention. The pharmaceutically acceptable prodrug is a compound having a group that can be converted into an amino group, hydroxyl group, carboxyl group, and the like. through solvolysis or under physiological conditions. The groups described in Prog. Med., Vol. 5, p. 2157-2161, 1985 and "Iyakuhin No Kaihatsu (Development of Medicines) " (Hirokawa Pub. Co. , 1990), Vol. 7, Molecular Design, p. 163-198 are taken as examples of groups forming such prodrugs.

The compound represented by the formula (I) may form acid or base addition salts. These salts should only be pharmaceutically acceptable salts. More specifically, the salts may include an acid addition salt with an inorganic acid (e.g., hydrochloric acid, hydrobromic acid, hydroiodic acid, sulfuric acid, nitric acid, and phosphoric acid), and an acid addition salt with an organic acid (e.g., formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, and glutamic acid) ; a salt with an inorganic base (e.g., sodium, potassium, magnesium, calcium, and aluminum), and a salt with an organic base (e.g., methylamine, ethylamine, ethanolamine, lysine, and ornithine) ; an ammonium salt; and the like.

Still further, various hydrates, solvates, and crystalline polymorphic forms of the compound represented by the formula (I) and salts thereof are also included in the scope of the present invention.

### <PRODUCTION METHOD>

The production method of compound (I) was shown below. Each production method can be used in referred to known papaer In addition, the production method of the present invention was not restricted to examples descrived below.

The compound according to the present invention can be produced using the characteristics based on the basic skeleton or the type of substituent thereof and by applying various known synthesis methods. During the production, protecting the relevant functional group with a suitable protective group or replacing the relevant functional group with a group that can be easily converted into the functional group at the stage of a starting substance or an intermediate may occasionally be effective depending on the type of the functional group in production technology. The protective group for such a functional group may include, for example, the protective groups described in "Protective Groups in Organic Synthesis (3rd. Ed, 1999)" written by T. W. Greene and P. G. M. Wuts, and one of these should only be selected and used as necessary depending on reaction conditions. In this kind of method, the desired compound can be afforded by introducing the protective group, by carrying out reaction and by eliminating the protective group as necessary, or by converting the group into a desired group.

In addition, the prodrug of the compound according to the present invention can be produced by introducing a specific group or by carrying out reaction using the afforded compound represented by the formula (I) at the stage of a starting substance or an intermediate, just as in the case of the above-mentioned protective group. The reaction can be carried out using methods known to those skilled in the art, such as ordinary esterification, amidation, and dehydration.

### <ABBREVIATIONS>

The following abbreviations are used in the present specification:
AcOH: acetic acid,
API-ES: API-ES MS,
CDI: 1,1'-carbonylbis-1H-imidazole,
DCC: N,N'-dicyclohexylcarbodiimide,
DCM: dichloromethane,
DIPEA: N,N'-diisopropylethylamine,
DMA: N,N'-dimethylacetamide
DMAP: 4-(N,N-dimethylamino)pyridine
DMF: N,N'-dimethylformamide,
DMI: 1,3-dimethyl-2-imidazolidinone,
DMSO: dimethylsulfoxide,
DPPA: diphenylphosphoryl azide,
EDCI: 1-(3-dimethylaminopropyl)-3-ethylcarbodiimide,
EDCI·HCl: 1-ethyl-3-(3-dimethylaminopropyl)carbodiimide hydrochloride,
EtOAc: ethyl acetate,
EtOH: ethanol,
HCl aqueous solution:hydrochloric acid aqueous solution,
HCl/dioxane: hydrogen chloride in dioxane,
HCl/EtOH: hydrogen chloride in ethanol,
HOBt: N-hydroxybenzotriazole,
KHCO₃: potassium hydrogencarbonate,
K₂CO₃: sodium carbonate,
LiAlH₄: lithium aluminium hydride,
LiCl: lithium chloride,
MeCN: acetonitrile,
MeOH: methanol,
MgSO₄: magnesium sulfate,
MEK: methyl ethyl ketone,
n-hexane: normal-hexane,
KOBu^{t}: potassium tert-butoxide,
NH₃/MeOH: ammonia in MeOH
NH₃: ammonia
NH₄Cl: ammonium chloride
NMM: N-methylmorpholine
NMP: 1-methyl-2-pyrrolidinone,
N₂ atmosphere: Nitrogen atmosphere,
Na₂CO₃: potassium carbonate,
Na₂SO₄: sodium sulfate,
NaH: sodium hydride,
NaHCO₃: sodium hydrogencarbonate
NaOH: sodium hydroxide
Pd(OAc)₂: palladium acetate,
Pd(OH)₂: palladium hydroxide,
Pd(PPh₃)₄: tetrakis(triphenylphosphine) palladium(O)
TBAF: tetrabutylammonium fluoride,
TEA: triethylamine,
TFA: trifluoroacetic acid,
TFAA: trifluoroacetic anhydride,
THF: tetrahydrofuran,
TLC: thin layer chromatography,
brine: saturated sodium chloride aqueous solution
i-PrOH: 2-propanol,
n-BuOH: 1-butanol,
t-BuOH: tert-butanol
Ex: Example Number,
Pr: Preparation Number,
rel: relative structure; either enantiomer is showm.
Syn: process (the number indicates that the compound corresponding to the Example was produced using a process similar to that for the compound corresponding to the Example identified by the number.)

The compound according to the present invention can be produced using the characteristics based on the basic skeleton or the type of substituent thereof and by applying various known synthesis methods. During the production, protecting the relevant functional group with a suitable protective group or replacing the relevant functional group with a group that can be easily converted into the functional group at the stage of a starting substance or an intermediate may occasionally be effective depending on the type of the functional group in production technology. This kind of functional group may include, for example, amino group, hydroxyl group, and carboxyl group. The protective group for such a functional group may include, for example, the protective groups described in "Protective Groups in Organic Synthesis (3rd. Ed, 1999) " written by T. W. Greene and P. G. M. Wuts, and one of these should only be selected and used as necessary depending on reaction conditions. In this kind of method, the desired compound can be afforded by introducing the protective group, by carrying out reaction and by eliminating the protective group as necessary, or by converting the group into a desired group.

In addition, the prodrug of the compound according to the present invention can be produced by introducing a specific group or by carrying out reaction using the afforded compound represented by the formula (I) at the stage of a starting substance or an intermediate, just as in the case of the above-mentioned protective group. The reaction can be carried out using methods known to those skilled in the art, such as ordinary esterification, amidation, and dehydration.

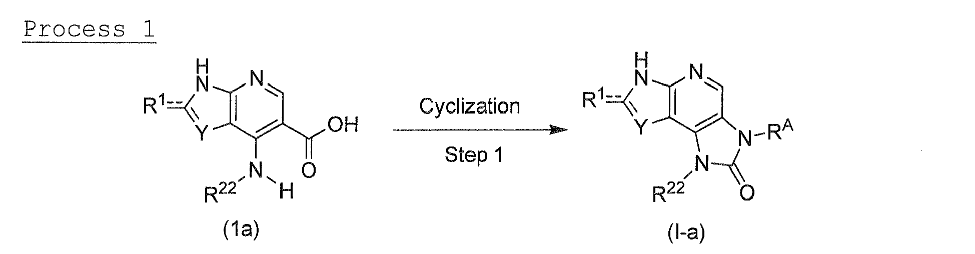

In this process, the compound according to the present invention represented by the formula (1a) and having a carboxyl group is used as a starting compound to produce the compound according to the present invention represented by the formula (I-a).

In this step, the carboxyl group of the compound represented by the formula (1a) is reacted with an azidation agent, such as DPPA and sodium azide, to construct an imidazolone ring according to the so-called Curtius rearrangement reaction. It is advantageous that the reaction is carried out in the presence of a base. When -R^{A} is not -H, derivation to a compound in which -R^{A} is not -H is made possible as necessary by applying usual alkylation or the like to >N-H of an imidazolone ring constructed by the above-mentioned reaction. The reaction is carried out in the presence of a solvent which does not adversely influence the reaction, such as t-BuOH and toluene. Usually, TEA, pyridine and the like can be used as a base, and the reaction can be carried out under ambient temperature, under ambient temperature to heating, or under heating and refluxing. 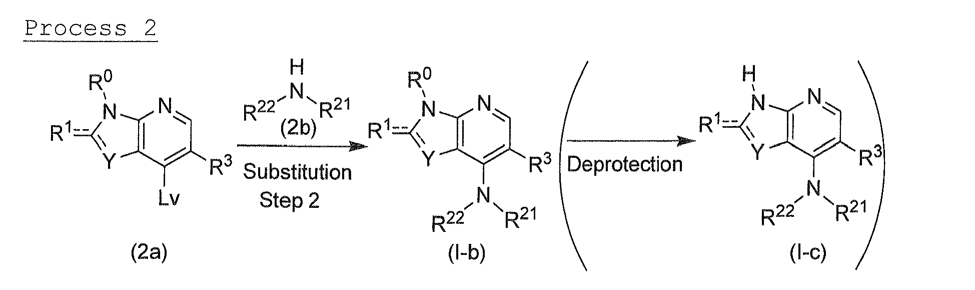 [wherein Lv is a leaving group. R⁰ is -H or a protecting group for the nitrogen atom. These definitions are applicable to the following similarly.]

In this process, the compound represented by the formula (2a) and having a leaving group is reacted with the amine represented by the formula (2b) to produce the compound represented by the formula (I-b) . The leaving group may include halogen (e.g., chloro and bromo); sulfonyloxy (e.g., methanesulfonyloxy, ethanesulfonyloxy, benzenesulfonyloxy, p-toluenesulfonyloxy, p-nitrobenzenesulfonyloxy, and trifluorometanesulfonyloxy); and the like.

In this step, the leaving group of the compound represented by the formula (2a) is substituted with amine represent by the formula (2b). This reaction is carried out under atmospheric pressure or under pressure in the absence or presence of a suitable solvent.

The solvent may include, for example, aromatic hydrocarbons (e.g., toluene and xylene); ketones (e.g., acetone and MEK); ethers (e.g., ether, THF, dioxane, and diethoxyethane); alcohols (e.g., MeOH, EtOH, i-PrOH, and n-BuOH); halogenated hydrocarbons (e.g., DCM, 1,2-dichloroethane, chloroform, and carbon tetrachloride); MeCN; aprotic polar solvents (e.g., DMF, DMI, NMP, and DMSO); water; or a mixture of these. It is preferable that the reaction is carried out in the presence of a base, and the base may include, for example, alkaline carbonates (e.g., K₂CO₃ and Na₂CO₃) ; alkaline hydrogencarbonates (e.g., NaHCO₃ and KHCO₃); alkoxides (e.g., sodium methoxide, sodium ethoxide, and KOBu^{t}); tertiary amines (e.g., TEA, tributylamine, and DIPEA); and organic bases (e.g., 1,8-diazabicyclo[5.4.0]undeca-7-ene, pyridine, and lutidine). However, an excess amount of the compound represented by the formula (2b) can also be used. Although the reaction temperature differs depending on the type of a starting compound and reaction conditions, the reaction can usually be carried out at a temperature approximately ranging from ambient temperature to the refluxing temperature of a solvent. The reaction can also usually be carried out in the presence of a base, such as NaH and K₂CO₃, in an organic solvent inert to the reaction, such as DMF and DMA, under ambient temperature to heating. In addition, the amine represented by the formula (2b) can also be used as a salt thereof for the reaction.

Furthermore, microwave irradiation can also be carried out under heating. Still further, the reaction can also be carried out by a coupling reaction using a phosphorus reagent, such as 2-(di-tert-butylphosphino)biphenyl, and a palladium catalyst, such as Pd(OAc)₂, in the presence of a base, such as cesium carbonate.

The compound represented by the formula (I-c) is also afforded by deprotecting R⁰ of the compound represented by the formula (I-b).

For the reaction, it is possible to use the methods described in the Preparations or the Examples of the present specification or methods similar to those. The compound represented by the formula (2a) can thus be produced using known methods, methods obvious to those skilled in the art, or the methods described in the Preparations or the Examples of the present specification or methods similar to those.

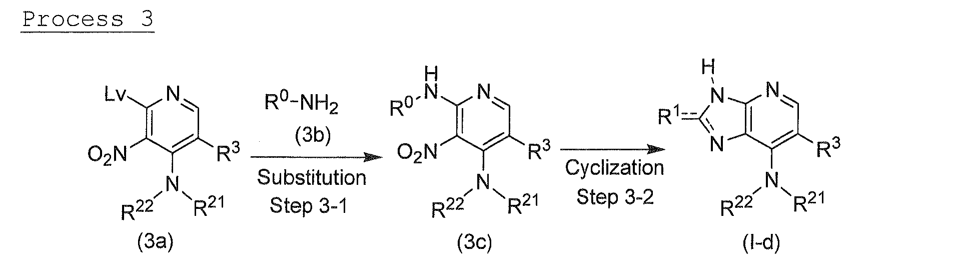

In this process, the nitropyridine compound represented by the formula (3a) is reacted with the amine represented by the formula (3b), and the leaving group at the second position is substituted with the amine to derive the aminonitropyridine compound represented by the formula (3c). The derived compound is used to produce the compound according to the present invention represented by the formula (I-d).

The second process can be incorporated in Step 3-1. The amine represented by the formula (3b) can also be used as a salt thereof for the reaction.

In Step 3-2 in the case that -R⁰ is -H, an imidazole ring can be constructed by reacting an orthoformate, such as ethyl orthoformate, in the presence of an acid catalyst. It is desirable that the nitro group should be reduced before the orthoformate is used for the reaction. Furthermore, the method to be used when -R⁰ is not -H may include, for example, a method in which the amino group of the compound represented by the formula (3c) is acylated in advance, a method in which tetraalkylorthocarbonate or alkylisothiocyanate is used instead of the orthoformate, and a method in which carboxylic acid or carboxylic anhydride is reacted with a strong acid, such as sulfonic acid. These reactions can be carried out in a solvent inert to the reactions or in the absence of a solvent, under ambient temperature, under ambient temperature to heating, or under heating and refluxing. 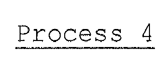 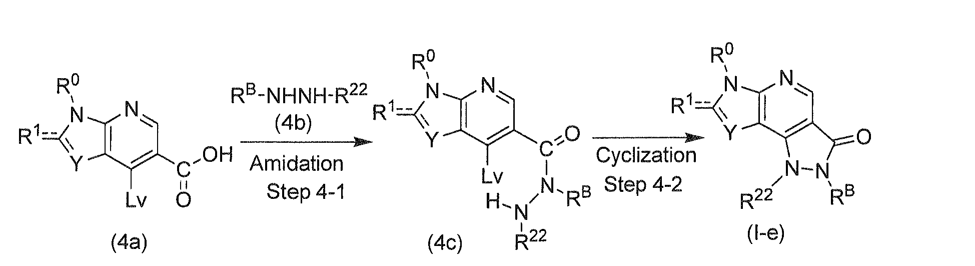 In this process, the carboxylic compound represented by the formula (4a) is reacted with the hydrazine derivative represented by the formula (4b) to afford the hydrazide represented by the formula (4c). From the hydrazide, the compound according to the present invention represented by the formula (I-e) is produced.

Step 4-1 is a reaction in which the compound represented by the formula (4a) and the compound represented by the formula (4b) are condensed by amidation. The compound represented by the formula (4a) can be used as a free acid for the reaction, and the reactive derivative thereof can also be used for the reaction. The reactive derivative of the compound represented by the formula (4a) may include an acid halide (e.g., acid chloride and acid bromide) ; an ordinary ester (e.g., methyl ester, ethyl ester, and benzyl ester); acid azide; an activated ester with HOBt, p-nitrophenyl, or N-hydroxysuccinimide); a symmetric acid anhydride; a mixed acid anhydride with a halocarboxylic acid alkyl ester (e.g., alkyl halide carbonate), pivaloyl halide, p-toluenesulfonic acid chloride and the like; and a mixed acid anhydride, such as a phosphoric acid-type mixed acid anhydride afforded by reaction with diphenylphosphoryl chloride or NMM; and the like.

When the compound represented by the formula (4a) is reacted in the form of a free acid or reacted without isolating the activated ester, it is preferable to use a condensing reagent, such as DCC, CDI, DPPA, diethyphosphoryl cyanide, and EDCI·HCl.

The reaction is carried out in an organic solvent inert to the reaction, such as halogenated hydrocarbons, aromatic hydrocarbons, ethers, esters (e.g., EtOAc), MeCN, DMF, and DMSO, under cooling, under cooling to ambient temperature, or under ambient temperature to heating, although the conditions differ depending on the reactive derivative or the condensing reagent to be used.

In order to smoothly advance the reaction, it is occasionally advantageous that an excess amount of the compound represented by the formula (4b) is used for the reaction or the reaction is carried out in the presence of a base, such as NMM, trimethylamine, TEA, DIPEA, N,N-dimethylaniline, pyridine, DMAP, picoline, and lutidine. Pyridine can also be used as a solvent.

The method used in Step 1 of the first process can be incorporated in Step 4-2. 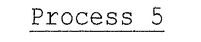 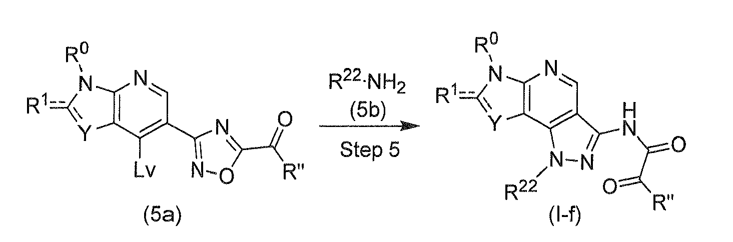 [wherein R", -C(=O)C(=O)-R" and R^{D} are the same. These definitions are applicable to the following similarly.]

In Step 5, in the case that the compound represented by the formula (5a) is reacted with the primary amine represented by the formula (5b), after ipso substitution, the oxadiazole ring is opened to construct an aminopyrazolone ring, and the compound represented by the formula (I-f) can be produced. The reaction conditions described in the second process can be incorporated herein as the reaction conditions. The reaction can be carrying out under ambient temperature to refluxing temperature. 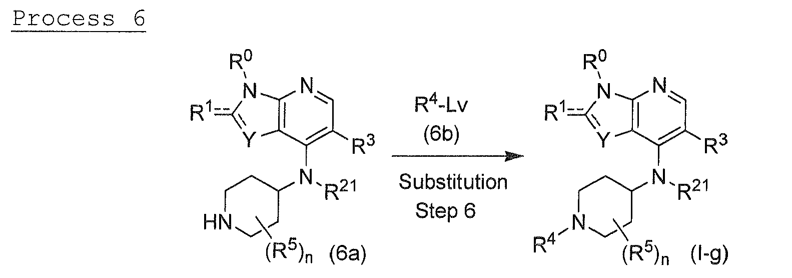 The compound represented by the formula (I-g), wherein R⁴ is aryl, 5- to 6- membered heteroaryl can be synthesized from compound represented by the formula (6a) with similar manner of the method written in Process 2. The compound represented by the formula (I-g), wherein R⁴ is benzyl, carbamoyl, -C (=O) - (5- to 6- membered heteroaryl), -C(=O)-(5- to 6- membered nitrogen-containing heterocycloalkyl), -C(=O)-carbamoyl, -C(=O)C(=O)-(5- to 6-membered nitrogen-containing heterocycloalkyl can be synthesized as described above by appropriately combining processes usually used by those skilled in the art.

In addition, some of the compounds represented by the formula (I) can also be produced from the compound according to the present invention produced as described above by appropriately combining processes usually used by those skilled in the art, such as known alkylation, acylation, substitution, oxidation, reduction, hydrolysis, deprotection, halogenation, and Mannich reaction. For example, when producing the compound (I) wherein -R^{A} is C₁-C₆ alkyl from compound (I) wherein -R^{A} is -H, alkylation can be used referring to the method described in "Jikken Kagaku Koza (Courses in Experimental Chemistry) (5th Ed., 2003)." Furthermore, the processes capable of being usually used by those skilled in the art are not only used for the compound according to the present invention but can also be used for intermediates formed during production. The processes can also advance to subsequent processes.

The compound produced as described above is in a free form or subjected to salt-forming processing using a conventional method and isolated and purified as a salt thereof. The isolation and purification are carried out by performing ordinary chemical operations, such as extraction, concentration, evaporation, crystallization, filtration, recrystallization, and various types of chromatography.

Various types of isomers can be isolated by utilizing the difference in physicochemical properties between the isomers using a conventional method. For example, a racemic mixture can be converted into an optically pure isomer using a general racemic resolution method, such as a method in which the racemic mixture is converted into a diastereomer salt with a general optically-active acid or optically-active base, and subjected to optical resolution. Furthermore, the diastereo mixture can be separated by fractional crystallization or various types of chromatography, for example. Still further, the optically-active compound can also be produced using a suitable optically-active material.

The pharmacological activity of the compound according to the present invention was verified by carrying out the following test.

### Test example 1 JAK3 inhibition test

The JAK3 inhibition test was performed as described below

### (1) Preparation of human JAK3

Purified human JAK3 kinase domain was purchased from Carna Biosciences, Inc. (Kobe, Japan). This was afforded as described below. His-tag (41 kDa) was attached to the N-terminal of the 796-1124 (C-terminal) fragment of the human JAK3 protein (accession number #NM_000215), expressed using baculovirus expression system, and then purified using Ni-NTA affinity column chromatography.

### (2) Measurement of JAK3 activity

As substrates, Biotin-Lyn-Substrate-2 (Biotin-XEQED EPEGF YFEWL EPE), X = ε -Acp (PEPTIDE INSTITUTE, INC., Osaka, Japan) and ATP were used. As an assay buffer, 15mM Tris-HCl pH 7.5 containing 0.01% Tween 20 and 2mM DTT (dithiothreitol) was used. Normally, 20 µL of a substrate solution (an assay buffer containing 627 nM Biotin-Lyn-Substrate-2, 20µM ATP, and 25mM MgCl₂), an assay buffer containing 10 µL of a compound to be tested, and 20 µL of an enzyme solution were added to a microplate, and stirred sufficiently.

After incubation at ambient temperature for one hour, the plate was washed with a cleaning buffer (50mM Tris-HCl pH 7.5, 150mM NaCl, 0.02% Tween 20), and a blocking buffer (a cleaning buffer containing 0.1% bovine serum albumin) was added to the plate. After incubation at ambient temperature for 30 minutes, the blocking buffer was removed, and an HRP-PY-20 solution (afforded by diluting HRP-PY-20 solution with the blocking buffer 500 times) was added. After incubation at ambient temperature for 30 minutes, the plate was washed four times, and a TMB substrate solution (Sigma) was added to the plate. After incubation at ambient temperature for 4 minutes, 1M sulfuric acid was added to stop the reaction. Enzyme activity was measured as absorbance at 450 nm. The JAK3 inhibitory activity of the test compound was calculated assuming that the concentration of the test compound inhibiting JAK3 activity by 50% is an IC₅₀ value.

As a result, the compounds according to the present invention described in the following Examples exhibited the following IC₅₀ values:

**Table 1**

| Example No. | IC₅₀ value (nM) | Example No. | IC₅₀ value (nM) |
|---|---|---|---|
| 2 | 0.86 | 66 | 1.0 |
| 6 | 0.76 | 72 | 1.6 |
| 12 | 10 | 77 | 4.8 |
| 21 | 0.52 | 82 | 2.7 |
| 22 | 1.7 | 91 | 3.2 |
| 41 | 1.1 | 94 | 0.30 |
| 49 | 2.2 | 110 | 10 |
| 58 | 3.4 | 129 | 3.8 |
| 65 | 0.78 | | |

In addition this invention compound was compared with rerferece compound shown in Table 2. These compounds were produced according to the method disclosed therein.

**Table 2**

| No | Structure | IC₅₀ value (nM) |
|---|---|---|
| Reference compound A EX.NO. 33 in WO2006/069080 | 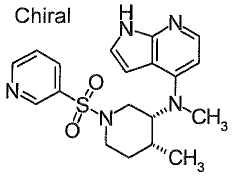 | 230 |
| Reference compound B No. 182 in WO2006/127587 | 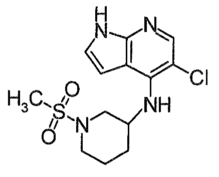 | 31 |

As a result, it has been verified that the compound according to the present invention has inhibition activity against JAK3 and is useful as an active ingredient of an agent for treating or preventing diseases caused by undesirable cytokine signal transduction (e.g., rejection during live organ/tissue transplantation, autoimmune diseases, asthma, atopic dermatitis and atherosclerosis, psoriasis, and rheumatism), or diseases caused by abnormal cytokine signal transduction (e.g., cancer and leukemia).

In addition, on the basis of the JAK3 inhibition activity, the compound according to the present invention is useful for treating and/or preventing the following diseases:
Autoimmune diseases, asthma, atopic dermatitis, atherosclerosis, cancer and leukemia, and diseases and conditions caused by undesirable cytokine signal transduction, as exemplified below:
   for example, rejection reactions by transplantation of organs or tissues, such as heart, kidney, liver, bone marrow, skin, cornea, lung, pancreas, pancreatic islet, islet, small intestine, limb, muscle, nerve, intervertebral disc, trachea, myoblast, and cartilage; graft-versus-host reactions following bone marrow transplantation; and autoimmune diseases, such as rheumatism, systemic erythematosus, Hashimoto's thyroiditis, multiple sclerosis, myasthenia gravis, type I diabetes, and diabetes complication.

Furthermore, on the basis of the JAK3 inhibition activity, the compound according to the present invention is also useful for treating and/or preventing the following diseases:
inflammatory or hyperproliferative skin diseases or cutaneous manifestations of immunologically-mediated diseases (e.g., psoriasis, atopic dermatitis, contact dermatitis, eczematoid dermatitis, seborrheic dermatitis, lichen planus, pemphigus, bullous pemphigoid, epidermolysis bullosa, urticaria,
angioedema, vasculitis, erythema, dermal eosinophilia, lupus erythematosus, acne, and alopecia areata);
autoimmune diseases of the eye (e.g., keratoconjunctivitis, vernal conjunctivitis, uveitis associated with Behcet' s disease, keratitis, herpetic keratitis, conic keratitis, corneal epithelial dystrophy, keratoleukoma, ocular pemphigus, Mooren's ulcer, scleritis, Grave's ophthalmopathy, Vogt-Koyanagi-Harada syndrome, keratoconjunctivitis sicca (dry eye), phlyctenule, iridocyclitis, sarcoidosis, and endocrine ophthalmopathy);
reversible obstructive airways diseases [asthma (e.g., bronchial asthma, allergic asthma, intrinsic asthma, extrinsic asthma, and dust asthma), particularly chronic or inveterate asthma (e.g., late asthma and airway hyper-responsiveness), bronchitis, and the like.];
mucosal or vascular inflammations (e.g., gastric ulcer, ischemic or thrombotic vascular injury, ischemic bowel diseases, enteritis, necrotizing enterocolitis, intestinal damages associated with thermal burns, and leukotriene B4-mediated diseases);
intestinal inflammations/allergies (e.g., proctitis, eosinophilic gastroenteritis, mastocytosis, Crohn's disease, and ulcerative colitis);
food-related allergic diseases with symptomatic manifestation remote from the gastrointestinal tract (e.g., migrain, rhinitis, and eczema);
autoimmune diseases and inflammatory diseases (e.g., primary mucosal edema, autoimmune atrophic gastritis, premature menopause, juvenile diabetes mellitus, pemphigus vulgaris, pemphigoid, sympathetic ophthalmitis, lens-induced uveitis, idiopathic leukopenia, active chronic hepatitis, idiopathic cirrhosis, discoid lupus erythematosus, autoimmune orchitis, arthritis (e.g., arthritis deformans), and polychondritis); allergic conjunctivitis.

Furthermore, the compound according to the present invention is useful for treating and/or preventing liver diseases [e.g., immunogenic diseases (e.g., chronic autoimmune liver diseases, such as autoimmune hepatic diseases, primary biliary cirrhosis, and sclerosing cholangitis), partial liver resection, acute liver necrosis (e.g., necrosis caused by toxins, viral hepatitis, shock, and anoxia), hepatitis B, non-A non-B hepatitis, hepatocirrhosis, hepatic failure (e.g., fulminant hepatitis, late-onset hepatitis, liver failure (acute liver failure or chronic liver diseases)].

Besides some of the present invention compounds showed low adverse effect, e.g., Example 41 was found to exhibit IC₅₀ values in the HERG assay of 100 µM or greater.

The pharmaceutical composition containing one or two or more kinds of the compound represented by the formula (I) or pharmaceutically acceptable salts thereof as an active ingredient can be prepared using carriers, excipients, and other additives usually used for pharmaceutical preparation.

Therapeutic administration can be accomplished either by oral administration via tablets, pills, capsules, granules, powders, solutions, and the like., or parenteral administration via intravenous or intramascular injections, suppositories, transdermal agents, transnasal agents, inhalers, and the like.

The solid composition for use in the oral administration according to the present invention is used in the form of tablets, powders, granules, and the like. In such a solid composition, one or more active ingredient(s) are mixed with at least one inactive excipient, such as lactose, mannitol, glucose, hydroxypropyl cellulose, microcrystalline cellulose, starch, polyvinyl pyrrolidone, and magnesium aluminometasilicate. According to a usual method, the composition may contain inactive additives, such as a lubricant (e.g., magnesium stearate), a disintegrating agent (e.g., carboxymethyl starch sodium), and a solubilization assisting agent. If necessary, tablets or pills may be coated with sugar or a film of a gastric or enteric coating substance.

The liquid composition for oral administration contains pharmaceutically acceptable emulsions, solutions, suspensions, syrups, or elixirs, and also contains a generally used inert diluent, such as purified water or ethanol. In addition to the inert diluent, the composition may also contain auxiliary agents, such as a solubilization assisting agent, a moistening agent, and a suspending agent, as well as sweeteners, flavors, aromatics, and antiseptics.

The injections for parenteral administration contain aseptic aqueous or non-aqueous solutions, suspensions, or emulsions. The diluent for use in the aqueous solutions may include, for example, distilled water for injection use and physiological saline. The diluent for use in the non-aqueous solutions may include, for example, propylene glycol, polyethylene glycol, plant oil (e.g., olive oil), alcohol (e.g., ethanol), and polysorbate 80 (official name). Such a composition may further contain additive agents, such as a tonicity agent, an antiseptic agent, a moistening agent, an emulsifying agent, a dispersing agent, a stabilizing agent, and a solubilization assisting agent. These compositions are sterilized by filtration through a bacteria retaining filter, blending of a germicide, or irradiation. Furthermore, they may also be produced in the form of sterile solid compositions and dissolved or suspended in sterile water or a sterile solvent for injection use prior to their use.

The transmucosal agents, such as inhalers and transnasal agents, are used in the form of solid, liquid or semisolid and can be produced according to conventional known methods. For example, excipients (e.g., lactose and starch), pH adjusters, antiseptics, surface active agents, lubricants, stabilizers, and thickners may also be added as necessary. For administration, suitable devices for inhalation or insufflation can be used. For example, using known devices and sprayers, such as measuring inhalation devices, the compound can be administered independently or in the form of prescribed mixture powders. Furthermore, the compound combined with pharmaceutically acceptable carriers can also be administered in the form of solutions or suspensions. Dry powder inhalers and the like may be devices for single or multiple administrations, and dry powders or capsules containing powders can also be used. Still further, the devices may be in the form of a pressure aerosol spray or the like that uses a suitable ejection agent, such as chlorofluoroalkane or hydrofluoroalkane, or a suitable gas, such as carbon dioxide.

The drug for external use may include ointments, plasters, creams, jellies, patches, sprays, lotions, eye-drops, eye ointments, and the like. The drug contains generally used ointment bases, lotion bases, aqueous or non-aqueous solutions, suspensions, emulsions, and the like. The ointment bases or lotion bases may include, for example, polyethylene glycol, carboxyvinal polymer, white vaseline, bleached bee wax, polyoxyethylene hydrogenated castor oil, glyceryl monostearate, stearyl alcohol, cetyl alcohol, lauromacrogol, and sorbitan sesquioleate.

The dose of the compound is determined appropriately in consideration of the conditions, age, sex, and the like of each patient to be treated. Usually, in the case of oral administration, a daily dose of approximately 0.001 to 100 mg/kg, preferrably 0.1 to 30 mg/kg, more preferrably 0.1 to 10 mg/kg, of the compound can be administered one or two to four times per day for an adult patient. When intravenous administration is required depending on conditions, a dose of 0. 0001 to 10 mg/kg of the compound can usually be administered one to multiple times per day for an adult patient. Furthermore, in the case of inhalation, a dose of 0.001 to 100 mg/kg of the compound can usually be administered one to multiple times per day for an adult patient.

Moreover, the compound according to the present invention can be administered alone as a JAK3 inhibitor or in combination with one or more additional agent(s) in the same or different dosage via the same or different routes of administration. Agents that can be used in combination may include but not limited to cyclosporin A, tacrolimus, sirolimus, everolimus, micophenolate, azathioprine, brequinar, leflunomide, fingolimod, anti-IL-2 receptor antibody (e.g. daclizumab), anti-CD3 antibody (e.g. OKT3), Anti-T cell immunogloblin (e.g. AtGam), aspirin, acetaminophen, ibuprofen, naproxen, piroxicam, and anti inflammatory steroid (e.g. prednisolone or dexamethasone).

### EXAMPLES

Although the present invention will be described below specifically by way of Examples, the present invention is not limited by these Examples at all. Starting compounds being used in Examples include novel substances, and the methods for producing such starting compounds from known compounds will be described by way of Preparations.

The following Table(s) describes the processes and physicochemical data for the compounds described in Examples.

Pr: preparation, Structure: chemical structure, Data: physicochemical data. NMR: ¹H-NMR (400MHz, d₆-DMSO) δ, NMR(CDCl₃) : ¹H-NMR (400MHz, CDCl₃). MS: API-ES or ESI-MS.

### Preparation 1

The compound(s) in Preparations 1-1 to 1-14 shown in the following Table were produced using the corresponding starting materials in a similar manner to that of Example 6.

### Preparation 2

The compound(s) in Preparations 2-1 to 2-9 shown in the following Table(s) were produced using the corresponding starting materials in a similar manner to that of Example 7.

### Preparation 3

A mixture of 4,5-dihydro-1H-pyrazole(350 mg) and 4-nitrophenyl carbonyloxy chloride (1.10 g) in dioxane(3 mL) was added 4M saturated NaHCO₃ aqueous solution (3.7 mL) and stirred at ambient temperature for 1 hour. The reaction mixture was diluted with chloroform. The organic layer was dried over anhydrous MgSO₄. The mixture was filtered and the filtrate was evaporated in vacuo to afford 4-nitrophenyl 4,5-dihydro-1H-pyrazole-1-carboxylate (1.12 g) as white solid.

The compound(s) in Preparations 3-1 to 3-7 shown in the following Table were produced using the corresponding starting materials in a similar manner to that of Preparations 3.

### Preparation 4

The compound(s) in Preparations 4-1 to 4-3 shown in the following Table were produced using the corresponding starting materials in a similar manner to that of Example 12.

### Preparation 4-4

To a solution of methyl 5-(2-oxopyrrolidin-1-yl)thiophene-2-carboxylate (240 mg) in methanol (2 mL), was added 1M NaOH aqueous solution (2 mL) dropwise at ambient temperature. The reaction mixture was gently refluxed for 3 hours. After the reaction mixture was cooled to ambient temperature, the solution was half evaporated and acidified with 1M HCl aqueous solution. Resulting precipitate was collected by filtration, washed with water and dried under vacuum to afford 5-(2-oxopyrrolidin-1-yl)thiophene-2-carboxylic acid (190 mg) as brown powder.

The compound (s) in Preparations 4-5 to 4-11 shown in the following Table were produced using the corresponding starting materials in a similar manner to that of Preparations 4-4.

### Preparation 4-12

To a solution of ethyl 4-{[1-(5-cyanopyridin-2-yl)piperidin-4-yl]amino}-1H-pyrrolo[2,3-b]pyridine-5-carboxylate (304 mg) in MeOH (3.0 mL) was added 1M NaOH aqueous solution (0.78 mL), the mixture was stirred at ambient temperature for 12 hours. The pH of the reaction mixture was adjusted to ca.7.0 with 1M HCl aqueous solution and extracted with chloroform three times. The organic extract was combined, dried over anhydrous MgSO₄ and concentrated in vacuo to give 4-{[1-(5-cyanopyridin-2-yl)piperidin-4-yl]amino}-1H-pyrrolo[2,3-b]pyridine-5-carboxylic acid (245 mg).

### Preparation 5

The compound(s) in Preparations 5-1 to 5-14 shown in the following Table were produced using the corresponding starting materials in a similar manner to that of Example 14.

### Preparation 6

To a solution of a mixture of N-{1-[(3R,4R)-1-benzyl-4-methyl-piperidine-3-yl]-1,6-dihydropyrazolo[3,4-d]pyrrolo[2,3-b]-pyridine-3-yl}-N'-methylethanediamide and N-{1-[(3S,4S)-1-benzyl-4-methyl-piperidine-3-yl]-1,6-dihydropyrazolo[3,4-d]pyrrolo[2,3-b]-pyridine-3-yl}-N'-methylethanediamide (150 mg) in EtOH (3 mL) was added 20% Pd(OH)₂ on carbon (71 mg), and the mixture was stirred at 50°C for 5 hours under hydrogen atmosphere. The mixture was cooled to ambient temperature, and was filtered through a pad of Celite. The filtrate was evaporated in vacuo to afford a mixture (126 mg) of N-methyl-N'-{1-[(3R,4R)-4-methyl-piperidine-3-yl]-1,6-dihydropyrazolo[3,4- d]pyrrolo[2,3-b]-pyridine-3-yl}ethanediamide and N-methyl-N'-{1-[(3S,4S)-4-methylpiperidine-3-yl]-1,6-dihydropyrazolo[3,4-d]pyrrolo[2,3-b]-pyridine-3- yl}ethanediamide as a white solid.

The compound (s) in Preparations 6-1 to 6-19 shown in the following Table were produced using the corresponding starting materials in a similar manner to that of Preparation 6.

### Preparation 7

A mixture of 8-[(3R,4R)-1-benzyl-4-methyl-piperidine-3-yl]-3-(3,4-dimethoxybenzyl)-6-methyl-6,8-dihydrodiimidazo[4,5-b:4',5'-d]pyridine-7(3H)-one and 8-[(3S,4S)-1-benzyl-4-methylpiperidine-3-yl]-3-(3,4-dimethoxybenzyl)-6-methyl-6,8-dihydrodiimidazo[4,5-b:4',5'-d]pyridine-7(3H)-one (45 mg) was dissolved in TFA (0.5 mL). The reaction mixture was stirred at ambient temperature for 48 hours. The mixture was concentrated in vacuo to afford a mixture of 8-[(3R,4R)-1-benzyl-4-methylpiperidine-3-yl]-6-methyl-6,8-dihydro-diimidazo[4,5-b:4',5'-d]pyridine-7(3H)-one trifluoroacetate and 8-[(3S,4S)-1-benzyl-4-methylpiperidine-3-yl]-6-methyl-6,8-dihydrodiimidazo[4,5-b:4',5'-d]pyridine-7(3H)-one trifluoroacetate (41 mg) as a brown solid.

The compound(s) in Preparations 7-1 and 7-2 shown in the following Table were produced using the corresponding starting compounds in a similar manner to that of Preparation 7.

### Preparation 8

To a solution of tert-butyl 4-[3-{[(methylamino)(oxo)-acetyl]amino}pyrazolo[3,4-d]pyrrolo[2,3-b]pyridine-1(6H)-yl]piperidine-1-carboxylate (150 mg) in dioxane (1.5 mL) was added 4M HCl/dioxane (3 mL). The reaction mixture was stirred at ambient temperature for 2 hours. The mixture was evaporated in vacuo, and the residue was washed with diisopropyl ether to afford N-methyl-N'-(1-piperidine-4-yl-1,6-dihydropyrazolo[3,4-d]-pyrrolo[2,3-b]pyridine-3-yl)ethanediamide hydrochloride (129 mg) as a white solid.

The compound(s) in Preparations 8-1 to 8-11 shown in the following Table were produced using the corresponding starting materials in a similar manner to that of Preparation 8.

### Preparation 9

The compound(s) in Preparations 9-1 to 9-9 shown in the following Table were produced using the corresponding starting materials in a similar manner to that of Example 4.

### Preparation 10

To a solution of methyl 5-chloropyrazine-2-carboxylate (500 mg) in MeOH (5 mL) was added 28% NH₃ aqueous solution (5 mL), and the mixture was stirred at ambient temperature. The precipitated solid was collected by filtration and washed with diisopropyl ether to afford 5-chloropyrazine-2-carboxamide (303 mg) as a solid.

The compound(s) in Preparations 10-1 to 10-3 shown in the following Table were produced using the corresponding starting materials in a similar manner to that of Preparation 10.

### Preparation 11

The compound(s) in Preparations 11-1 shown in the following Table were produced using the corresponding starting materials in a similar manner to that of Example 2.

### Preparation 12

A solution of 4-{[(3R,4R)-1-benzyl-4-methylpiperidine-3-yl]amino}-1H-pyrrolo[2,3-b]pyridine-5-carbonitrile and 4-{[(3S,4S)-1-benzyl-4-methylpiperidine-3-yl]amino}-1H-pyrrolo[2,3-b]pyridine-5-carbonitrile (2.4 g) and 50% aqueous hydroxylamine in EtOH was refluxed for 6 hours. The flask was cooled to ambient temperature. The mixture was evaporated in vacuo to remove EtOH. The residue was purified by silica gel column chromatography (chloroform:MeOH = 100:0 to 88:12) to afford a mixture (2.26 g) of 4-{[(3R,4R)-1-benzyl-4-methylpiperidine-3-yl]amino}-N'-hydroxy-1H-pyrrolo[2,3-b]pyridine-5-carboxy-imidamide and 4-{[(3S,4S)-1-benzyl-4-methylpiperidine-3-yl]-amino}-N'-hydroxy-1H-pyrrolo[2,3-b]pyridine-5-carboxy-imidamide as a white solid.

The compounds in Preparation 12-1 shown in the following Table were produced using the corresponding starting materials in a similar manner to that of Preparation 12.

### Preparation 13

To a solution of 1-cyclohexyl-6-{[2-(trimethylsilyl)-ethoxy]methyl}-1,6-dihydropyrazolo[3,4-d]pyrrolo[2,3-b]-pyridine-3(2H)-one (272 mg) in DMF (3 mL) was added NaH (60% dispersion in oil, 34 mg) at 0°C. The mixture was stirred at ambient temperature for 30 minutes and was added iodomethane (88 µL). The mixture was stirred at ambient temperature for 15 minutes. The mixture was added saturated NaHCO₃ aqueous solution (10 mL) and was extracted with EtOAc, the organic layer was washed with brine, dried over anhydrous Na₂SO₄, and filtered. The filtrate was evaporated in vacuo. The residue was purified by silica gel column chromatography (EtOAc:n-hexane) to afford 1-cyclohexyl-2-methyl-6-{[2-(trimethylsilyl)ethoxy]methyl}-1,6-dihydro-pyrazolo[3,4-d]pyrrolo[2,3-b]pyridine-3(2H)-one (70.7 mg) and 1-cyclohexyl-3-methoxy-6-{[2-(trimethylsilyl)ethoxy]methyl}-1,6-dihydropyrazolo[3,4-d]pyrrolo[2,3-b]pyridine (201 mg).

The compound(s) in Preparations 13-1a to 13-3 shown in the following Table were produced using the corresponding starting materials in a similar manner to that of Preparation 13.

### Preparation 14

To a solution of ethyl 4-chloro-1H-pyrrolo[2,3-b]-pyridine-5-caboxylate (2 g) in DMF (20 mL) was added NaH (60% dispersion in oil, 427 mg) at 5°C. After the reaction mixture was stirred at ambient temperature for 1 hour, [2-(chloromethoxy)ethyl]-(trimethyl)silane (1.72 mL) was added, and the mixture was stirred at ambient temperature for additional 2 hours. After the reaction mixture was diluted with EtOAc (50 mL), the solution was washed with saturated NaHCO₃ aqueous solution (50 mL) and brine (50 mL) successively. After the organic layer was dried over anhydrous MgSO₄, filtered and evaporated in vacuo. The residue was purified by silica gel column chromatography (n-hexane) to afford ethyl 4-chloro-1-{[2-(trimethylsilyl)ethoxy]methyl}-1H-pyrrolo[2,3-b]pyridine-5-caboxylate (2. 91 g) as a colorless viscous liquid.

### Preparation 15

To a mixture of (3R,4S)-1-benzyl-N-(2,4-dimethoxybenzyl)-3-methylpiperidine-4-amine and (3S,4R)-1-benzyl-N-(2,4-dimethoxybenzyl)-3-methylpiperidine-4-amine in dichloroethane (74 mL) were added TEA (5.8 mL) and TFAA (2.2 mL) at 4°C. The reaction mixture was stirred at the same temperature for 2 hours. The mixture was extracted with chloroform. The organic layer was washed with water, dried over anhydrous MgSO₄, filtered. The filtrate was evaporated in vacuo. The residue was purified by silica gel column chromatography (EtOAc:n-hexane = 1:4) to afford a mixture of N-[(3R,4S)-1-benzyl-3-methylpiperidine-4-yl]-N-(2,4-dimethoxybenzyl)-2,2,2-trifluoroacetamide and N-[(3S,4R)-1-benzyl-3-methylpiperidine-4-yl]-N-(2,4-dimethoxybenzyl)-2,2,2-trifluoroacetamide (4.1 g) as a white amorphous solid.

### Preparation 16 and 16-1

A mixture of 4-{[(3R,4R)-1-benzyl-4-methylpiperidine-3-yl]amino}-N'-hydroxy-1H-pyrrolo[2,3-b]-pyridine-5-carboxyimidamide and 4-{[(3S,4S)-1-benzyl-4-methylpiperidine-3-yl]amino}-N'-hydroxy-1H-pyrrolo[2,3-b]-pyridine-5-carboxyimidamide (500 mg) was dissolved in DMF (5 mL). The mixture was added pyridine (0.32 mL) and 2-ethylhexyl chlorocarbonate (0.77 mL) under ice-cooling. The mixture was stirred for 1 hour and was added water to quench the reaction. The reaction mixture was extracted with chloroform. The organic layer was dried over anhydrous MgSO₄ and filtered. The filtrate was evaporated in vacuo to remove chloroform. The residue was dissolved in DMF (5 mL), and 1,4-diazabicyclo-[2,2,2]octane (20 mg) was added. The mixture was stirred at 150°C for 1 hour under N₂ atmosphere. The reaction mixture was cooled to ambient temperature The mixture was purified by silica gel column chromatography (chloroform:MeOH) to afford a mixture of 1-[(3S,4R)-1-benzyl-4-methylpiperidine-3-yl]-1,6-dihydropyrazolo[3,4-d]pyrrolo[2,3-b]pyridine-3-amine and 1-[(3R,45)-1-benzyl-4-methylpiperidine-3-yl]-1,6-dihydropyrazolo[3,4-d]pyrrolo[2,3-b]pyridine-3-amine (230mg) and a mixture of (2S)-2-ethylhexyl{1-[(3S,4R)-1-benzyl-4-methylpiperidine-3-yl]-1,6-dihydropyrazolo[3,4-d]pyrrolo[2,3-b]pyridine-3-yl}carbamate,(2R)-2-ethylhexyl{1-[(3S,4R)-1-benzyl-4-methylpiperidine-3-yl]-1,6-dihydropyrazolo[3,4-d]-pyrrolo[2,3-b]pyridine-3-yl}carbamate, (2S)-2-ethylhexyl{1-[(3R,4S)-1-benzyl-4-methylpiperidine-3-yl]-1,6-dihydropyrazolo[3,4-d]pyrrolo[2,3-b]pyridine-3-yl}-carbamate and (2R)-2-ethylhexyl{1-[(3R,4S)-1-benzyl-4-methylpiperidine-3-yl]-1,6-dihydropyrazolo[3,4-d]pyrrolo[2,3-b]pyridine-3-yl}carbamate (50 mg).

### Preparation 17

To a solution of 5-bromo-2-thiophenecarboxylic acid (5 g) and cesium carbonate (10.2 g) in DMF (50 mL) was added iodomethane (5.14 g) dropwise at ambient temperature. The mixture was stirred at ambient temperature for 12 hours. The reaction mixture was added water and extracted with chloroform. The organic layer was washed with water and brine, dried over anhydrous MgSO₄ and filtered. The filtrate was evaporated in vacuo. The residue was purified by silica gel column chromatography (n-hexane:EtOAc = 100:0 to 80:20) to afford methyl 5-bromo-2-thiophene-carboxylate (5.42 g) as a white powder.

### Preparation 18

To a suspension of 4-Chloro-N'-hydroxy-1H-pyrrolo[2,3-b]pyridine-5-carboxyimidamide (3.0 g) in THF (60 mL)was added pyridine (3.5 mL) and ethyl chloroglyoxylate (2.1 mL) under ice-cooling. The reaction mixture was stirred at ambient temperature for 1 hour. To the reaction mixture were added EtOAc and water. The solution was extracted with a mixture solvent of THF and EtOAc, and the organic layer was washed with brine. The organic layer was dried over anhydrous MgSO₄ and filtered, and the filtrate was evaporated in vacuo. The residue was dissolved in DMA (30 mL), and the solution was stirred at 140°C for 1 hour. The reaction mixture was cooled to ambient temperature and was added water (200 mL). The reaction mixture was stirred at ambient temperature for 20 minutes. The precipitated solid was collected by filtration and dried under reduced pressure to afford ethyl 3-(4-chloro-1H-pyrrolo[2,3-b]-pyridine-5-yl)-1,2,4-oxaziazole-5- carboxylate (2.7 g) as a light brown solid.

### Preparation 19

A solution of methyl 5-bromothiophene-2-carboxylate (1.00g), pyrrolidine-2-one (462 mg), N,N'-dimethylethane-1,2-diamine (40 mg), copper (I) iodide (43 mg) and Na₂CO₃ (1.38 g) in dioxane (5 mL) was stirred in a sealed tube at 150°C for 1 hour in N₂ atmosphere under microwave irradiation. The flask was cooled to ambient temperature. The reaction mixture was diluted EtOAc and filtered. The filtrate was washed with water and brine, dried over anhydrous MgSO₄ and filtered. The filtrate was evaporated in vacuo. The residue was purified by silica gel column chromatography (n-hexane:EtOAc = 90:10 to 60:40) to afford methyl 5-(2-oxopyrrolidine-1-yl)thiophene-2-carboxylate (243 mg) as a pale brown powder.

### Preparation 20

The compound in Preparation 20 shown in the following Table was produced using the corresponding starting materials in a similar manner to that of Example 11.

### Preparation 21

To a solution of a mixture of 4-{[(3R,4R)-1-benzyl-4-methylpiperidine-3-yl]amino}-6-[(3,4-dimethoxybenzyl)amino]-5-nitronicotinic acid and 4-{[(3S,4S)-1-benzyl-4-methylpiperidine-3-yl]amino}-6-[(3,4-dimethoxybenzyl)-amino]-5-nitronicotinic acid (635 mg) was added DPPA (0. 511 mL). The reaction mixture was stirred at 120°C for 1.5 hours. The reaction mixture was extracted with chloroform. The organic layer was washed with saturated NaHCO₃ aqueous solution and brine and was dried over anhydrous MgSO₄ and filtered, the filtrate was evaporated in vacuo. The residue was purified by silica gel column chromatography (chloroform: MeOH = 100:0 to 90:10) to afford a mixture (510 mg) of 1-[(3R,4R)-1-benzyl-4-methylpiperidine-3-yl]-6-[(3,4-dimethoxybenzyl)amino]-7-nitro-1,3-dihydro-2H-imidazo[4,5-c]pyridine-2-one and 1-[(3S,4S)-1-benzyl-4-methylpiperidine-3-yl]-6-[(3,4-dimethoxybenzyl)amino]-7-nitro-1,3-dihydro-2H-imidazo[4,5-c]pyridine-2-one as a brown amorphous solid.

The compound(s) in Preparations 21-1 to 21-2 shown in the following Table were produced using the corresponding starting materials in a similar manner to that of Preparation 21.

### Preparation 22

To a solution of methyl 5-(hydroxymethyl)thiophene-2-carboxylate (455 mg) and pyridine (418 mg) in DMF (5 mL) was added triisopropylsilyl chloride (764 mg) under ice-cooling. The reaction mixture was stirred at 50°C for overnight. The reaction mixture was cooled to ambient temperature and was added water. The mixture was extracted with EtOAc. The organic layer was washed with water and brine, dried over anhydrous MgSO₄ and filtered. The filtrate was evaporated in vacuo, and the residue was dissolved in MeOH (20 mL). To the solution was added 1M NaOH aqueous solution (10 mL) at ambient temperature, and the mixture was stirred at 60°C for 3 hours. The reaction mixture was cooled to ambient temperature and was neutralized by 1M HCl aqueous solution and extracted with EtOAc. The organic layer was washed with water and brine, dried over anhydrous MgSO₄ and filtered. The filtrate was evaporated in vacuo. The residue was purified by silica gel column chromatography (n-hexane:EtOAc = 90:10 to 50:50) to afford 5-{[(triisopropylsilyl)oxy]methyl}thiophene-2-carboxylic acid (625 mg) as a white solid.

### Preparation 23

To a solution of a mixture of 7-amino-1-[(3R,4R)-1-benzyl-4-methylpiperidine-3-yl]-6-[(3,4-dimethoxybenzyl)-amino]-1,3-dihydro-2H-imidazo[4,5-c]pyridine-2-one and 7-amino-1-[(3S,4S)-1-benzyl-4-methylpiperidine-3-yl]-6-[(3, 4-dimethoxybenzyl)amino]-1,3-dihydro-2H-imidazo[4,5-c]-pyridine-2-one (100 mg) in ethyl orthoformate (1. 98 mL) was added concentrated HCl aqueous solution (50 µL) under ice-cooling, and the reaction mixture was stirred at ambient temperature for 16 hours. To the mixture was added EtOAc, and the precipitate was collected by filtration to afford a mixture of 8-[(3R,4R)-1-benzyl-4-methylpiperidine-3-yl]-3-(3,4-dimethoxybenzyl)-6,8-dihydrodiimidazo[4,5-b:4',5'-d]pyridine-7(3H)-one hydrochloride and 8-[(3S,4S)-1-benzyl-4-methylpiperidine-3-yl]-3-(3,4-dimethoxybenzyl)-6,8-dihydrodiimidazo[4,5-b:4',5'-d]pyridine-7(3H)-one hydrochloride (100 mg) as a white solid.

### Preparation 24

A solution of methyl 5-formylthiophene-2-carboxylate (486 mg) in MeOH (20 mL) was cooled to 0°C, and was added sodium borohydride (108 mg) slowly. The reaction mixture was stirred at 0°C for 30 minutes. To the reaction mixture was added saturated NH₄Cl aqueous solution (20 mL) and stirred. The mixture was concentrated to approximately 25 mL under reduced pressure and extracted with EtOAc. The organic layer was washed with water and brine, dried over anhydrous MgSO₄ and filtered. The filtrate was evaporated in vacuo to afford methyl 5-(hydroxymethyl)thiophene-2-carboxylate (472 mg) as a colorless oil.

### Preparation 25

To a solution of a mixture of 1-[(3R,4R)-1-benzyl-4-methylpiperidine-3-yl]-6-[(3,4-dimethoxybenzyl)amino]-7-nitro-1,3-dihydro-2H-imidazo[4,5-c]pyridine-2-one and 1-[(3S, 4S)-1-benzyl-4-methylpiperidine-3-yl]-6-[(3,4-dimethoxybenzyl)amino]-7-nitro-1,3-dihydro-2H-imidazo[4,5-c]pyridine -2-one (120 mg) in EtOH (4 mL) and water (1 mL) were added iron powder (38 mg) and NH₄Cl (6mg). The reaction mixture was stirred at 120°C for 2 hours. The mixture was cooled to ambient temperature and was filtered through Celite to remove precipitate. The filtrate was evaporated in vacuo. The residue was extracted with a mixed solvent (chloroform:MeOH = 4:1) and washed with water. The organic layer was dried over anhydrous MgSO₄ and filtered. The filtrate was evaporated in vacuo to afford a mixture (100 mg) of 7-amino-1-[(3R,4R)-1-benzyl-4-methylpiperidine-3-yl]-6-[(3,4-dimethoxybenzyl)amino]-1,3-dihydro-2H-imidazo[4,5-c]pyridine-2-one and 7-amino-1-[(3S,4S)-1-benzyl-4-methylpiperidine-3-yl]-6-[(3,4-dimethoxybenzyl)amino]-1,3-dihydro-2H-imidazo[4,5-c]-pyridine-2-one as a brown amorphous solid.

### Preparation 26

A mixture of acetic anhydride (700 µL) and formic acid (2672 µL) was stirred at 60°C for 2 hours. The flask was cooled to ambient temperature. Then a mixture of racemic tert-butyl 4-{[2-amino-5-(ethoxycarbonyl)-3-nitropyridine-4-yl]amino}azepan-1-carboxylate (200 mg) and DCM (1 mL) was added dropwise. The resulting solution was stirred at 50°C for 2 hours. The reaction mixture was cooled to ambient temperature and was evaporated in vacuo. To the residue was added EtOH (2 mL), THF (1 mL) and water (1 mL),further and was added iron powder (131 mg) and NH₄Cl (12.6 mg). The reaction mixture was stirred at 120°C for 2 hours. The flask was cooled to ambient temperature, The reaction mixture was added 1M HCl aqueous solution (10 mL) and was stirred at ambient temperature for 10 minutes. The reaction mixture was neutralized by saturated NaHCO₃ aqueous solution and extracted with EtOAc. The organic layer was dried over anhydrous MgSO₄ and filtered. The filtrate was evaporated in vacuo. The residue was purified by silica gel column chromatography (chloroform:MeOH) to afford racemic ethyl 7-{[1-(tert-butoxycarbonyl)azepan-4-yl]amino}-3H-imidazo[4,5-b]pyridine-6-caboxylate (65 mg) as a white solid.

The compound in Preparation 26-1 shown in the following Table was produced using the corresponding starting materials in a similar manner to that of Preparation 26.

### Preparation 27

To a solution of 4-{[(1S,2R)-2-methylcyclohexyl]amino}-1H-pyrrolo[2,3-b]pyridine-5-carboxamide (150 mg) in t-BuOH (1.5 mL) was added Pyridinium tribromide (528 mg). The reaction mixture was stirred at 40°C for 3 hours. The reaction mixture was evaporated in vacuo, the residue was extracted with EtOAc. The organic layer was washed with water, dried over anhydrous MgSO₄ and filtered. The filtrate was evaporated in vacuo. The yellow residue was used for the subsequent reaction without purification.

### Preparation 28

To a mixture of cis-1-(diphenylmethyl)pyrrolidine-2,5-dicarbonitrile (500 mg), triethylsilane (607 mg) and anisole (564 mg) was added TFA (5 mL) under ice-cooling. The mixture was stirred at ambient temperature for 1 hour and evaporated in vacuo. The residue was purified by silica gel column chromatography (chloroform: MeOH = 99:1 to 80:20) to afford cis-pyrrolidine-2,5-dicarbonitrile (209 mg) as a colorless oil.

The compound in Preparation 28-1 shown in the following Table was produced using the corresponding starting materials in a similar manner to that of Preparation 28.

### Preparation 29

To a sealed tube reaction vessel (50mL) with racemic tert-butyl 4-{[2-chloro-5-(ethoxycarbonyl)-3-nitropyridine-4-yl]amino}azepan-1-carboxylate (2 g), 28% NH₃ aqueous solution(2.4 mL) and EtOH (21 mL) were added, and the tube was sealed airtightly. The mixture in the reaction vessel was stirred at 90°C for 2 hours. The reaction mixture was cooled to 0°C. The reaction mixture was diluted with MeOH (20 mL) and evaporated in vacuo. The residue was recrystallized from mixed solvent (EtOAc and n-hexane) to afford racemic tert-butyl 4-{[2-amino-5-(ethoxycarbonyl)-3-nitropyridine-4-yl]amino}azepan-1-carboxylate (1.38 g) as a yellow solid.

The compound in Preparation 29-1 shown in the following Table was produced using the corresponding starting materials in a similar manner to that of Preparation 29.

### Preparation 30

A mixture of ethyl 4,6-dichloro-5-nitronicotinate (3.05 g), racemic tert-butyl 4-aminoazepan-1-caboxylate (2.47 g), DIPEA (4 mL) and i-PrOH (7.625 mL) was stirred at 90°C for 30 minutes under microwave irradiation. The reaction mixture was cooled to ambient temperature. The mixture was diluted with EtOAc (20 mL), and water (20 mL) was added. The organic layer was extracted, washed with brine, dried over anhydrous MgSO₄, filtered. The filtrate was evaporated in vacuo. The residue was purified by silica gel column chromatography (EtOAc: n-hexane) to afford racemic tert-butyl 4-{[2-chloro-5-(ethoxycarbonyl)-3-nitropyridine-4-yl]amino}azepan-1-carboxylate (3.43 g) as a yellow solid.

The compound in Preparation 30-1 shown in the following Table was produced using the corresponding starting materials in a similar manner to that of Preparation 30.

### Preparation 31

To a solution of ethyl 4,6-dichloro-5-nitronicotinate (500 mg) in NMP (1 mL) were added DIPEA (0.328 mL) and a racemic mixture of (3R,4R)-1-benzyl-4-methylpiperidine-3-amine and (3S,4S)-1-benzyl-4-methylpiperidine-3-amine (385 mg). The reaction mixture was stirred at ambient temperature for 1 hour. To the reaction mixture were added DIPEA (0.328 mL) and 1-(3,4-dimethoxyphenyl)methaneamine (315 mg). The reaction mixture was stirred at 110°C for 2 hours. The mixture was extracted with EtOAc and washed with water. The organic layer was dried over anhydrous MgSO₄ and filtered. The filtrate was evaporated in vacuo. The residue was purified by silica gel column chromatography (EtOAc:n-hexane = 1:2) to afford a mixture (670 mg) of ethyl 4-{[(3R,4R)-1-benzyl-4-methylpiperidine-3-yl]-amino}-6-[(3,4-dimethoxybenzyl)amino]-5-nitronicotinate and ethyl 4-{[(3S,4S)-1-benzyl-4-methylpiperidine-3-yl]amino}-6-[(3,4-dimethoxybenzyl)amino]-5-nitroniootinate as a yellow amorphous solid.

### Preparation 32

A mixture of ethyl 1-benzyl-5,5-difluoro-4-hydroxy-1,2,5,6-tetrahydropyridine-3-carboxylate (2.00 g), LiCl (1.14 g), DMSO (20 mL) and water (1 mL) was stirred at 130°C for 2 hours . The flask was cooled to ambient temperature. The reaction mixture was added water and was extracted with EtOAc. The organic layer was washed with water and brine and dried over anhydrous MgSO₄. The mixture was filtered, and the filtrate was evaporated in vacuo. The afforded residue was reprecipitated with solvent (MeOH, chloroform and n-hexane) to afford 1-benzyl-3,3-difluoropiperidine-4-one (382 mg) as a white solid. Furthermore, the filtrate was evaporated in vacuo, and the residue was purified by silica gel column chromatography (n-hexane: chloroform = 60: 40 to 30:70) to afford 1-benzyl-3,3-difluoropiperidine-4-one (540 mg) as a white solid.

### Preparation 33

The compound in Preparation 33 shown in the following Table was produced using the corresponding starting materials in a similar manner to that of Example 9.

### Preparation 34

To a suspension of LiAlH₄ (237 mg) in THF (10 mL) was added a solution of 1-benzyl-3,3-difluoropiperidine-4-one oxime(750 mg) in THF (10 mL) dropwise under ice-cooling. The reaction mixture was stirred for 10 minutes and was stirred at 70°C for 4 hours. The mixture was cooled to 0°C,then water (0.24 mL), 15% NaOH aqueous solution (0.24 mL) and water (0.71 mL) were added dropwise. The mixture was stirred at ambient temperature for 30 minutes. The reaction mixture was filtered through Celite to remove solid and washed with THF, chloroform and MeOH. The filtrate was evaporated in vacuo to afford 1-benzyl-3,3-difluoropiperidine-4-amine (610 mg) as a white solid.

### Preparation 35

The compound in Preparation 35-1 to 35-2 shown in the following Table was produced using the corresponding starting materials in a similar manner written in patent literature of WO2006/087543.

The abbreviation used in the following table was shown below. Pr=Process No., Ex=Example No., Structure=Chemical structure, Data:Physical data, NMR=¹H-NMR (400MHz,d₆-DMSO), NMR(CDCl₃)=¹H-NMR (400MHz,CDCl₃).In other words,All the "NMR data" show NMR data in d₆-DMSO in a case without the declining in particular. MS:API-ES data or ESI-MS data.

**Table 3**

| Pr | Structure | Pr | Structure |
|---|---|---|---|
| 1-1 | 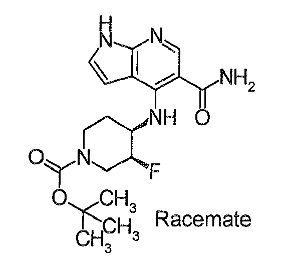 | 1-2 | 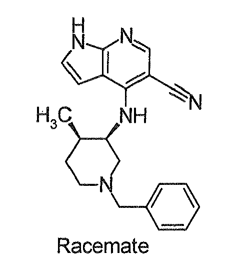 |
| 1-3 | 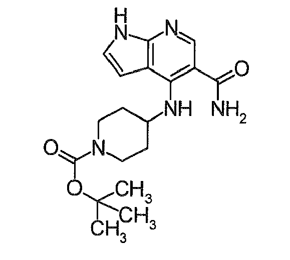 | 1-4 | 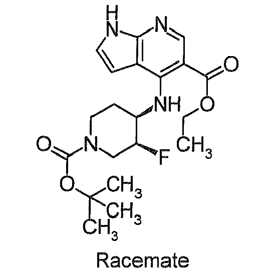 |
| 1-5 | 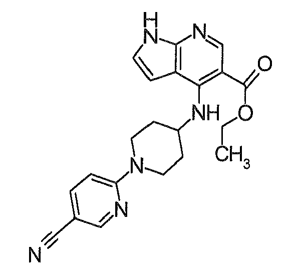 | 1-6 | 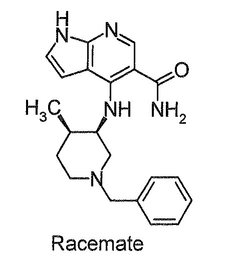 |
| 1-7 | 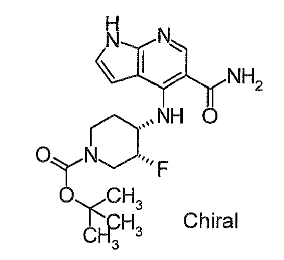 | 1-8 | 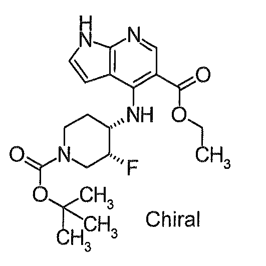 |
| 1-9 | 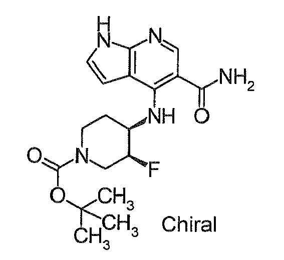 | 1-10 | 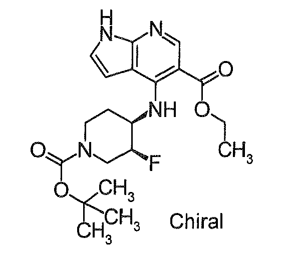 |
| 1-11 | 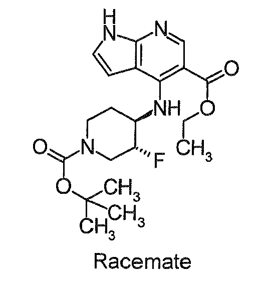 | 1-12 | 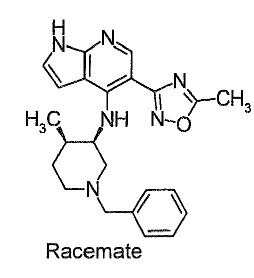 |
| 1-13 | 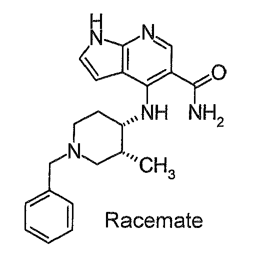 | 1-14 | 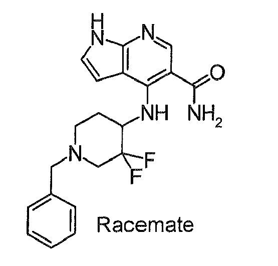 |
| 2-1 | 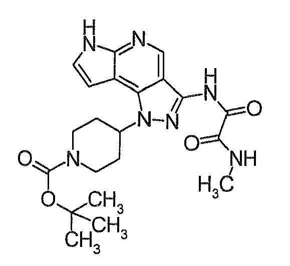 | 2-2 | 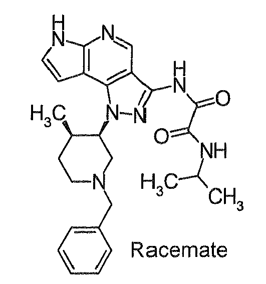 |
| 2-3 | 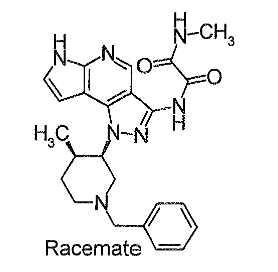 | 2-4 | 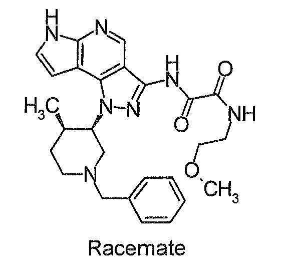 |
| 2-5 | 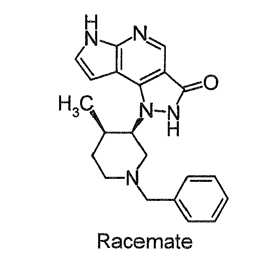 | 2-6 | 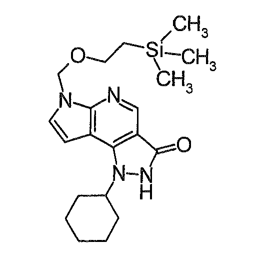 |
| 2-7 | 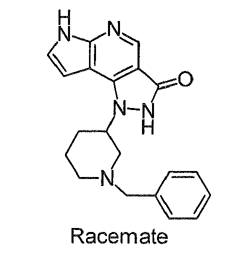 | 2-8 | 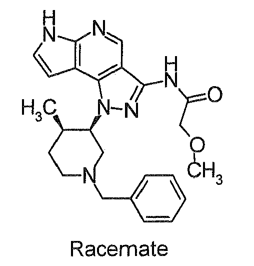 |
| 2-9 | 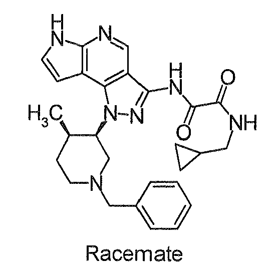 | 3 | 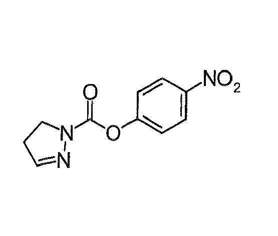 |
| 3-1 | 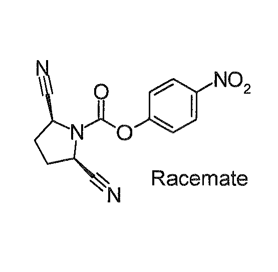 | 3-2 | 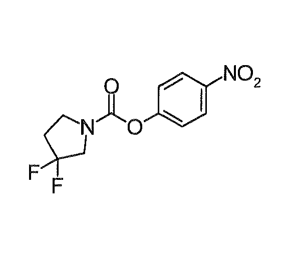 |
| 3-3 | 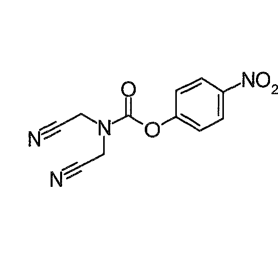 | 3-4 | 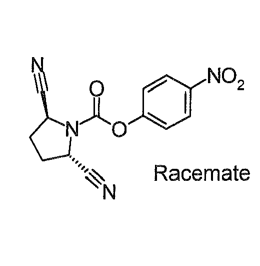 |
| 3-5 | 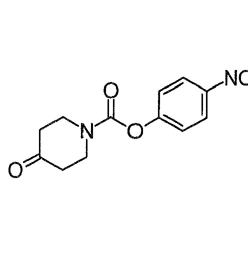 | 3-6 | 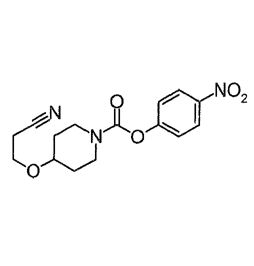 |
| 3-7 | 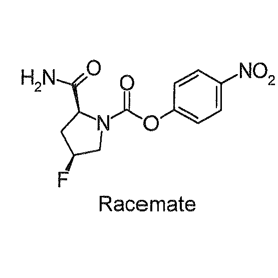 | 4-1 | 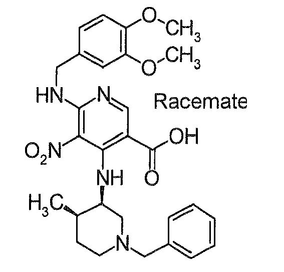 |
| 4-2 | 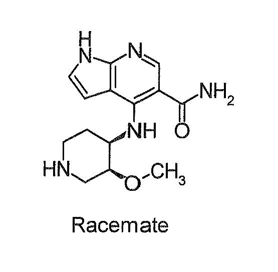 | 4-3 | 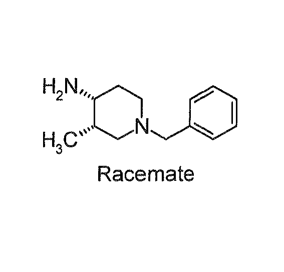 |
| 4-4 | 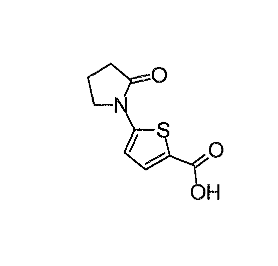 | 4-5 | 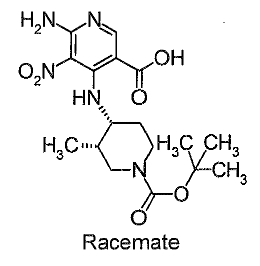 |
| 4-6 | 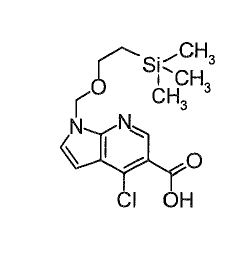 | 4-7 | 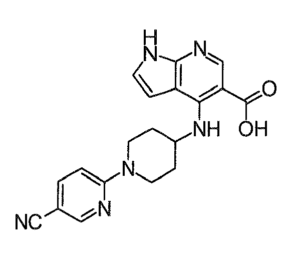 |
| 4-8 | 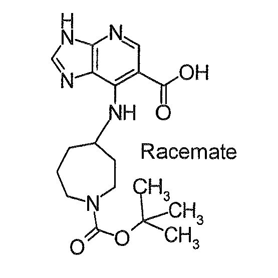 | 4-9 | 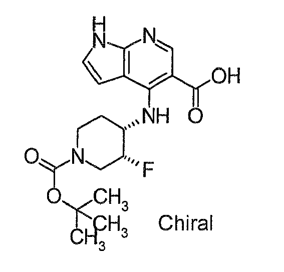 |
| 4-10 | 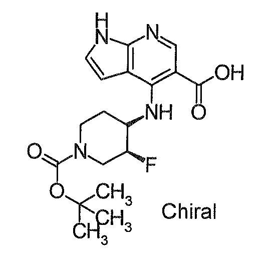 | 4-11 | 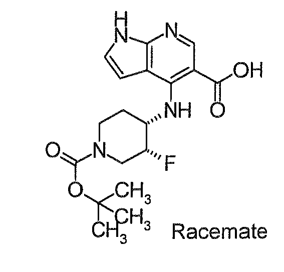 |
| 4-12 | 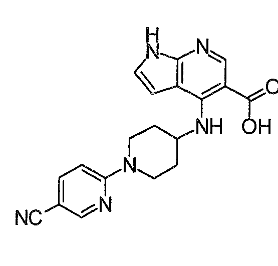 | 5-1 | 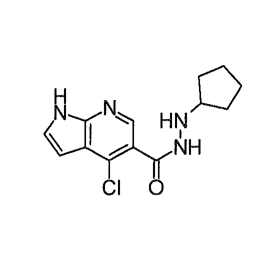 |
| 5-2 | 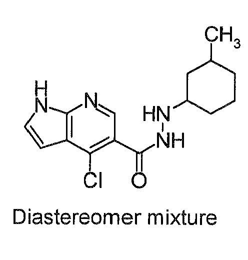 | 5-3 | 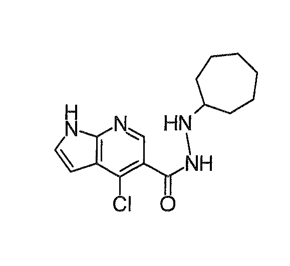 |
| 5-4 | 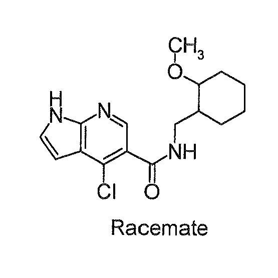 | 5-5 | 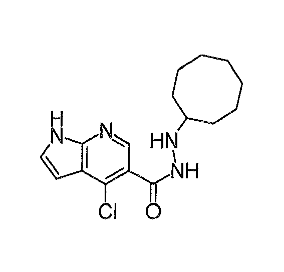 |
| 5-6 | 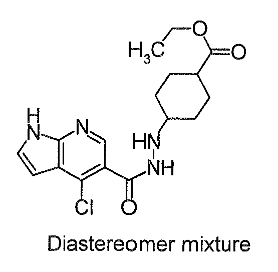 | 5-7 | 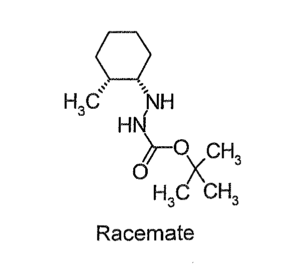 |
| 5-8 | 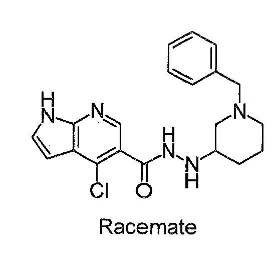 | 5-9 | 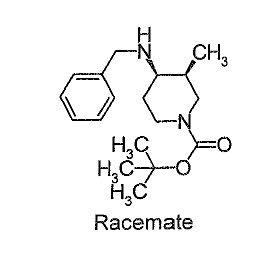 |
| 5-10 | 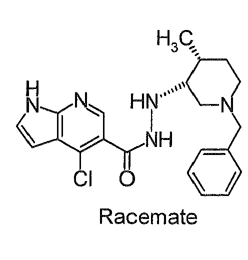 | 5-11 | 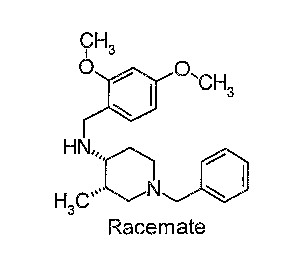 |
| 5-12 | 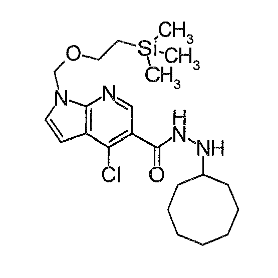 | 5-13 | 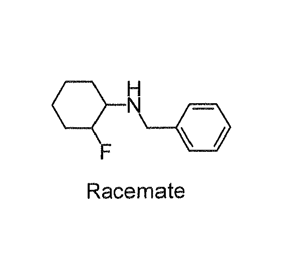 |
| 5-14 | 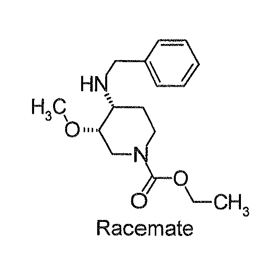 | 6 | 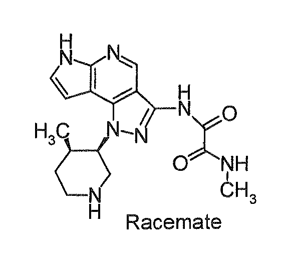 |
| 6-1 | 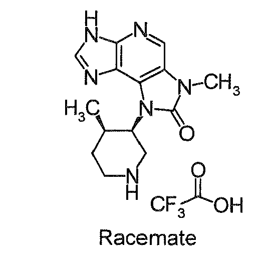 | 6-2 | 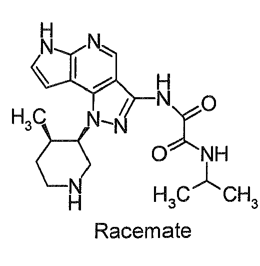 |
| 6-3 | 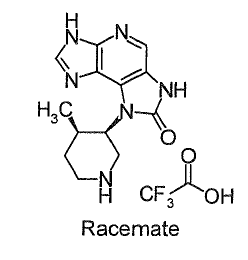 | 6-4 | 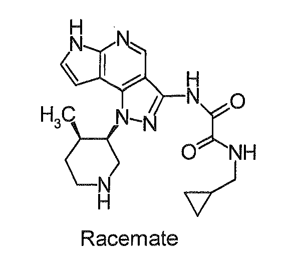 |
| 6-5 | 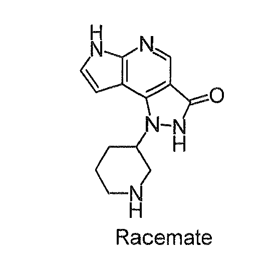 | 6-6 | 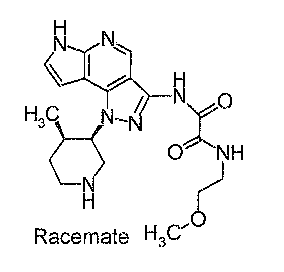 |
| 6-7 | 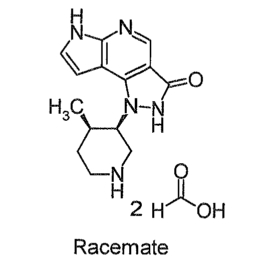 | 6-8 | 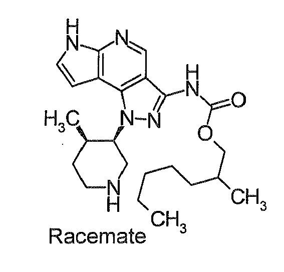 |
| 6-9 | 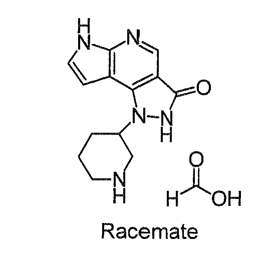 | 6-10 | 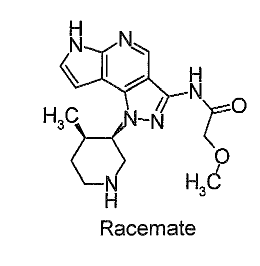 |
| 6-11 | 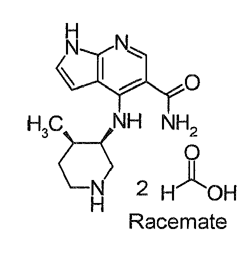 | 6-12 | 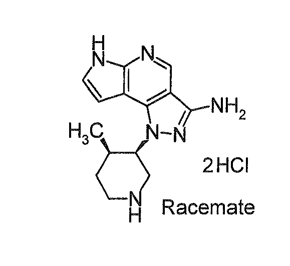 |
| 6-13 | 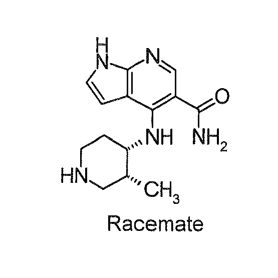 | 6-14 | 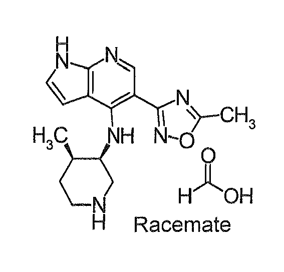 |
| 6-15 | 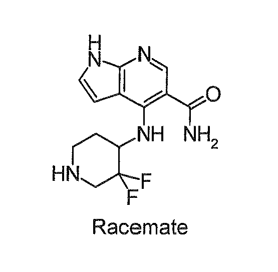 | 6-16 | 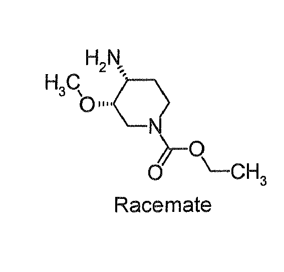 |
| 6-17 | 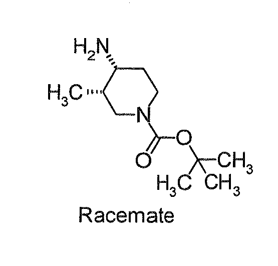 | 6-18 | 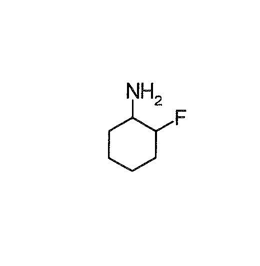 |
| 6-19 | 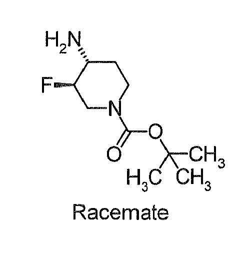 | 7 | 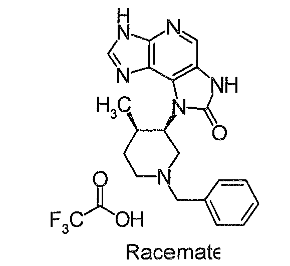 |
| 7-1 | 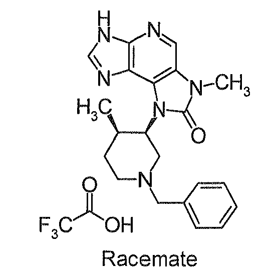 | 7-2 | 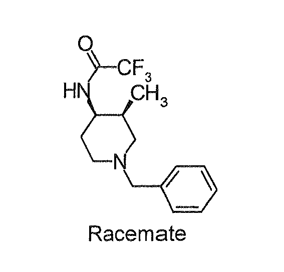 |
| 8 | 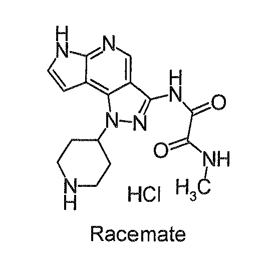 | 8-1 | 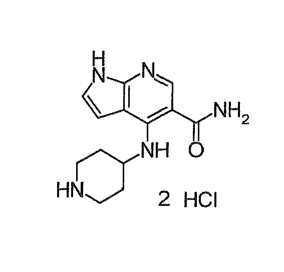 |
| 8-2 | 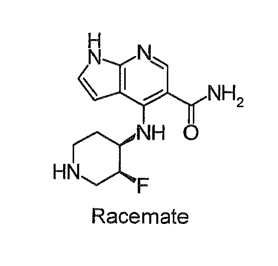 | 8-3 | 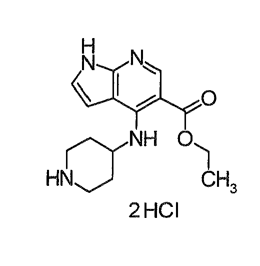 |
| 8-4 | 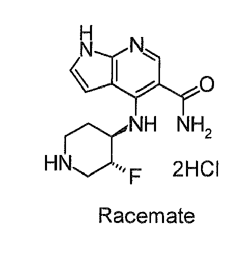 | 8-5 | 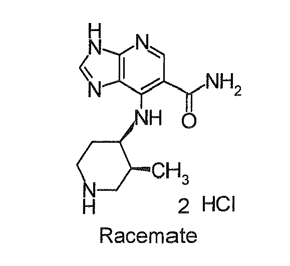 |
| 8-6 | 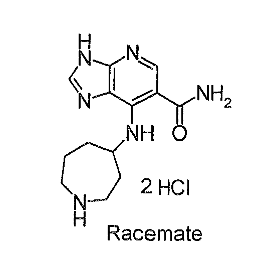 | 8-7 | 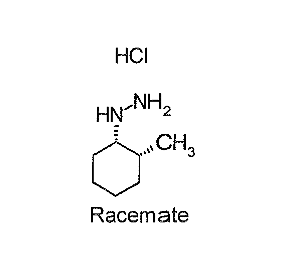 |
| 8-8 | 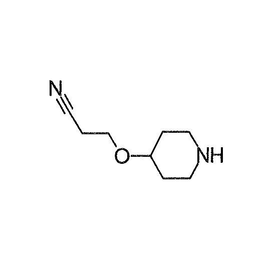 | 8-9 | 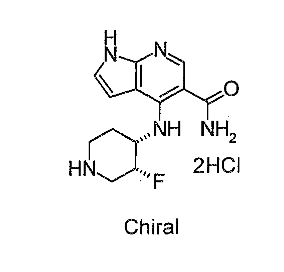 |
| 8-10 | 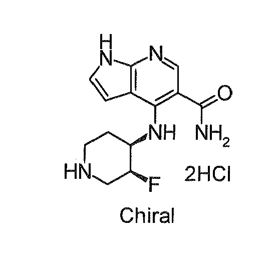 | 8-11 | 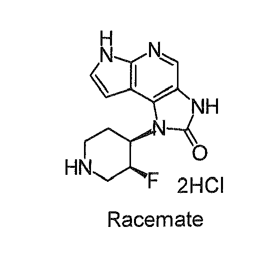 |
| 9-1 | 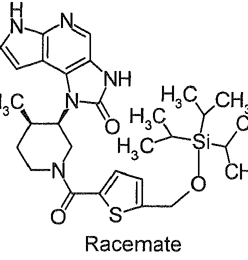 | 9-2 | 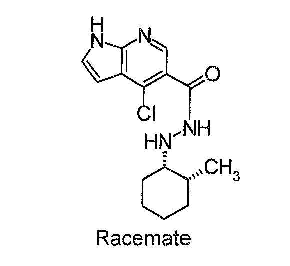 |
| 9-3 | 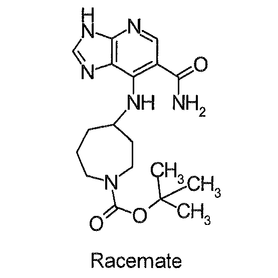 | 9-4 | 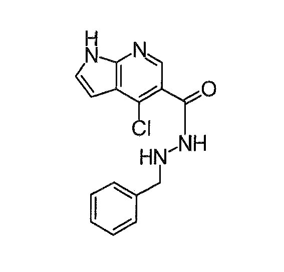 |
| 9-5 | 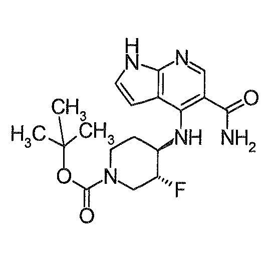 | 9-6 | 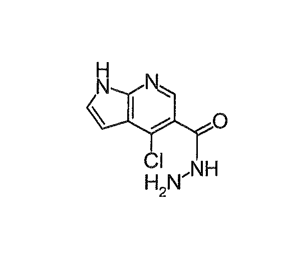 |
| 9-7 | 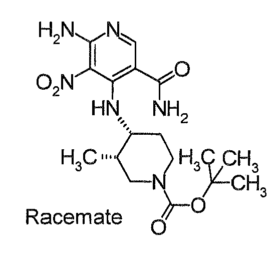 | 9-8 | 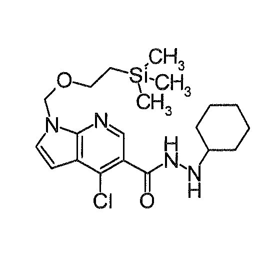 |
| 9-9 | 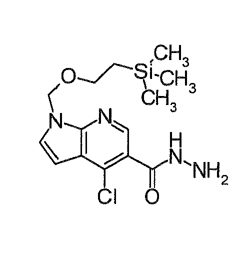 | 10 | 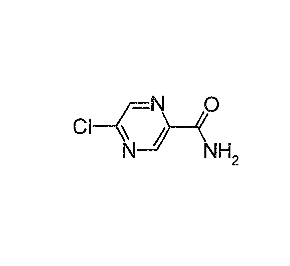 |
| 10-1 | 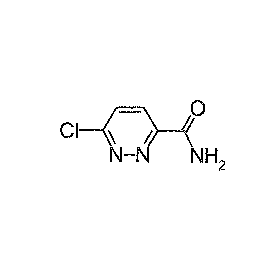 | 10-2 | 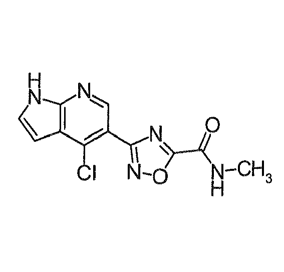 |
| 10-3 | 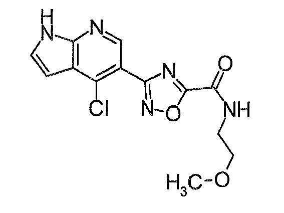 | 11-1 | 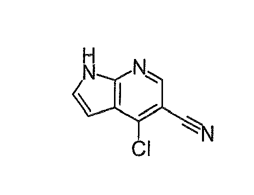 |
| 12 | 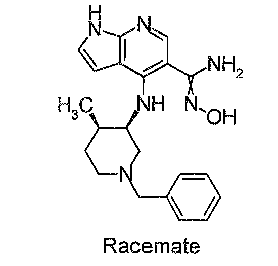 | 12-1 | 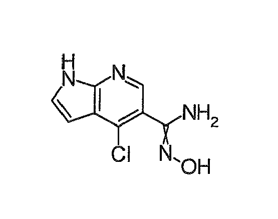 |
| 13 | 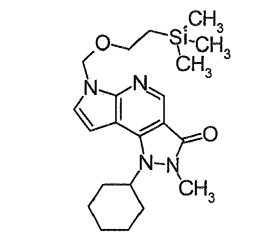 | 13 | 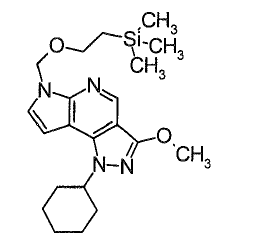 |
| 13-1a | 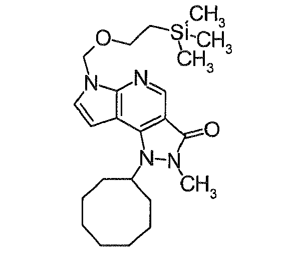 | 13-1b | 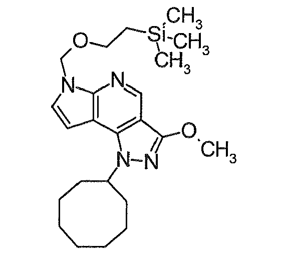 |
| 13-2a | 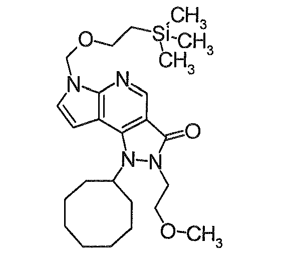 | 13-2b | 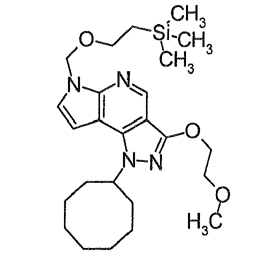 |
| 14 | 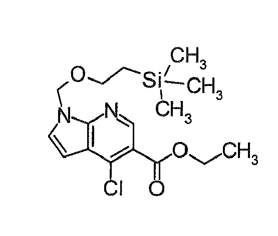 | 15 | 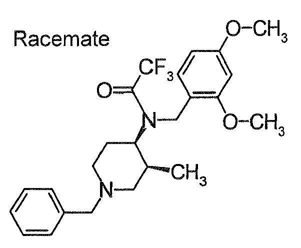 |
| 16 | 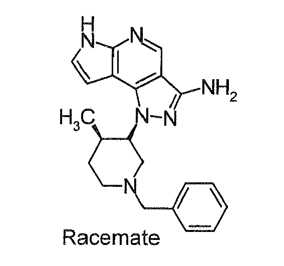 | 16-1 | 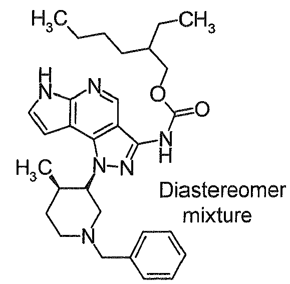 |
| 17 | 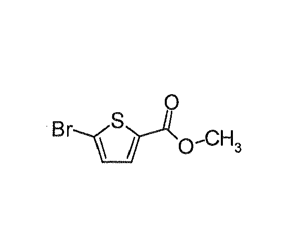 | 18 | 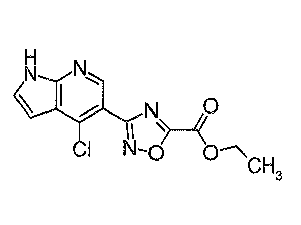 |
| 19 | 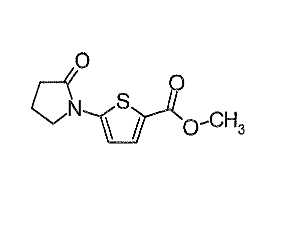 | 20 | 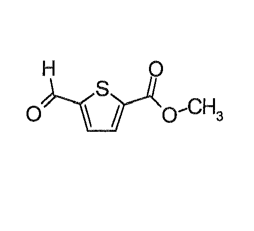 |
| 21 | 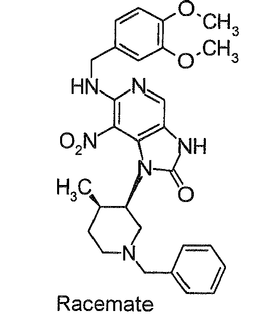 | 21-1 | 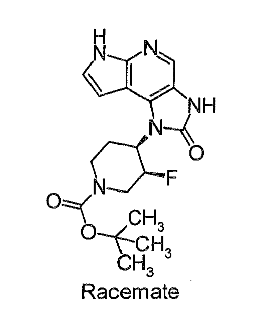 |
| 21-2 | 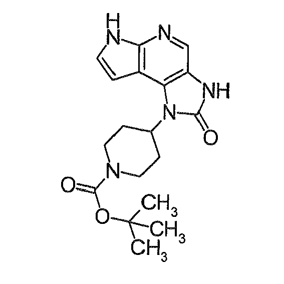 | 22 | 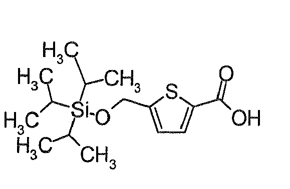 |
| 23 | 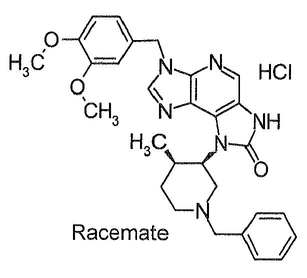 | 24 | 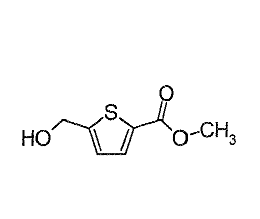 |
| 25 | 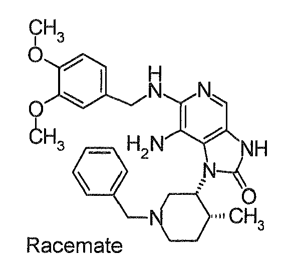 | 26 | 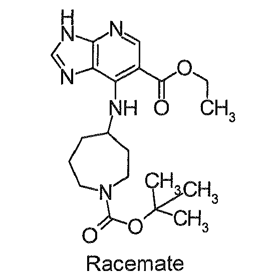 |
| 26-1 | 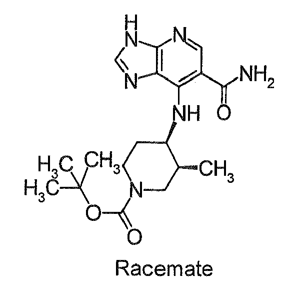 | 27 | 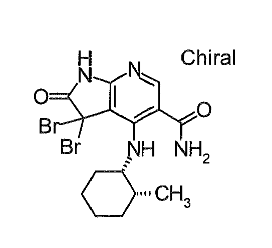 |
| 28 | 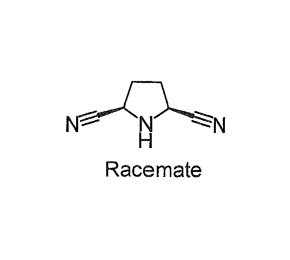 | 28-1 | 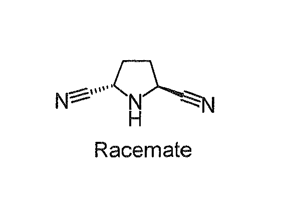 |
| 29 | 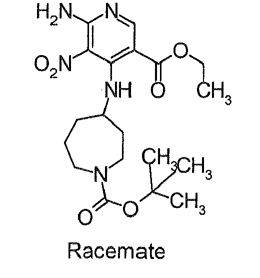 | 29-1 | 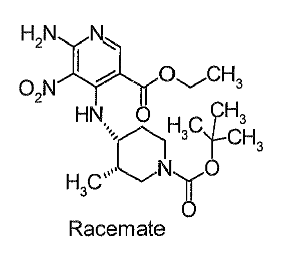 |
| 30 | 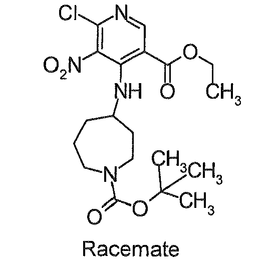 | 30-1 | 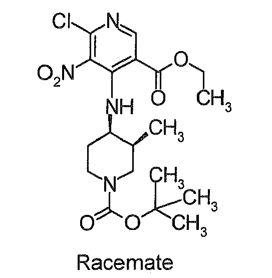 |
| 31 | 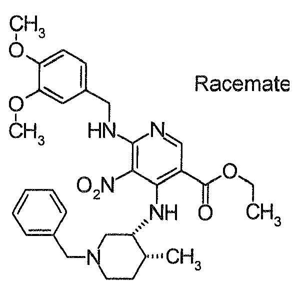 | 32 | 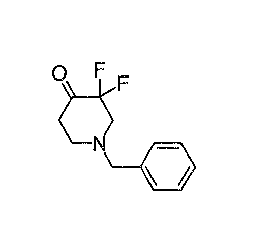 |
| 33 | 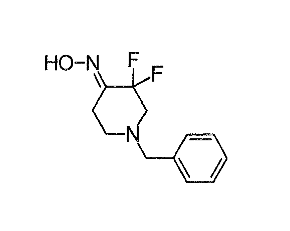 | 34 | 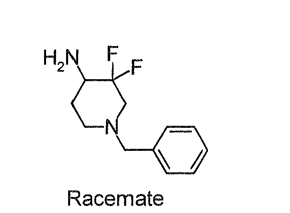 |
| 35 | 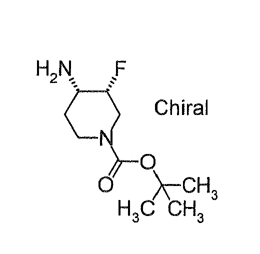 | 35-1 | 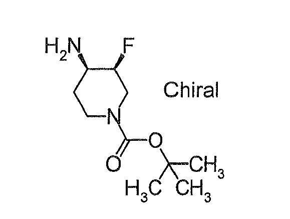 |
| 35-2 | 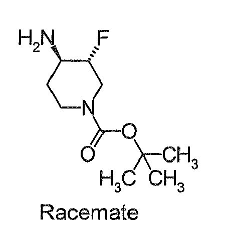 | | |

**Table 4 (contd.)**

| Pr | Data |
|---|---|
| 1-1 | API-ES:378(M+H)⁺ |
| 1-2 | ESI-MS:346(M+H)⁺ |
| 1-3 | API-ES:360(M+H)⁺ |
| 1-4 | ESI-MS:407(M+H)⁺ |
| 1-5 | ESI-MS:391(M+H)⁺ |
| 1-6 | ESI-MS:364(M+H)⁺ |
| 1-7 | ESI-MS:378(M+H)⁺ |
| 1-8 | ESI-MS:407(M+H)⁺ |
| 1-9 | ESI-MS:378(M+H)⁺ |
| 1-10 | ESI-MS:407(M+H)⁺ |
| 1-11 | ESI-MS:407(M+H)⁺ |
| 1-12 | ESI-MS:403(M+H)⁺ |
| 1-13 | ESI-MS:364(M+H)⁺ |
| 1-14 | ESI-MS:408(M+Na)⁺ |
| 2-1 | ESI-MS:464(M+Na)⁺ |
| 2-2 | ESI-MS:474(M+Na)⁺ |
| 2-3 | ESI-MS:446(M+H)⁺ |
| 2-4 | ESI-MS:490(M+H)⁺ |
| 2-5 | ESI-MS:362(M+H)⁺ |
| 2-6 | ESI-MS:387(M+H)⁺ |
| 2-7 | ESI-MS:348(M+H)⁺ |
| 2-8 | ESI-MS:433(M+H)⁺ |
| 2-9 | ESI-MS:486(M+H)⁺ |
| 3 | ESI-MS:258(M+Na)⁺ |
| 3-1 | ESI-MS:309(M+Na)⁺ |
| 3-2 | NMR(CDCl₃):8.27 (2H, d, J=5. 2Hz), 7.31-7.36 (2H,m), 3.72 -4.00(4H,m),2.38-2.54(2H,m) |
| 3-3 | ESI-MS:283(M+Na)⁺ |
| 3-4 | NMR(CDCl₃):2.53-2.74(4H,m),4.79-4.92(2H,m),7.44(2H ,d,J=9.1Hz),8.32(2H,d,J=9.1Hz) |
| 3-5 | NMR(CDCl₃):1.64-1.95(4H,m),1.97-2.26(4H,m),2.77(3H ,d,J=4.8Hz),5.27-5.38(1H,m),6.92(1H,d,J=3.0Hz),7.4 1-7.46(1H,m),8.67(1H,s),8.94-9.04(1H,m),10.78-10.9 0(1H,brs),11.96-12.10(1H,brs) |
| 3-6 | NMR(CDCl₃):1.6,8-1.97(4H,m),2.64(2H,t,J=6.2Hz),3.3 7-3.94(7H,m),7.30(2H,d,J=9.2Hz),8.25(2H,d,J=9.2Hz) |
| 3-7 | NMR(CDCl₃):2.28-3.02(2H,m),3.71-4.14(2H,m),4.53-4. 74(1H,m),5.20-5.65(2H,m),6.00-6.50(1H,m),7.28-7.45 (2H,m),8.16-8.35(2H,m) |
| 4-1 | ESI-MS:536(M+H)⁺ |
| 4-2 | ESI-MS:290(M+H)⁺ |
| 4-3 | ESI-MS:205(M+H)⁺ |
| 4-4 | ESI-MS:212(M+H)⁺ |
| 4-5 | ESI-MS:418(M+Na)⁺ |
| 4-6 | ESI-MS:349(M+Na)⁺ |
| 4-7 | ESI-MS:401(M+Na)⁺ |
| 4-8 | ESI-MS:376(M+H)⁺ |
| 4-9 | ESI-MS:379(M+H)⁺ |
| 4-10 | ESI-MS:379(M+H)⁺ |
| 4-11 | ESI-MS:379(M+H)⁺ |
| 4-12 | API-ES:363(M+H)⁺ |
| 4-1 | ESI-MS:536(M+H)⁺ |
| 4-2 | ESI-MS:349(M+Na)⁺ |
| 4-3 | EST-MS:418(M+Na)⁺ |
| 4-4 | ESI-MS:376(M+H)⁺ |
| 4-5 | API-ES:363(M+H)⁺ |
| 4-6 | ESI-MS:290(M+H)⁺ |
| 4-7 | ESI-MS:401(M+Na)⁺ |
| 4-8 | ESI-MS:212(M+H)⁺ |
| 4-9 | ESI-MS:401(M+H)⁺ |
| 4-10 | ESI-MS:401(M+H)⁺ |
| 4-11 | ESI-MS:401(M+H)⁺ |
| 4-12 | ESI-MS:379(M+H)⁺ |
| 5-1 | ESI-MS:301(M+Na)⁺ |
| 5-2 | ESI-MS:329(M+Na)⁺ |
| 5-3 | ESI-MS:329(M+Na)⁺ |
| 5-4 | ESI-MS:345(M+Na)⁺ |
| 5-5 | ESI-MS:343(M+Na)⁺ |
| 5-6 | ESI-MS:387(M+Na)⁺ |
| 5-7 | ESI-MS:251(M+Na)⁺ |
| 5-8 | ESI-MS:385(M+H)⁺ |
| 5-9 | ESI-MS:305(M+H)⁺ |
| 5-10 | ESI-MS:398(M+H)⁺ |
| 5-11 | ESI-MS:355(M+H)⁺ |
| 5-12 | ESI-MS:451(M+H)⁺ |
| 5-13 | ESI-MS:208(M+H)⁺ |
| 6 | ESI-MS:356(M+H)⁺ |
| 6-1 | ESI-MS:287(M+H)⁺ |
| 6-2 | ESI-MS:384(M+H)⁺ |
| 6-3 | ESI-MS:273(M+H)⁺ |
| 6-4 | ESI-MS:396(M+H)⁺ |
| 6-5 | ESI-MS:258(M+H)⁺ |
| 6-6 | ESI-MS:400(M+H)⁺ |
| 6-7 | ESI-MS:272(M+H)⁺ |
| 6-8 | ESI-MS:427(M+H)⁺ |
| 6-9 | ESI-MS:258(M+H)⁺ |
| 6-10 | ESI-MS:343(M+H)⁺ |
| 6-11 | ESI-MS:271(M+H)⁺ |
| 6-12 | ESI-MS:271(M+H)⁺ |
| 6-13 | ESI-MS:274(M+H)⁺ |
| 6-14 | |
| 6-15 | ESI-MS:318(M+Na)⁺ |
| 6-16 | ESI-MS:203(M+H)⁺ |
| 6-17 | ESI-MS:237(M+Na)⁺ |
| 6-18 | ESI-MS:118(M+H)⁺ |
| 6-19 | ESI-MS:241(M+Na)⁺ |
| 7 | ESI-MS:363(M+H)⁺ |
| 7-1 | ESI-MS:377(M+H)⁺ |
| 7-2 | ESI-MS:301(M+H)⁺ |
| 8 | ESI-MS:342(M+H)⁺ |
| 8-1 | APT-ES:260(M+H)⁺ |
| 8-2 | APT-ES:278(M+H)⁺ |
| 8-3 | API-ES:289(M+H)⁺ |
| 8-4 | API-ES:278(M+H)⁺ |
| 8-5 | ESI-MS:275(M+H)⁺ |
| 8-6 | ESI-MS:275(M+H)⁺ |
| 8-7 | ESI-MS:129(M+H)⁺ |
| 8-8 | ESI-MS:155(M+H)⁺ |
| 8-9 | ESI-MS:278(M+H)⁺ |
| 8-10 | ESI-MS:278(M+H)⁺ |
| 8-11 | ESI-MS:276(M+H)⁺ |
| 9-1 | ESI-MS:568(M+H)⁺ |
| 9-2 | ESI-MS:307(M+H)⁺ |
| 9-3 | ESI-MS:375(M+H)⁺ |
| 9-4 | API-ES:323(M+Na)⁺ |
| 9-5 | ESI-MS:376(M-H)⁻ |
| 9-6 | ESI-MS:233(M+Na)⁺ |
| 9-7 | ESI-MS:417(M+Na)⁺ |
| 9-8 | ESI-MS:423(M+H)⁺ |
| 9-9 | ESI-MS:340(M+H)⁺ |
| 10 | NMR:7.93(1H,brs),8.29(1H,brs),8.86(1H,d,J=1.3Hz),9 .00(1H,d,J=1.3Hz). |
| 10-1 | NMR:6.94(1H,d,J=9.8Hz),7.60(1H,brs),7.79(1H,brs),7 .83(1H,d,J=9.8Hz). |
| 10-2 | ESI-MS:300(M+Na)⁺ |
| 10-3 | ESI-MS:344(M+Na)⁺ |
| 11-1 | ESI-MS:176(M-H)- |
| 12 | ESI-MS:379(M+H)⁺ |
| 12-1 | ESI-MS:211(M+H)⁺ |
| 13 | ESI-MS:423(M+Na)⁺ |
| 13 | ESI-MS:401(M+H)⁺ |
| 13-1 a | ESI-MS:429(M+H)⁺ |
| 13-1 b | ESI-MS:429(M+H)⁺ |
| 13-2 a | ESI-MS:473(M+H)⁺ |
| 13-2 b | ESI-MS:473(M+H)⁺ |
| 13-3 | ESI-MS:527(M+H)⁺ |
| 14 | ESI-MS:377(M+Na)⁺ |
| 15 | ESI-MS:451(M+H)⁺ |
| 16 | ESI-MS:361(M+H)⁺ |
| 16-1 | ESI-MS:517(M+H)⁺ |
| 17 | NMR(CDCl₃):3.87(3H,s),7.07(1H,d,J=4.0Hz),7.55(1H,d ,J=4.0Hz) |
| 18 | ESI-MS:315(M+Na)⁺ |
| 19 | ESI-MS:248(M+Na)⁺ |
| 20 | NMR(CDCl₃):3.94(3H,s),7.73(1H,d,J=3.9Hz),7.84(1H,d ,J=3.9Hz), 9.98 (1H,s) |
| 21 | ESI-MS:533(M+H)⁺ |
| 21-1 | ESI-MS:376(M+H)⁺ |
| 21-2 | ESI-MS:358(M+H)⁺ |
| 22 | ESI-MS:376(M+H)⁺ |
| 23 | ESI-MS:513(M+H)⁺ |
| 24 | NMR(CDCl₃):3.88(3H,s),4.85(2H,s),6.99(1H,d,J=3.8Hz ),7.68(1H,d,J=3.8Hz) |
| 25 | ESI-MS:503(M+H)⁺ |
| 26 | ESI-MS:404(M+H)⁺ |
| 26-1 | ESI-MS:375(M+H)⁺ |
| 27 | |
| 28 | NMR(CDCl₃):1.30-2.20(1H,brs),2.23-2.44(4H,m),4.10-4.19(2H,m) |
| 28-1 | NMR(CDCl₃):1.4-1.8(1H,brs),2.21-2.44(4H,m),4.17-4. 26(2H,m) |
| 29 | ESI-MS:446(M+Na)⁺ |
| 29-1 | ESI-MS:424(M+H)⁺ |
| 30 | ESI-MS:443(M+H)⁺ |
| 30-1 | ESI-MS:465(M+H)⁺ |
| 31 | ESI-MS:564(M+H)⁺ |
| 32 | ESI-MS:224(M-H)⁻ |
| 33 | ESI-MS:241(M+H)⁺ |
| 34 | ESI-MS:227(M+H)⁺ |
| 35 | ESI-MS:241(M+Na)⁺ |
| 35-1 | ESI-MS:241(M+Na)⁺ |
| 35-2 | ESI-MS:241(M+Na)⁺ |

### Example 1

To a solution of 4-{[1-(5-bromopyrimidin-2-yl)-piperidine-4-yl]amino}-1H-pyrrolo[2,3-b]pyridine-5-carboxamide (100 mg) in DMF (2 mL) were added zinc (II) cyanide (84.6mg) and Pd(PPh₃)₄ (27.7 mg), and the mixture was stirred at 80°C for 1 hour under microwave irradiation. The reaction mixture was diluted with DCM, and was filtered to remove insoluble solid, and the filtrate was evaporated in vacuo. The residue was purified by preparative TLC (DCM:MeOH = 10:1) to afford 4-{[1-(5-cyanopyrimidin-2-yl)piperidine-4-yl]amino}-1H-pyrrolo[2,3-b]pyridine-5-carboxamide (59 mg) as a solid.

### Example 2

To a solution of a mixture (23 mg) of (4R)-4-fluoro-1-{[(3S,4S)-4-methyl-3-(2-oxo-3,6-dihydroimidazo[4,5-d]-pyrrolo[2,3-b]pyridine-1(2H)-yl)piperidine-1-yl]carbonyl}-D-proline amide and (4R)-4-fluoro-1-{[(3S,4S)-4-methyl-3-(2-oxo-3,6-dihydroimidazo[4,5-d]pyrrolo[2,3-b]pyridine-1(2H)-yl)piperidine-1-yl]carbonyl}-D-proline amide and a solution of TFAA (0.12 mL) in dioxane (1 mL) was added pyridine (0.052 mL) dropwise at ambient temperature, and the reaction mixture was stirred at ambient temperature for 4 hours. To the resulting solution was added saturated NaHCO₃ aqueous solution, and the solution was extracted with a mixed solvent (chloroform and MeOH). The organic layer was dried over anhydrous MgSO₄ and filtered. The filtrate was evaporated in vacuo. The residue was purified by silica gel column chromatography (NH silica gel, purchased from Fuji silysia com; eluate, chloroform:MeOH = 99:1 to 80:20) to afford a mixture (16.6 mg) of (2R,4R)-4-fluoro-1-{[(3S,4S)-4- methyl-3-(2-oxo-3,6-dihydroimidazo[4,5-d]pyrrolo[2,3-b]pyridine-1(2H)-yl)-piperidine-1-yl]carbonyl}pyrrolidine-2-carbonitrile and (2R,4R)-4-fluoro-1-{[(3R,4R)-4-methyl-3-(2-oxo-3,6-dihydroimidazo[4,5-d]pyrrolo[2,3-b]pyridine-1(2H)-yl)-piperidine-1-yl]carbonyl}pyrrolidine-2-carbonitrile as a white powder.

### Example 3

To a solution of 3,3-dibromo-4-{[(1S,2R)-2-methylcyclohexyl]amino}-2-oxo-2,3-dihydro-1H-pyrrolo-[2,3-b]pyridine-5-carboxamide (123 mg) in AcOH (1.23 mL) was added zinc powder (180 mg), and the mixture was stirred at ambient temperature for overnight. Then the catalyst was removed by filtration, the filtrate was evaporated in vacuo. The residue was partitioned with chloroform and water. The organic layer was dried over anhydrous MgSO₄ and filtered, the filtrate was evaporated in vacuo. The residue was purified by silica gel column chromatography (chloroform:MeOH = 100:0 to 90:10) to afford 4-{[(1S,2R)-2-methylcyclohexyl]amino}-2-oxo-2,3-dihydro- 1H-pyrrolo[2,3- b]pyridine-5-carboxamide (12 mg) as a white solid.

### Example 4

A solution of a mixture of N-{1-[(3R,4R)-4-methylpiperidine-3-yl]-1,6-dihydropyrazolo[3,4-d]pyrolo[2,3-b]pyridine-3-yl}acetamide and N-{1-[(3S,4S)-4-metylpiperidine-3-yl]-1,6-dihydropyrazolo[3,4-d]pyrrolo[2,3-b]pyridine-3-yl}acetamide (35 mg), cyanoacetic acid (19 mg), EDCI·HCl (54 mg), HOBt (38 mg) and TEA (57 mg) in DMF (1 mL) was stirred at ambient temperature for 2 hours. To the reaction mixture was added water, and the mixture was extracted with EtOAc. The organic layer was washed with water and brine, dried over anhydrous MgSO₄ and filtered. The filtrate was evaporated in vacuo. The residue was purified by silica gel column chromatography (chloroform:MeOH = 99:1 to 90:10) to afford a mixture of N-{1-[(3R,4R)-1-(cyanoacetyl)-4-methylpiperidine-3-yl]-1,6-dihydropyrazolo-[3,4-d]pyrrolo [2,3-b]pyridine-3-yl}acetamide and N-{1-[(3S,4S)-1-(cyanoacetyl)-4-methylpiperidine-3-yl]--1,6-dihydropyrazolo-[3,4-d]pyrrolo[2,3-b]pyridine-3-yl}acetamide (17.5 mg) as a white powder.

### Example 5

Methyl 6-{4-[(5-carbamoyl-1H-pyrrolo[2,3-b]pyridine-4-yl)amino]piperidine-1-yl}nicotinate (100 mg) was dissolved in 2M NH₃/MeOH, and the mixture was stirred at 90°C in a sealed tube. The reaction mixture was evaporated in vacuo and purified by preparative TLC (DCM:MeOH = 10:1) to afford 4-{[1-(5-carbamoylpyridine-2-yl)piperidine-4-yl]amino}-1H-pyrrolo[2,3-b]pyridine-5-carboxamide (19 mg) as a solid.

### Example 6

A mixture (13 mg) of 4-{[(3S,4R)-3-fluoropiperidine-4-yl]amino}-1H-pyrrolo[2,3-b]pyridine-5-carboxamide and 4-{[(3R,4S)-3-fluoropiperidine-4-yl]amino}-1H-pyrrolo-[2,3-b]pyridine-5-carboxamide and 6-chloropyridazine-3-carbonitrile (13 mg) in NMP (0.5 mL) was added TEA (19 mg). The mixture was stirred at 120°C for 1 hour under microwave irradiation. Then the mixture was cooled to ambient temperature, the residue was purified by silica gel column chromatography (NH silica gel, purchased from Fuji silysia com; eluate, chloroform:MeOH = 97:3 to 91:9) to afford a mixture of 4-{[(3S,4R)-1-(6-cyanopyridazine-3-yl)-3-fluoropiperidine-4-yl]aminol-1H-pyrrolo[2,3-b]pyridine-5-carboxamide and 4-{[(3R,4S)-1-(6-cyanopyridazine-3-yl)-3-fluoropiperidine-4-yl]amino}-1H-pyrrolo[2,3-b]pyridine-5-carboxamide (10 mg) as a pale yellow solid.

### Example 7

3-(4-Chloro-1H-pyrrolo[2,3-b]pyridine-5-yl)-N-(2-methoxyethyl)-1,2,4-oxadiazol-5-carboxamide (150 mg), (1S,2R)-2-methylcyclohexaneamine hydrochloride (130 mg) and DIPEA (181 mg) were dissolved in NMP (1.5 mL). The mixture was stirred at 200°C for 2 hours under microwave irradiation. The mixture was cooled to ambient temperature and was purified by silica gel column chromatography (chloroform:MeOH = 99:1 to 90:10) to afford N-(2-methoxyethyl)-N'-{1-[(1S,2R)-2-methylcyclohexyl]-1,6-dihydropyrazolo[3,4-d]pyrrolo[2,3-b]-pyridine-3-yl}ethanediamide (72 mg) as an oil. This compound was afforded as oxalate as a white solid (52 mg).

### Example 8

To a solution of a mixture of N-methyl-N'-{1-[(3R,4R)-4-methylpiperidine-3-yl]-1,6-dihydropyrazolo[3,4-d]pyrrolo[2,3-b]pyridine-3-yl}ethanediamide and N-methyl-N'-{1-[(3S,4S)-4-methylpiperidine-3-yl]-1,6-dihydropyrazolo[3,4-d]pyrrolo[2,3-b]pyridine-3-yl}ethanediamide (50 mg) and DIPEA (55 mg) in DMF (1 mL) was added pyrrolidine-1-carbonyl chloride (28 mg) dropwise at ambient temperature. The reaction mixture was stirred at ambient temperature for 2 hours. The reaction mixture was added water and was extracted with chloroform. The organic layer was dried over anhydrous MgSO₄ and filtered. The filtrate was evaporated in vacuo. The residue was purified by silica gel column chromatography (chloroform: MeOH = 98:2 to 92:8) to afford a colorless solid. This was reprecipitated from chloroform and diisopropyl ether to afford a mixture of N-methyl-N'-{1-[(3R,4R)-4-methyl-1-(pyrrolidine-1-ylcarbonyl)piperidine-3-yl]-1,6-dihydropyrazolo[3,4-d]pyrrolo[2,3-b]pyridine-3-yl}-ethanediamide and N-methyl-N'-{1-[(3S,4S)-4-methyl-1-(pyrrolidine-1-ylcarbonyl)piperidine-3-yl]-1,6-dihydropyrazolo[3,4-d]pyrrolo[2,3-b]pyridine-3-yl}-ethanediamide (25.4 mg) as a white powder.

### Example 9

A solution of a mixture of 1-{(3R,4R)-4-methyl-1-[(4-oxopiperidine-1-yl)carbonyl]piperidine-3-yl}-3,6-dihydroimidazo[4,5-d]-pyrrolo[2,3-b]pyridine-2(1H)-one and 1-{(3S,4S)-4-methyl-1-[(4-oxopiperidine-1-yl)carbonyl]piperidine-3-yl}-3,6-dihydroimidazo[4,5-d]-pyrrolo[2,3-b]pyridine-2(1H)-one (34 mg), hydroxyamine hydrochloride (18 mg) and a solution of sodium acetate (35 mg) in EtOH (1 mL) was stirred at 80°C for 30 minutes. The reaction mixture was cooled to ambient temperature and was evaporated in vacuo. The residue was dissolved in water and extracted with a mixed solvent (chloroform and MeOH). The organic layer was dried over anhydrous MgSO₄ and filtered. The filtrate was evaporated in vacuo. The residue was purified by silica gel column chromatography (chloroform:MeOH = 9:1 to 2:8) to afford 1-[(3R,4R)-1-{[4-(hydroxyimino)piperidine-1-yl]-carbonyl}-4- methylpiperidine-3-yl]-3,6-dihydroimidazo[4,5-d]pyrrolo[2,3-b]pyridine-2(1H)-one and 1-[(3S,4S)-1-{[4-(hydroxyimino)piperidine-1-yl]-carbonyl}-4-methylpiperidine-3-yl]-3,6-dihydroimidazo[4,5-d]pyrrolo[2,3-b]pyridine-2(1H)-one (35 mg) as a white powder.

### Example 10

To a solution of CDI (90 mg) in DMF (2 mL) was added 3-hydroxypropanenitrile (39 mg) at 0°C. The mixture was stirred at ambient temperature for 30 minutes. To the reaction mixture was added a mixture of 1-[(3R,4R)-4-methylpiperidine-3-yl]-3,6-dihydroimidazo[4,5- d]pyrrolo[2,3-b]pyridine-2(1H)-one and 1-[(3S,4S)-4-methylpiperidine-3-yl]-3,6-dihydroimidazo[4,5-d]pyrrolo[2,3-b]pyridine-2(1H)-one (100 mg). The mixture was stirred at ambient temperature for overnight and further at 50°C for overnight. The reaction mixture was cooled to ambient temperature. The mixture was poured into water and was extracted with a mixed solvent (chloroform and MeOH). The organic layer was dried over anhydrous MgSO₄ and filtered. The filtrate was evaporated in vacuo. The residue was purified by silica gel column chromatography (chloroform:MeOH = 99:1 to 90:10) to afford a mixture of 2-cyanoethyl (3R,4R)-4-methyl-3-(2-oxo-3,6-dihydroimidazo[4,5-d]pyrrolo[2,3-b]pyridine-1(2H)-yl)-piperidine-1-carboxylate and 2-cyanoethyl (3S,4S)-4-methyl-3-(2-oxo-3,6-dihydroimidazo[4,5-d]pyrrolo[2,3-b]pyridine-1(2H)-yl)-piperidine-1-carboxylate (68.5 mg) as a white powder.

### Example 11

To a solution of a mixture of 1-{(3R,4R)-4-methyl-1-[(4-oxopiperidine-1-yl)carbonyl]piperidine-3-yl}-3,6-dihydroimidazo[4,5-d]-pyrrolo[2,3-b]pyridine-2(1H)-one and 1-{(3S,4S)-4-methyl-1-[(4-oxopiperidine-1-yl)carbonyl]piperidine-3-yl}-3,6-dihydroimidazo[4,5-d]-pyrrolo[2,3-b]pyridine-2(1H)-one (40 mg) in THF (1 mL) was added a solution of 1M methylmagnesium bromide (36 mg) in THF (0.3 mL) dropwise at 0°C. The reaction mixture was stirred at 0°C for 1 hour. To the reaction mixture was added saturated NH₄Cl aqueous solution , and was extracted with a mixed solvent (chloroform and MeOH). The organic layer was dried over anhydrous MgSO₄ and filtered. The filtrate was evaporated in vacuo. The residue was purified by silica gel column chromatography (chloroform:MeOH = 99:1 to 70:30) to afford a mixture of 1-{(3R,4R)-1-[(4-hydroxy-4-methylpiperidine-1-yl)carbonyl]-4-methylpiperidine-3-yl}-3,6-dihydroimidazo[4,5-d]pyrrolo[2,3-b]pyridine-2(1H)-one and 1-{(3S,4S)-1-[(4-hydroxy-4-methylpiperidine-1-yl)carbonyl]-4-methylpiperidine-3-yl}-3,6-dihydroimidazo[4,5-d]pyrrolo[2,3-b]pyridine-2(1H)-one (20.9 mg) as a white powder.

### Example 12

To a solution of N-(1-cycloheptyl-1,6-dihydropyrazolo-[3,4-d]pyrrolo[2,3-b]pyridine-3-yl)-N'-(2-methoxyethyl)-ethanediamide (59 mg) in MeOH (3 mL) was added 5M NaOH aqueous solution (0.1 mL). The reaction mixture was stirred at 60°C for 5 hours. The reaction mixture was cooled to ambient temperature and was extracted with chloroform (20 mL). The organic layer was dried over anhydrous MgSO₄, filtered and evapolated in vacuo. The residue was purified by silica gel column chromatography (chloroform: MeOH = 100:0 to 92:8) to afford 1-cycloheptyl-1, 6-dihydropyrazolo-[3,4-d]pyrrolo[2,3-b]pyridine-3-amine (33mg) as a white solid.

### Example 13

A solution of methyl 6-{4-[(5-carbamoyl-1H-pyrrolo-[2,3-b]pyridine-4-yl)amino]piperidine-1-yl}nicotinate (100 mg) in THF (1 mL) was stirred under ice-cooling. To the solution was added LiAlH₄ (9.6 mg) under N₂ atmosphere. The mixture was stirred under ice-cooling. To the reaction mixture were added water (10 µl) and 15% NaOH aqueous solution (30 µL) successively, and the mixture was stirred under ice-cooling. The precipitated insoluble solid was removed by filtration. The filtrate was evaporated in vacuo. The residue was purified by preparative TLC (DCM:MeOH = 10:1) to afford 4-({1-[5-(hydroxymethyl)pyridine-2-yl]piperidine-4-yl}amino)-1H-pyrrolo[2,3-b]pyridine-5-carboxamide (74 mg) as a solid.

### Example 14

To a suspension of 4-(piperidine-4-ylamino)-1H-pyrrolo-[2,3-b]pyridine-5-carboxamide dihydrochloride (100 mg) in DCM (1 mL) were added TEA (83.9 µL), 2-formylbenzonitrile (78.9 mg) and triacetoxy sodium borohydride (127.5 mg) successively, and the mixture was stirred at ambient temperature for overnight. To the reaction mixture were added saturated NaHCO₃ aqueous solution and chloroform, and the mixture was stirred at ambient temperature. The organic layer was separated and dried over anhydrous MgSO₄, filtered and evaporated in vacuo. The residue was purified by preparative TLC (DCM: MeOH = 10:1) to afford 4-{[1-(2-cyanobenzyl)piperidine-4-yl]amino}-1H-pyrrolo[2,3-b]-pyridine-5-carboxamide (18 mg) as a solid.

### Example 15

A mixture of cyclohexyl-3-methoxy-6-{[2-(trimethylsilylethoxy)]methyl}-1,6-dihydropyrazolo[3,4-d]-pyrrolo[2,3-b]pyridine (100 mg) and 4M HCl/EtOH (624 µL) was stirred at 90°C for 2 hours. The reaction mixture was cooled to ambient temperature and was evaporated in vacuo. The residue was dissolved in THF (1.4 mL), and were added ethylenediamine (117 µL) and 2M NaOH aqueous solution (437 µL). The mixture was stirred at ambient temperature for overnight and stirred at 50°C for 2 hours. The reaction mixture was cooled to ambient temperature and acidified pH=5 with 1M HCl aqueous solution. The resulting solid was collected by filtration. The white solid was reprecipitated from mixed solvent (EtOAc and n-hexane) to afford 1-cyclohexyl-3-methoxy-1,6-dihydropyrazolo[3,4-d]pyrrolo[2,3-b]pyridine (37 mg).

### Example 16

To a solution of a mixture of 1-{(3R,4R)-4-methyl-1-[(5-{[(triisopropylsilyl)oxy]methyl}-2-thienyl)carbonyl]-piperidine-3-yl}-3,6-dihydroimidazo[4,5-d]pyrrolo[2,3-b]-pyridine-2(1H)-one and 1-{(3S,4S)-4-methyl-1-[(5-{[(triisopropylsilyl)oxy]methyl}-2-thienyl)carbonyl]-piperidine-3-yl}-3,6-dihydroimidazo[4,5-d]pyrrolo[2,3-b]-pyridine-2(1H)-one (209 mg) in THF (3 mL) was added a solution of TBAF (125 mg) in THF (0.48 mL) at 0°C. The mixture was stirred at ambient temperature for 2 hours. The reaction mixture was added water and was extracted with a mixed solvent (chloroform and MeOH). The organic layer was dried over anhydrous MgSO₄ and filtered. The filtrate was evaporated in vacuo. The residue was purified by silica gel column chromatography (chloroform: MeOH = 99:1 to 80:20) to afford the mixture of 1-[(3R,4R)-1-{[5-(hydroxymethyl)-2-thienyl]carbonyl}- 4-methylpiperidine-3-yl]-3,6-dihydroimidazo[4,5-d]pyrrolo[2,3-b]pyridine-2(1H)-one and 1-[(3S,4S)-1-{[5-(hydroxymethyl)-2-thienyl]carbonyl}-4-methylpiperidine-3-yl]-3,6-dihydroimidazo[4,5-d]pyrrolo[2,3-b]pyridine-2(1H)-one (94.2 mg) as a white powder.

The compound (s) shown in the following Table were produced according to the above-mentioned production methods, the methods obvious to those skilled in the art, or modified methods of these. The table (s) show the structures and physicochemical data of the compounds described in these Example(s) and also show the methods for producing the compounds.

**Table 5**

| Ex | Structure | Ex | Structure |
|---|---|---|---|
| 1 | 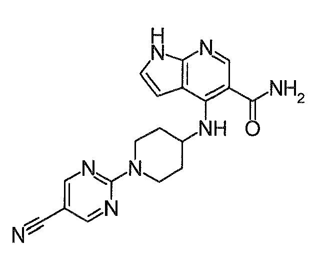 | 2 | 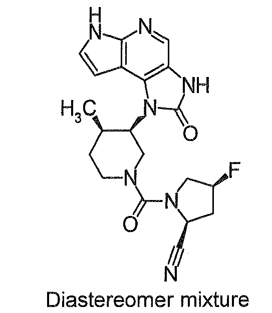 |
| 3 | 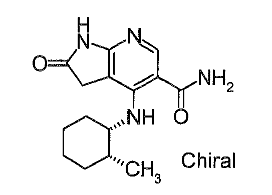 | 4 | 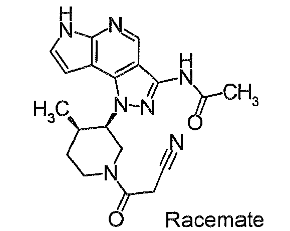 |
| 5 | 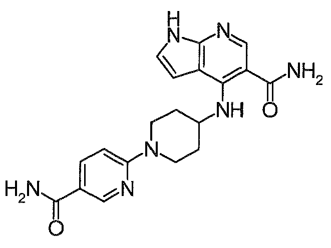 | 6 | 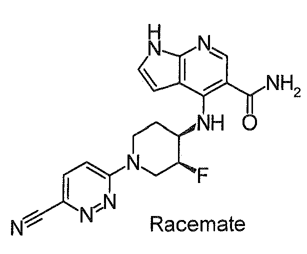 |
| 7 | 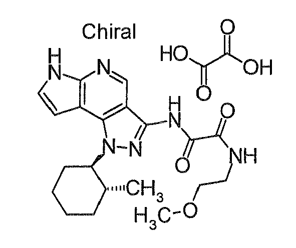 | 8 | 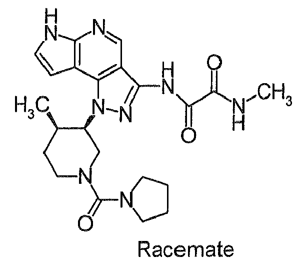 |
| 9 | 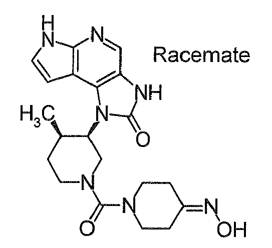 | 10 | 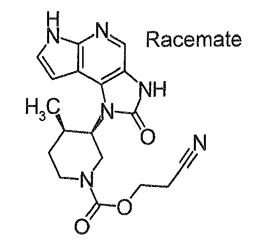 |
| 11 | 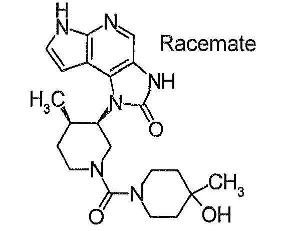 | 12 | 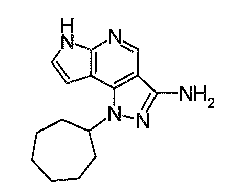 |
| 13 | 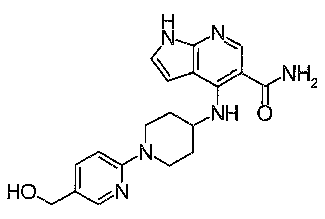 | 14 | 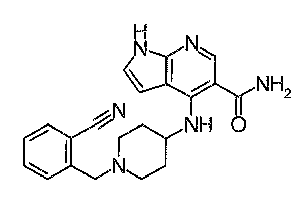 |
| 15 | 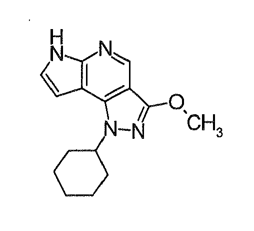 | 16 | 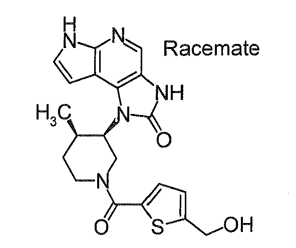 |
| 17 | 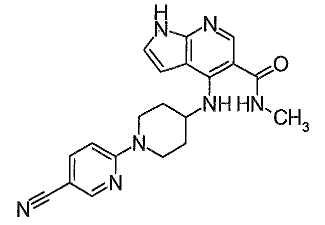 | 18 | 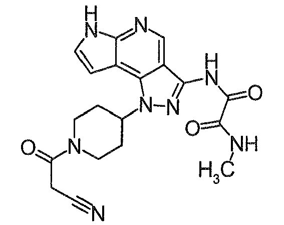 |
| 19 | 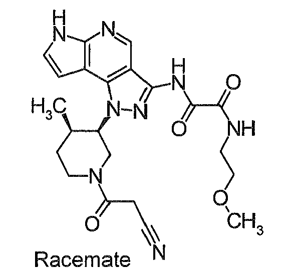 | 20 | 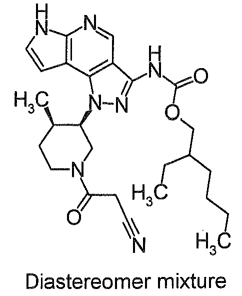 |
| 21 | 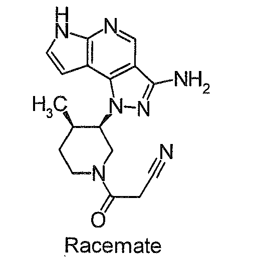 | 22 | 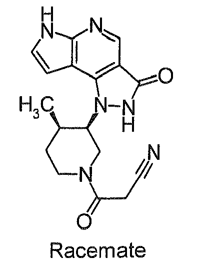 |
| 23 | 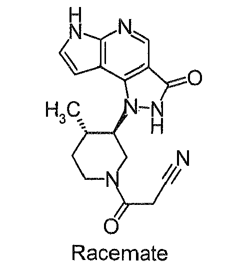 | 24 | 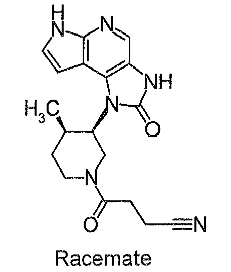 |
| 25 | 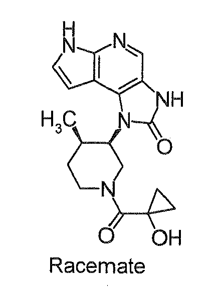 | 26 | 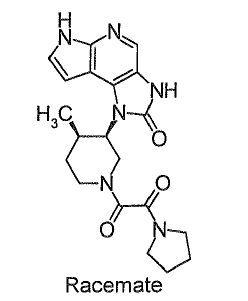 |
| 27 | 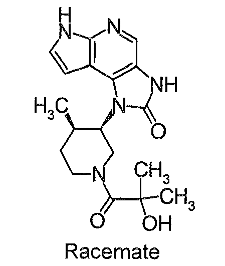 | 28 | 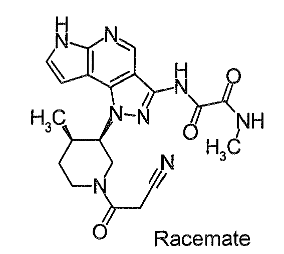 |
| 29 | 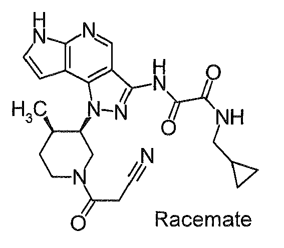 | 30 | 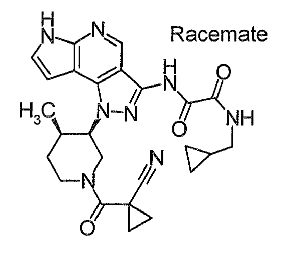 |
| 31 | 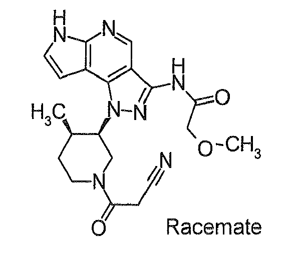 | 32 | 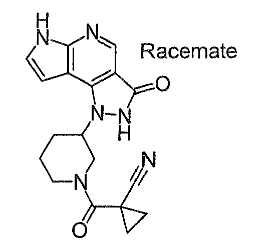 |
| 33 | 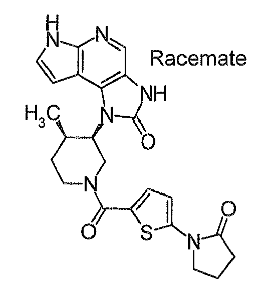 | 34 | 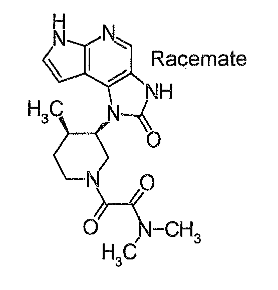 |
| 35 | 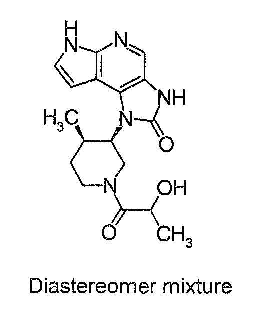 | 36 | 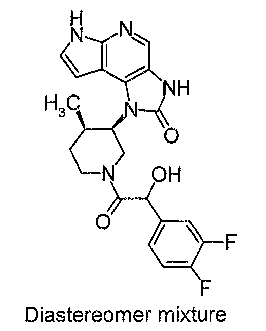 |
| 37 | 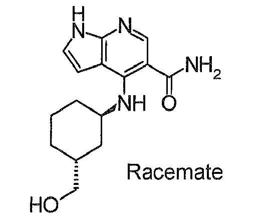 | 38 | 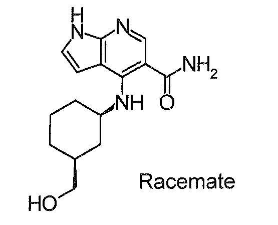 |
| 39 | 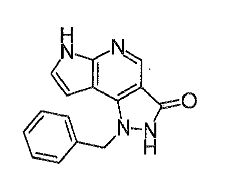 | 40 | 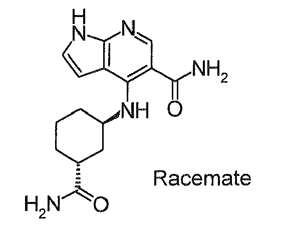 |
| 41 | 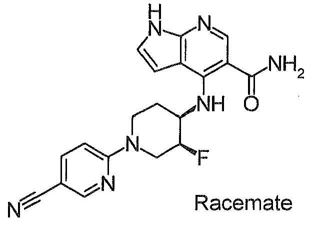 | 42 | 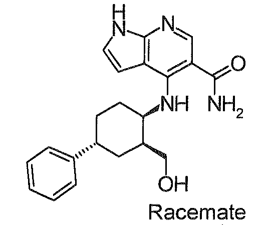 |
| 43 | 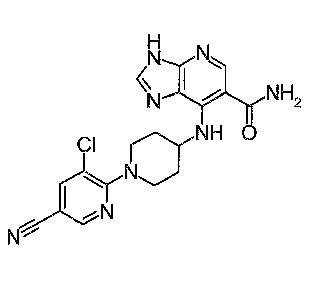 | 44 | 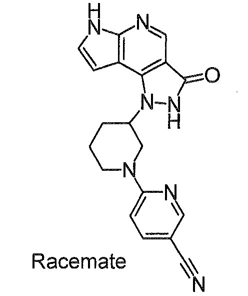 |
| 45 | 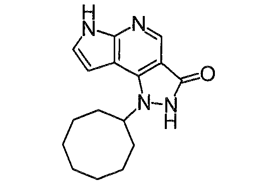 | 46 | 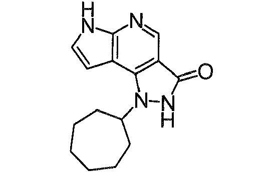 |
| 47 | 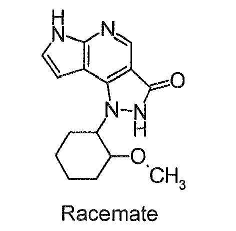 | 48 | 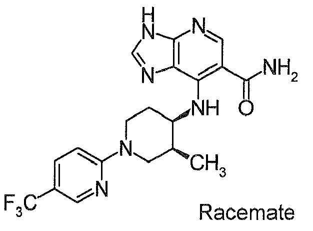 |
| 49 | 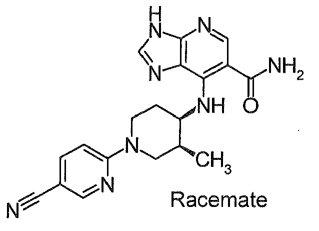 | 50 | 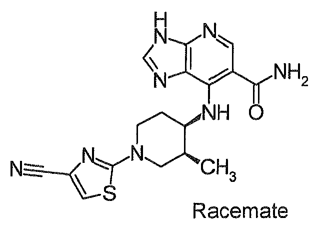 |
| 51 | 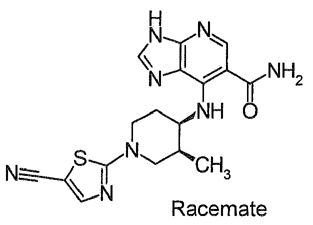 | 52 | 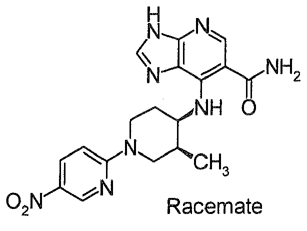 |
| 53 | 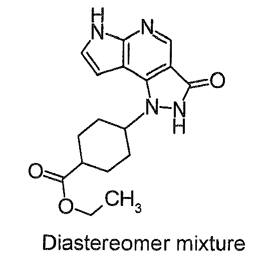 | 54 | 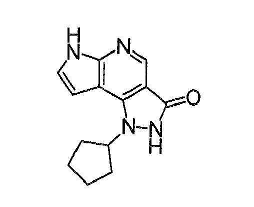 |
| 55 | 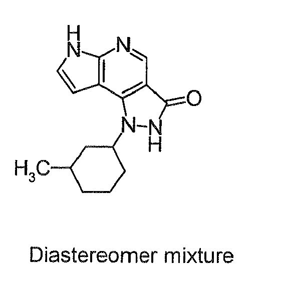 | 56 | 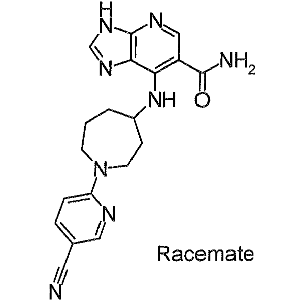 |
| 57 | 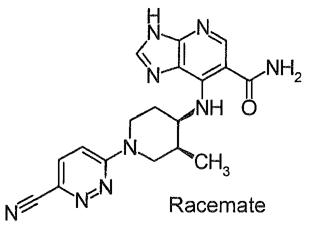 | 58 | 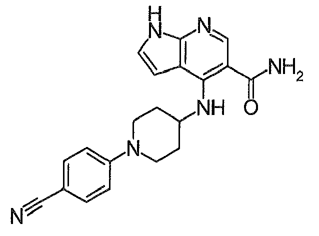 |
| 59 | 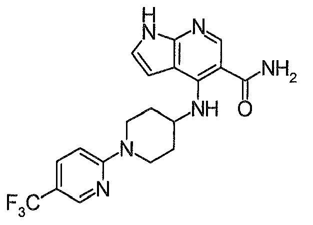 | 60 | 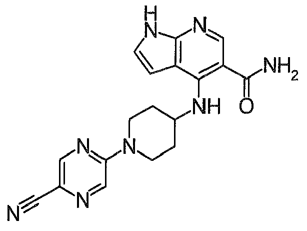 |
| 61 | 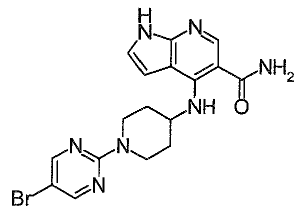 | 62 | 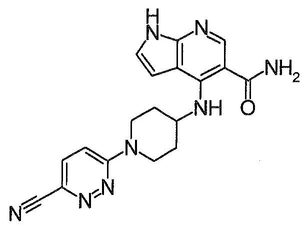 |
| 63 | 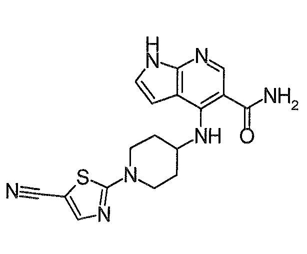 | 64 | 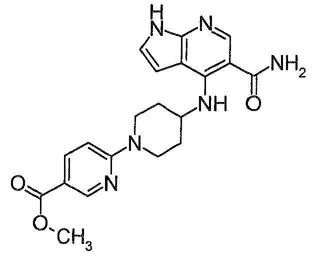 |
| 65 | 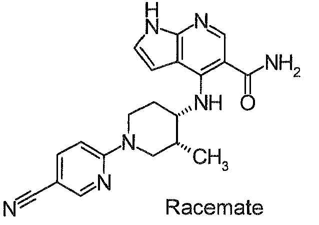 | 66 | 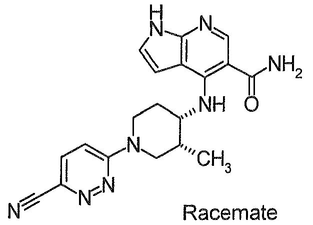 |
| 67 | 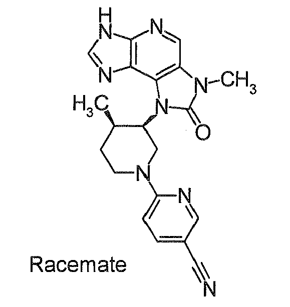 | 68 | 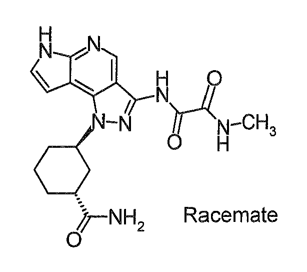 |
| 69 | 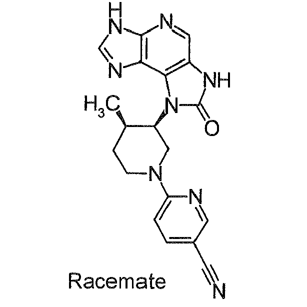 | 70 | 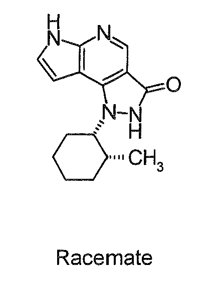 |
| 71 | 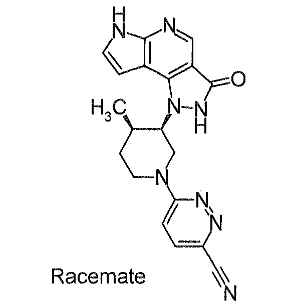 | 72 | 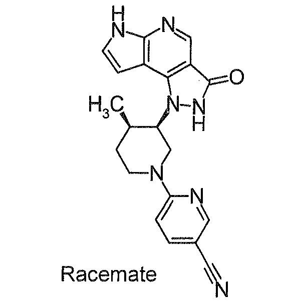 |
| 73 | 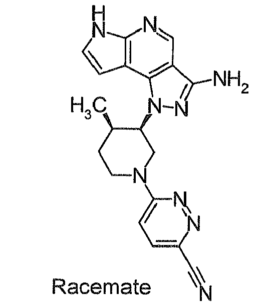 | 74 | 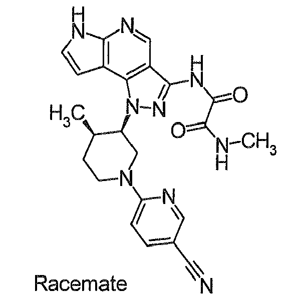 |
| 75 | 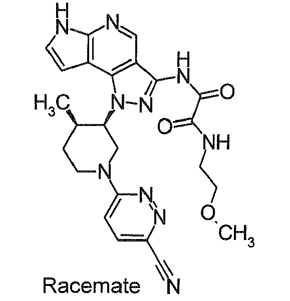 | 76 | 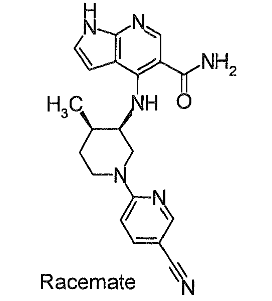 |
| 77 | 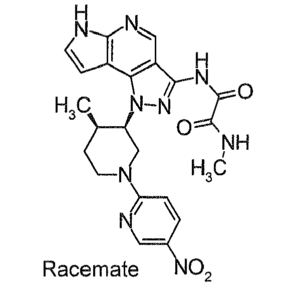 | 78 | 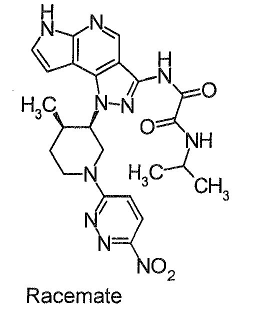 |
| 79 | 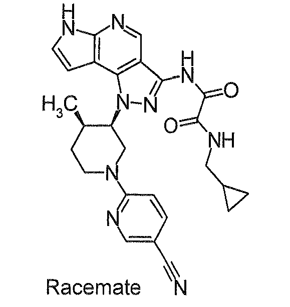 | 80 | 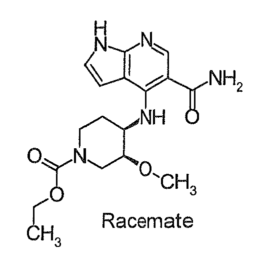 |
| 81 | 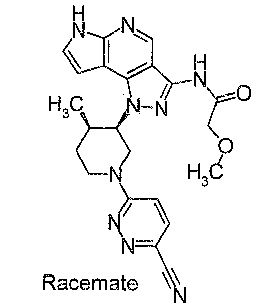 | 82 | 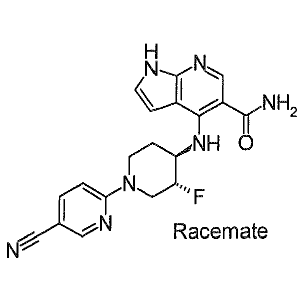 |
| 83 | 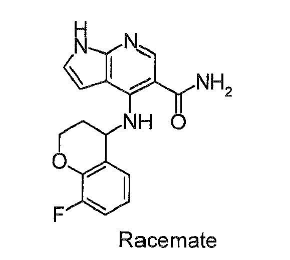 | 84 | 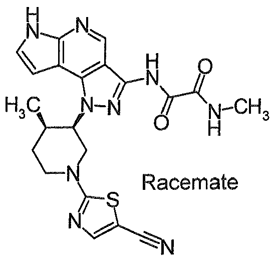 |
| 85 | 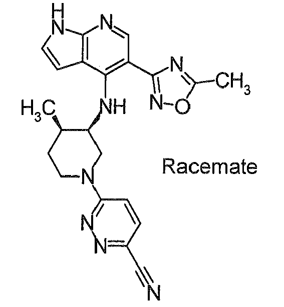 | 86 | 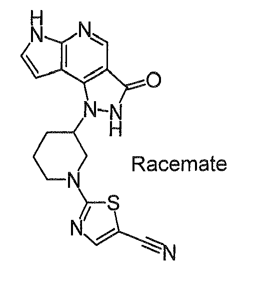 |
| 87 | 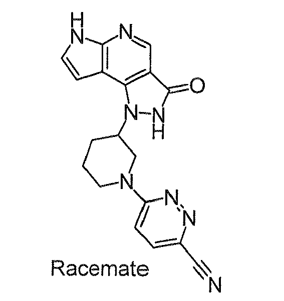 | 88 | 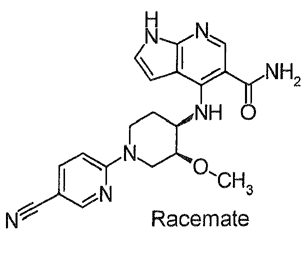 |
| 89 | 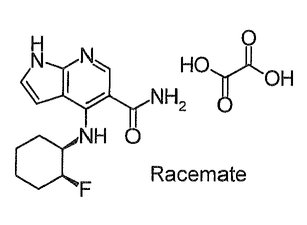 | 90 | 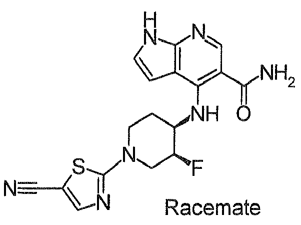 |
| 91 | 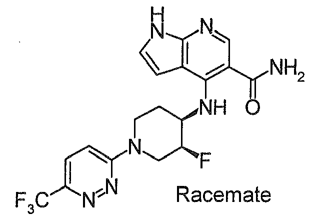 | 92 | 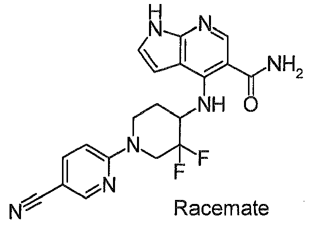 |
| 93 | 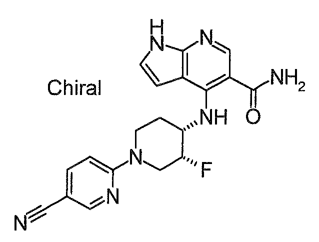 | 94 | 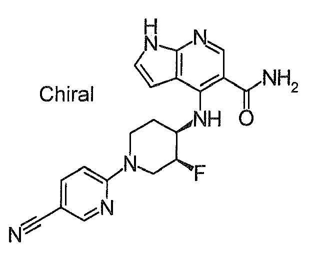 |
| 95 | 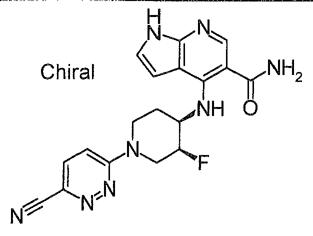 | 96 | 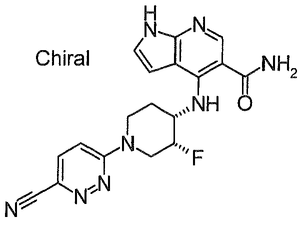 |
| 97 | 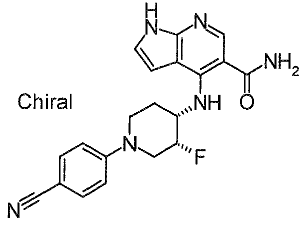 | 98 | 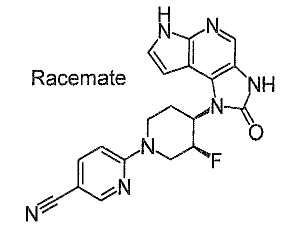 |
| 99 | 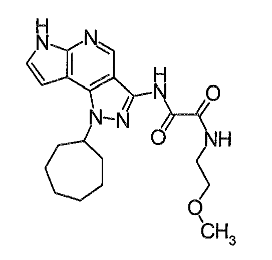 | 100 | 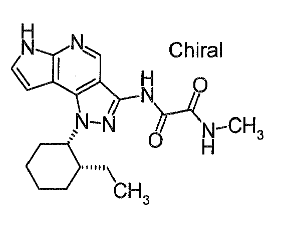 |
| 101 | 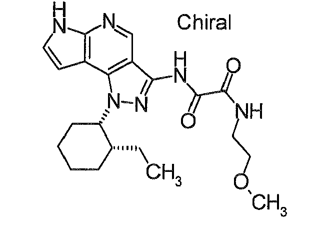 | 102 | 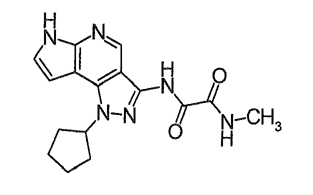 |
| 103 | 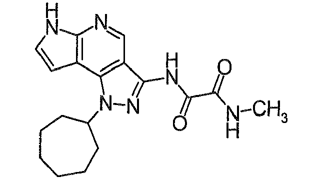 | 104 | 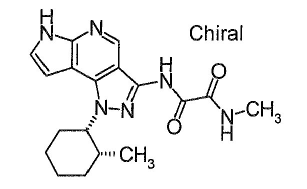 |
| 105 | 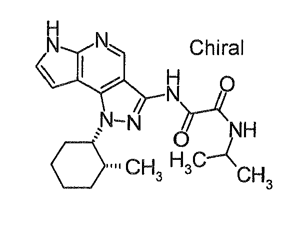 | 106 | 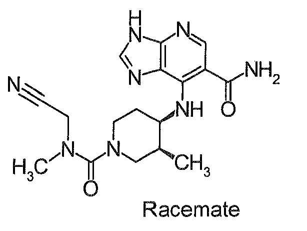 |
| 107 | 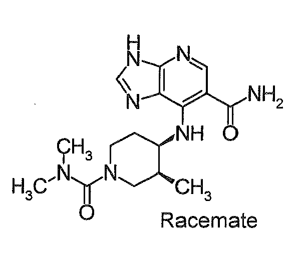 | 108 | 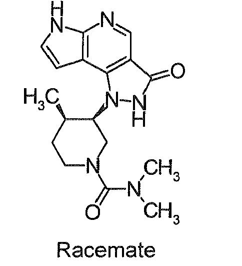 |
| 109 | 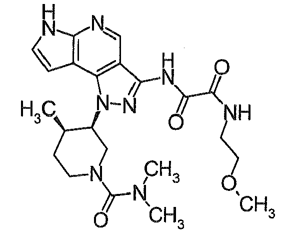 | 110 | 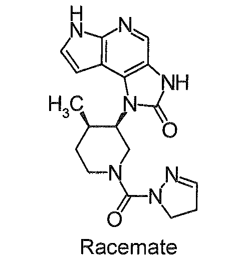 |
| 111 | 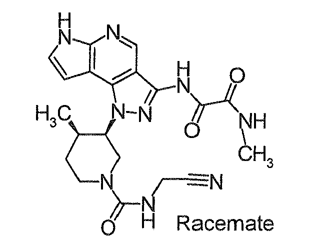 | 112 | 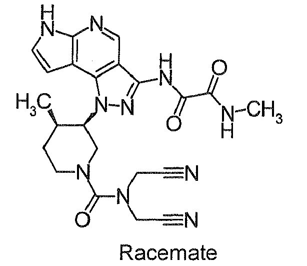 |
| 113 | 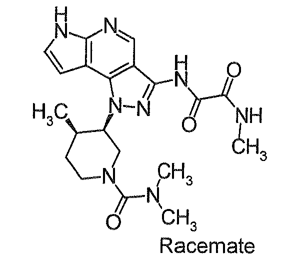 | 114 | 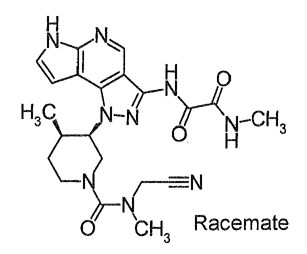 |
| 115 | 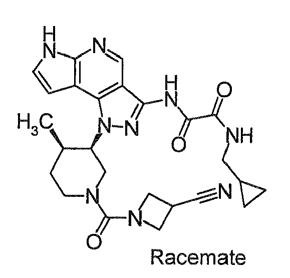 | 116 | 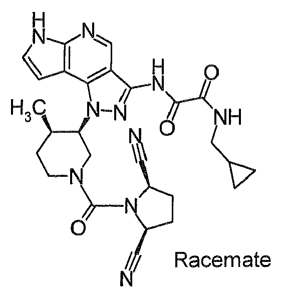 |
| 117 | 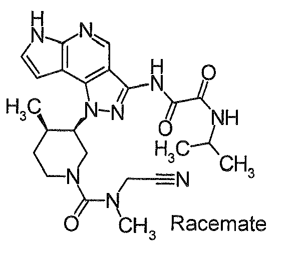 | 118 | 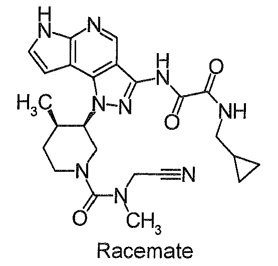 |
| 119 | 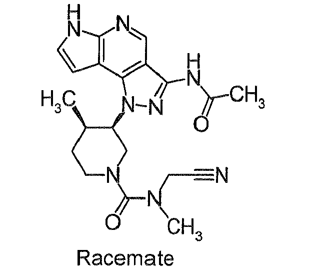 | 120 | 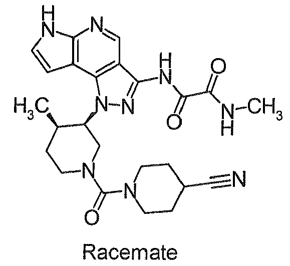 |
| 121 | 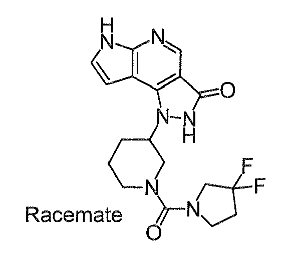 | 122 | 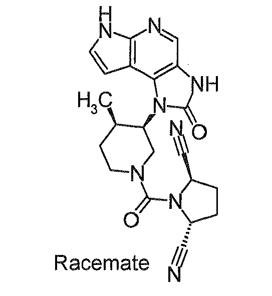 |
| 123 | 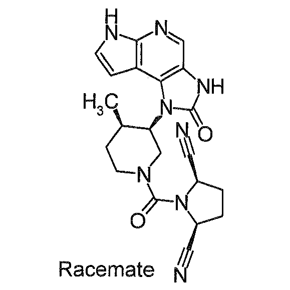 | 124 | 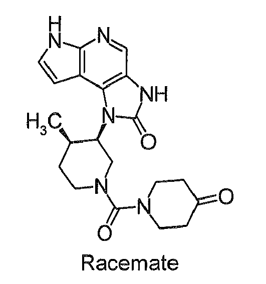 |
| 125 | 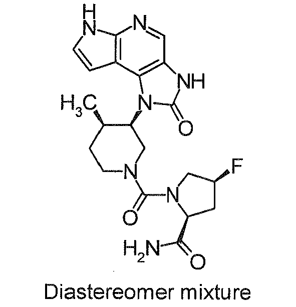 | 126 | 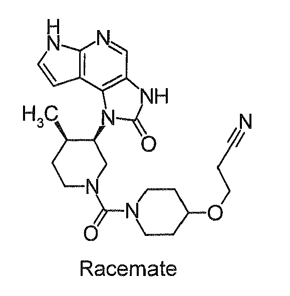 |
| 127 | 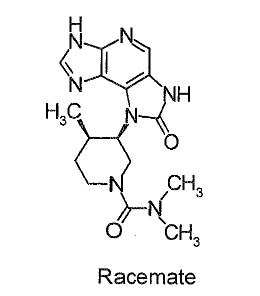 | 128 | 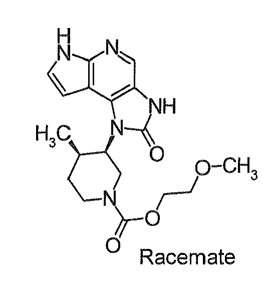 |
| 129 | 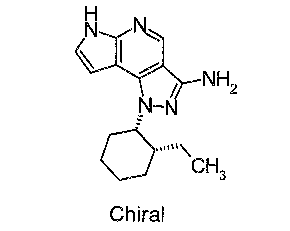 | 130 | 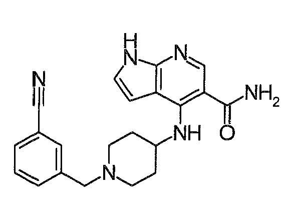 |
| 131 | 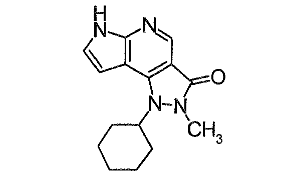 | 132 | 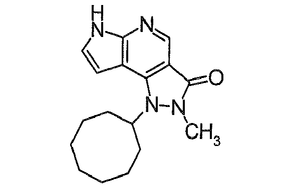 |
| 133 | 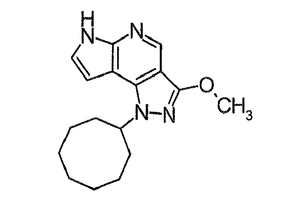 | 134 | 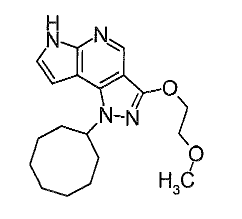 |

The following Table describes the processes and physicochemical data for the compounds described in Examples.

NMR: ¹H-NMR (400MHz, d₆-DMSO) δ; NMR(CDCl₃) δ: ¹H-NMR (400MHz, CDCl₃) δ; Data: physicochemical data; Ex: Example Number; Syn: process (the number indicates that the compound corresponding to Example was produced using a process similar to that for the compound corresponding to Example identified by the number.). MS indicates the results of API-ES or ESI-MS.

**Table 6**

| Ex | Syn | Data |
|---|---|---|
| 1 | - | NMR:1.47-1.49(2H,m),2.09-2.13(2H,m),3.52-3.58(2 H,m),4.34(1H,m),4.44-4.48(2H,m),6.65(1H,m),7.00 (1H,brs),7.17-7.18(1H,m),7.79(1H,brs),8.37(1H,s ),8.76(2H,s),9.76(1H,d,J=8.2Hz),11.52(1H,s). API-ES:363(M+H)⁺,385(M+Na)⁺ |
| 2 | - | NMR:0.99(3H,d,J=7.2Hz),1.66-1.77(1H,m),1.96-2.0 8(1H,m),2.30-3.93(8H,m),4.24-4.36(1H,m),4.53-4. 63(1H,m),4.97-5.09(1H,m),5.28-5.48(1H,m),6.46-6 .53(1H,m),7.40-7.49(1H,m),7.91(1H,s),10.77-10.9 1(1H,brs),11.60(1H,s) ESI-MS:412(M+H)⁺ |
| 3 | - | NMR:0.85(3H,d,J=7.0Hz),1.27-1.91(8H,m),1.76-1.8 2(1H,m),3.52(1H,d,J=21.6Hz),3.69(1H,d,J=21.6Hz) ,3.72-3.77(1H,m),7.16(1H,br),7.89(1H,br),8.27(1 H,s),9.16(1H,d,J=9.8Hz),10.77(1H,brs) ESI-MS:311(M+Na)⁺ |
| 4 | - | NMR:0.53-0.72(3H,m),1.62-1.88(2H,m),2.15(3H,s),2.08-4.20(7H,m),4.90-5.10(1H,m),6.82-6.94(1H,m) ,7.39-7.44(1H,m),8.72-8.85(1H,m),10.42-10.52(1H ,m),11.96-12.06(1H,brs) ESI-MS:402(M+Na)⁺ |
| 5 | - | NMR:1.42-1.50(2H,m),2.07-2.10(2H,m),3.36-3.41(2H,m),4.15-4.20(2H,m),4.28-4.30(1H,m),6.63(1H,dd ,J=1.9,3.5Hz),6.89(1H,d,J=9.0Hz),7.00(1H,brs),7 .13(1H,brs),7.16-7.17(1H,m),7.725(1H,brs),7.75(1H,brs),7.96(1H,dd,J=2.5,9.0Hz),8.37(1H,s),8.62 (1H,d,J=2.2Hz),9.76(1H,d,J=8.2Hz),11.51(1H,s). API-ES:380(M+H)⁺,402(M+Na)⁺ |
| 6 | - | NMR:1.66-1.78(1H,m),2.02-2.10(1H,m),3.34-3.71(2H,m),4.49-4.72(2H,m),4.87-5.14(2H,m),6.67-6.71( 1H,m),7.03(1H,brs),7.20-7.24(1H,m),7.47(1H,d,J=9.6Hz),7.79(1H,brs),7.89(1H,d,J=9.6Hz),8.39(1H, s),9.86(1H,d,J=9.2Hz),11.57(1H,brs) ESI-MS:381(M+H)⁺ |
| 7 | - | NMR:0.65(3H,d,J=6.8Hz),1.42-2.36(9H,m),3.28(3H,s),3.37-3.51(4H,m),4.91-4.97(1H,m),6.80-6.94(1H ,m),7.43-7.46(1H,m),8.69(1H,s),8.95(1H,t,J=5.6Hz),10.88(1H,s),12.04(1H,brs) ESI-MS:399(M+H)⁺ |
| 8 | - | NMR:1.04(3H,d,J=6.0Hz),1.52-1.82(6H,m),2.08-2.20(1H,m),2.42-2.47(1H,m),2.77(3H,d,J=3.3Hz),3.10 -3.24(4H,m),3.47-3.58(2H,m),3.80-3.88(1H,m),4.97-5.03(1H,m),6.82-6.90(1H,m),7.43-7.47(1H,m),8. 71(1H,s),8.97-9.04(1H,m),10.81(1H,s),12.06(1H,s ) ESI-MS:475(M+Na)⁺ |
| 9 | - | NMR:0.99(3H,d,J=7.1Hz),1.67-1.75(1H,m),1.92-2.0 8(1H,m),3.17-2.30(1H,m),2.38-3.49(10H,m),3.68-3.78(1H,m),4.21-4.31(1H,m),4.52-4.60(1H,m),6.44-6.47(1H,m),7.42-7.46(1H,m),7.91(1H,s),10.38(1H, s),10.83(1H,s),11.60(1H,s) ESI-MS:434(M+Na)⁺ |
| 10 | - | NMR:0.98(3H,d,J=7.1Hz),1.67-1.80(1H,m),1.88-2.02(1H,m),2.40-2.51(1H,m),2.80-2.96(2H,m),3.16-3. 34(1H,m),3.0-3.84(1H,m),4.04-4.54(5H,m),6.52(1H,s),7.41-7.44(1H,m),7.91(1H,s),10.86(1H,s),11.5 8-11.65(1H,brs) ESI-MS:391(M+Na)⁺ |
| 11 | - | NMR:0.99(3H,d,J=7.1Hz),1.10(3H,s),1.32-3.39(13H,m),3.62-3.71(1H,m),4.17-4.29(2H,m),4.50-4.59(1 H,m),6.41-6.45(1H,m),7.42-7.46(1H,m),7.91(1H,s) ,10.82(1H,s),11.61(1H,s) ESI-MS:413(M+H)⁺ |
| 12 | - | NMR:1.54-2.08(12H,m),4.64-4.72(1H,m),5.64(2H,s) ,6.68-6.73(1H,m),7.27-7.32(1H,m),8.52(1H,s),11. 74(1H,brs) ESI-MS:270(M+H)⁺ |
| 13 | - | NMR:1.45-1.48(2H,m),2.04-2.07(2H,m),3.25-3.30(2H,m),4.03-4.06(2H,m);4.24(1H,m),4.35(2H,d,J=5.6 Hz),4.98(1H,t,J=5.6Hz),6.61(1H,m),6.86(1H,d,J=8.6Hz),6.99(1H,brs),7.15-7.17(1H,m),7.49(1H,dd,J =2.4,8.6Hz),7.77(1H,brs),8.05(1H,d,J=2.4Hz),8.3 7(1H,s),9.73(1H,d,J=8.1Hz),11.50(1H,s). API-ES:367(M+H)⁺,389(M+Na)⁺ |
| 14 | - | NMR:1.50-1.53(2H,m),1.99-2.02(2H,m),2.35-2.40(2H,m),2.72(2H,brs),2.14(2H,s),4.01(1H,brs),6.51( 1H,s),6.99(1H,brs),7.13(1H,s),7.47(1H,t,J=7.5Hz ),7.61(1H,d,J=7.5Hz),7.69(1H,t,J=7.5Hz),7.75(1H,brs),7.81(1H,d,J=7.5Hz),8.36(1H,s),9.73(1H,d,J =7.0Hz),11.47(1H,s). API-ES:397(M+Na)⁺ |
| 15 | - | NMR:1.20-2.00(10H,m),4.01(3H,s),4.50-4.60(1H,m) ,6.79-6.80(1H,m),7.38-7.40(1H,m),8.44(1H,s),12. 0(1H,brs) ESI-MS:271(M+H)⁺ |
| 16 | - | NMR:1.01(3H,d,J=7.2Hz),1.72-1.84(1H,m),2.00-2.16(1H,m),3.40-4.69(8H,m),5.45-5.71(1H,brs),6.58(1H,d,J=3.4Hz),6.86-6.98(1H,m),7.22-7.33(1H,m),7.42-7.48(1H,m),7.91(1H,s),10.72-11.02(1H,brs),1 1.56-11.71(1H,brs) ESI-MS:434(M+Na)⁺ |
| 17 | 4 | NMR:1.43-1.50(2H,m),2.08-2.11(2H,m),2.72(3H,d,J=4.4Hz),3.41-3.46(2H,m),4.21-4.25(2H,m),4.31-4. 34(1H,m),6.64(1H,d,J=2.6Hz),6.99(1H,d,J=9.1Hz),7.19(1H,m),7.85(1H,dd,J=2.4,9.1Hz),8.25(1H,d,J= 4.4Hz),8.49(1H,d,J=2.0Hz),9.50(1H,d,J=8.2Hz),11 .52(1H,s). API-ES:376(M+H)⁺,398(M+Na)⁺ |
| 18 | 4 | NMR:1.90-2.16(4H,m),2.76(3H,d,J=4.8Hz),3.04-3.12(1H,m),3.41-3.50(1H,m),3.80-3.86(1H,m),4.08-4. 21(2H,m),4.43-4.48(1H,m),5.03-5.11(1H,m),7.00-7.02(1H,m),7.46-7.48(1H,m),8.69(1H,s),8.96-8.99( 1H,m),10.89(1H,brs),12.07(1H,brs) ESI-MS:431(M+Na)⁺ |
| 19 | 4 | NMR:0.55-0.73(3H,m),1.56-2.46(3H,m),3.11-4.32(10H,m),3.28(3H,s),4.98-5.12(1H,m),6.90-6.99(1H,m ),7.44-7.47(1H,m),8.68-8.19(1H,m),8.90-9.00(1H,m),10.89-10.96(1H,m),12.06(1H,brs) ESI-MS:489(M+Na)⁺ |
| 20 | 4 | NMR:0.81-4.97(30H,m),6.62-6.70(1H,m),7.38-7.57(1H,m),8.33-8.46(1H,m),11.84-11.95(1H,m),12.28(1 H,brs) ESI-MS:516(M+Na)⁺ |
| 21 | 4 | NMR:0.92-0.99(3H,m),1.55-4.60(10H,m),5.97(2H,brs),6.56(1H,brs),7.30-7.39(1H,brs),8.15(1H,s),11 .43-11.55(1H,m) ESI-MS:338(M+H)⁺ |
| 22 | 4 | NMR:0.60-0.74(3H,m),1.58-2.40(3H,m),3.18-4.37(6H,m),4.75-4.92(1H,m),6.77-6.86(1H,m),7.35-7.39( 1H,m),8.49-8.52(1H,m),10.77-11.00(1H,m),11.92(1 H,m)ESI-MS:339(M+H)⁺ |
| 23 | 4 | NMR:0.66-0.72(3H,m),1.37-4.54(10H,m),6.77-7.05(1H,m),7.34-7.39(1H,m),8.46-8.50(1H,m),11.06(1H, brs),11.87-11.96(1H,m) ESI-MS:339(M+H)⁺ |
| 24 | 4 | NMR:0.94-1.03(3H,m),1.62-2.12(2H,m),2.41-4.60(8H,m),6.47-6.53(1H,m),7.38-7.46(1H,m),7.91(1H,s) ,10.78-10.86(1H,brs),11.53-11.64(1H,m) ESI-MS:353(M+H)⁺ |
| 25 | 4 | NMR:0.61-1.06(7H,m),1.63-2.12(2H,m),2.41-4.74(6H,m),6.37(1H,s),6.45-6.50(1H,m),7.41-7.46(1H,m) ,7.91(1H,s),10.80-10.92(1H,brs),11.57-11.68(1H, brs) ESI-MS:356(M+H)⁺ |
| 26 | 4 | NMR:0.97-1.06(3H,m),1.68-4.70(16H,m),6.46-6.49(1H,m),7.42-7.47(1H,m),7.90-7.94(1H,m),10.86-10. 92(1H,brs),11.60-11.66(1H,brs) ESI-MS:397(M+H)⁺ |
| 27 | 4 | NMR:1.01(3H,d,J=7.1Hz),1.32(6H,s),1.62-2.04(2H,m),2.42-5.10(6H,m),5.46(1H,s),6.42-6.48(1H,m),7 .41-7.48(1H,m),7.91(1H,s),10.81-10.90(1H,brs),1 1.57-11.65(1H,brs) ESI-MS:358(M+H)⁺ |
| 28 | 4 | NMR:0.50-0.74(3H,m),1.52-1.90(2H,m),2.20-2.50(1H,m),2.71-2.82(3H,m),3.10-4.33(6H,m),4.97-5.13(1H,m),6.88-7.00(1H,m),7.40-7.50(1H,m),8.62-8.83(1H,m),8.92-9.08(1H,m),10.82-10.92(1H,m),12.00-12.12(1H,m) ESI-MS:445(M+Na)⁺ |
| 29 | 4 | NMR:0.22-0.75(4H,m),1.00-4.35(15H,m),4.97-5.15(1H,m),6.88-7.00(1H,m),7.40-7.50(1H,m),8.67-8.85 (1H,m),9.05-9.20(1H,m),10.80-10.95(1H,m),12.00-12.10(1H,m) ESI-MS:485(M+Na)⁺ |
| 30 | 4 | NMR:0.23-0.29(2H,m),0.42-0.48(2H,m),0.62-0.79(4H,m),1.04(3H,d,J=6.0Hz),1.12-4.56(10H,m),5.00-5 .20(1H,m),6.97(1H,s),7.46(1H,s),8.73-8.78(1H,m),9.09-9.20(1H,m),10.60-11.00(1H,m),12.00-12.13( 1H,brs) ESI-MS:511(M+Na)⁺ |
| 31 | 4 | NMR:0.55-0.74(3H,m),1.60-4.24(11H,m),3.42(3H,s),4.92-5.11(1H,m),6.87-6.95(1H,m),7.43(1H,s),8.7 1-8.82(1H,m),10.25(1H,s),12.04(1H,s) ESI-MS:432(M+Na)⁺ |
| 32 | 4 | NMR:1.20-4.88(13H,m),6.88-6.99(1H,m),7.18-7.22(1H,m),8.49(1H,s),10.41-11.11(1H,brs),11.94(1H,s ) ESI-MS:373(M+Na)⁺ |
| 33 | 4 | NMR:0.99-1.05(3H,m),1.74-1.82(1H,m),2.00-2.19(3H,m),2.47-2.57(3H,m),3.40-3.52(1H,m),3.81-3.89( 2H,m),4.02-4.11(1H,m),4.33-4.40(1H,m),4.42-4.60 (2H,m),6.53-6.59(2H,m),7.22-7.30(1H,m),7.42-7.47(1H,m),7.91(1H,s),10.86(1H,s),11.58-11.64(1H,b rs) ESI-MS:465(M+H)⁺ |
| 34 | 4 | NMR:0.98-1.06(3H,m),1.72-2.06(2H,m),2.74-2.98(6H,m),3.04-4.72(6H,m),6.45-6.50(1H,m),7.42-7.48( 1H,m),7.92-7.94(1H,m),10.89(1H,s),11.60-11.67(1 H,brs) ⁺ ESI-MS:371(M+H)⁺ |
| 35 | 4 | NMR:0.96-1.04(3H,m),1.12-5.60(12H,m),6.38-6.52(1H,m),7.40-7.47(1H,m),7.88-7.95(1H,m),10.81-10. 92(1H,brs),11.54-11.67(1H,m) ESI-MS:344(M+H)⁺ |
| 36 | 4 | NMR:0.92-1.01(3H,m),1.48-4.54(8H,m),5.43-7.95(7H,m),10.71-11.01(1H,brs),11.50-11.68(1H,m) ESI-MS:464(M+Na)⁺ |
| 37 | 6 | NMR:0.97-1.07(1H,m),1.33-1.41(1H,m),1.49-1.86(7H,m),3.21-3.26(2H,m),4.35-4.45(2H,m),6.50-6.56( 1H,m),6.78-7.15(2H,m),7.55-7.87(1H,br),8.35(1H,s),9.95(1H,d,J=8.0Hz),11.42(1H,brs) API-ES:289(M+H)⁺. |
| 38 | 6 | NMR:1.03-2.18(9H,m),3.20-3.30(2H,m),3.81-3.91(1H,m),4.43-4.47(1H,m),6.46-6.50(1H,m),6.90-7.01( 1H,br),7.11-7.14(1H,m),7.57-7.82(1H,br),8.34(1H,s),9.56(1H,d,J=8.0Hz),11.44(1H,brs) API-ES:,289.3(M+H)⁺. |
| 39 | 6 | NMR:5.52(2H,s),6.79-6.81(1H,m),7.21-.7.35(6H,m),8.51(1H,s),11.08(1H,brs),11.94(1H,brs) API-ES:,287.2(M+Na)⁺. |
| 40 | 6 | NMR:1.44-1.93(8H,m),2.39-2.48(1H,m),4.40-4.47(1H,m),6.63-6.66(1H,m),6.71(1H,brs),6.86-7.05(1H, m),7.08-7.12(1H,m),7.28(1H,brs),7.61-7.88(1H,m) ,8.36(1H,s),9.93(1H,d,J=8.0Hz),11.44(1H,brs) API-ES:302(M+H)⁺. |
| 41 | 6 | NMR:1.61-1.73(1H,m),1.98-2.05(1H,m),3.24-3.57(2H,m),4.44-4.59(2H,m),4.79-5.08(2H,m),6.66-6.68( 1H,m),6.94-7.13(2H,m),7.19-7.22(1H,m),7.70-7.92(2H,m),8.39(1H,s),8.48-8.50(1H,m),9.84-9.87(1H, m) , 11.57 (1H,brs) API-ES:380(M+H)⁺_{.} |
| 42 | 6 | NMR:1.57-2.30(7H,m),2.82-2.92(1H,m),3.55-3.61(1H,m),3.70-3.77(1H,m),4.22-4.32(1H,m),4.41-4.44( 1H,m),6.59-6.61(1H,m),6.85-7.12(1H,brs),7.12-7.22(2H,m),7.27-7.36(4H,m),7.62-7.83(1H,brs),8.37 (1H,s),9.76(1H,d,J=8.5Hz),11.4(1H,brs) ESI-MS:365(M+H)⁺ |
| 43 | 6 | NMR:1.52-1.62(2H,m),2.13-2.20(2H,m),3.20-3.52(3H,m),3.99-4.07(2H,m),5.05-5.20(1H,m),7.10-7.20( 1H,brs),7.81-7.95(1H,brs),8.10(1H,s),8.28(1H,d,J=2.1Hz),8.46(1H,s),8.60(1H,d,J=2.1Hz),9.55(1H, d,J=8.6Hz),12.9(1H,brs) ESI-MS:397(M+H)⁺ |
| 44 | 6 | NMR:1.70-2.34(4H,m),3.00-3.10(1H,m),3.25-3.34(1H,m),4.41-4.51(1H,m),4.56-4.66(1H,m),4.70-4.771 (1H,m),6.71-6.73(1H,m),7.01(1H,d,J=9.1Hz),7.35-7.37(1H,m),7.84(1H,dd,J=2.4,9.1Hz),8.47(1H,d,J= 2.4Hz),8.49(1H,s),10.9-11.1(1H,brs),11.9(1H,brs ) ESI-MS:360(M+H)⁺ |
| 45 | 6 | NMR:1.58-2.14(14H,m),4.73-4.80(1H,m),6.67-6.70(1H,m),7.36-7.38(1H,m),8.44(1H,s),10.9-11.0(1H,n rs),11.9(1H,brs) ESI-MS:285(M+H)⁺ |
| 46 | 6 | NMR:1.57-2.01(12H,m),4.67-4.87(1H,m),6.79-6.80(1H,m),7.38-7.39(1H,m),8.49(1H,s),10.9-11.1(1H,b rs),12.0(1H,brs) ESI-MS:271(M+H)⁺ |
| 47 | 6 | NMR:1.45-2.10(7H,m),2.35-2.50(1H,m),2.85(3H,s),3.67-3.71(1H,m),4.45-4.71(1H,m),6.69-6.70(1H,m) ,7.35-7.37(1H,m),8.46(1H,s),10.8-11.0(1H,brs),1 1.9(1H,brs) ESI-MS::287(M+H)⁺ |
| 48 | 6 | NMR:0.88(3H,d,J=6.9Hz),1.72-1.92(2H,m),2.15-2.26(1H,m),3.55-3.70(4H,m),5.30-5.40(1H,m),7.01(1H ,d,J=9.2Hz),7.15(1H,brs),7.76(1H,d,J=9.2Hz),7.9 0(1H,brs),8.09(1H,s),8.39(1H,s),8.48(1H,s),9.89 (1H,d,J=9.0Hz),12.9(1H,brs) ESI-MS:420(M+H)⁺ |
| 49 | 6 | NMR:0.87(3H,d,J=6.9Hz),1.72-1.90(2H,m),2.18-2.26(1H,m),3.62-3.85(4H,m),5.25-5.40(1H,m),6.99(1H ,d,J=9.1Hz),7.18(1H,brs),7.82(1H,dd,J=2.4,9.1Hz ),7.95(1H,brs),8.09(1H,s),8.47(1H,d,J=2.4Hz),8. 48(1H,s),9.86(1H,d,J=9.1Hz),12.8(1H,brs) ESI-MS:377(M+H)⁺ |
| 50 | 6 | NMR:0.92(3H,d,J=7.0Hz),1.82-1.95(2H,m),2.20-2.29(1H,m),3.35-3.65(4H,m),5.28-5.40(1H,m),7.08(1H ,brs),7.90(1H,brs),7.95(1H,s),8.08(1H,s),8.48(1 H,s),9.85(1H,d,J=9.0Hz),12.9(1H,brs) ESI-MS:383(M+H)⁺ |
| 51 | 6 | NMR:0.92(3H,d,J=6.9Hz),1.81-2.00(2H,m),2.22-2.28(1H,m),3.38-3.68(4H,m),5.25-5.38(1H,m),7.10(1H ,brs),7.99(1H,brs),8.09(1H,s),8.31(1H,s),8.49(1 H,s),9.85(1H,d,J=9.0Hz),12<9(1H,brs) ESI-MS:383(M+H)⁺ |
| 52 | 6 | NMR:0.88(3H,d,J=7.0Hz),]-.72-1.92(2H,m),2.62-2.70(1H,m),3.71-4.00(4H,m),5.31-5.42(1H,m),7.02(1H ,d,J=9.8Hz),7.18(1H,brs),7.92(1H,brs),8.10(1H,s ),8.20(1H,dd,J=2.9,9.8Hz),8.49(1H,s),8.96(1H,d,J=2.9Hz),9.87(1H,d,J=9.0Hz),12.9(1H,brs) ESI-MS:397(M+H)⁺ |
| 53 | 6 | ESI-MS:329(M+H)⁺ |
| 54 | 6 | NMR:1.62-2.12(8H,m),5.08-5.16(1H,m);6.80-6.82(1H,m),7.34-7.36(1H,m),8.44(1H,s),10.9-11.0(1H,br s),11.9(1H,brs) ESI-MS::243(M+H)⁺ |
| 55 | 6 | ESI-MS:271(M+H)⁺ |
| 56 | 6 | NMR:1.52-1.64(1H,m),1.69-1.80(1H,m),1.84-1.94(3H,m),2.14-2.22(1H,m),3.59-3.92(4H,m),5.00-5.12( 1H,m),6.79(1H,d,J=9.1Hz),7.02-7.20(1H,brs),7.75 -7.91(1H,brs),7.81(1H,dd,J=2.4,9.1Hz),8.05(1H,s ),8.44(1H,s),8.48(1H,d,J=2.4Hz),9.53(1H,d,J=8.6 Hz),12.8(1H,brs) ESI-MS:377(M+H)⁺ |
| 57 | 6 | NMR:0.88(3H,d,J=7.0Hz),1.75-1.98(2H,m),2.23-2.35(1H,m),3.70-4.00(4H,m),5.35-5.40(1H,m),7.08(1H ,brs),7.42(1H,d,J=9.8Hz),7.84(1H,d,J=9.8Hz),7.9 8(1H,brs),8.10(1H,s),8.49(1H,s),9.87(1H,d,J=8.9 Hz),12.9(1H,brs) ESI-MS:378(M+H)⁺ |
| 58 | 6 | NMR:1.51-1.53(2H,m),2.08(2H,m),3.26-3.29(2H,m),3.78-3.82(2H,m),4.27(1H,m),6.62(1H,dd,J=1.8,3.5 Hz),7.01(1H,brs),7.06(2H,d,J=9.1Hz),7.16-7.17(1H,m),7.57(2H,d,J=9.1Hz),7.79(1H,brs),8.37(1H,s) ,9.74(1H,d,J=7.0Hz),11.50(1H,s). API-ES:361(M+H)⁺,383(M+Na)⁺ |
| 59 | 6 | NMR:1.46-1.48(2H,m),2.08-2.11(2H,m),3.39-3.45(2H,m),4.18-4.21(2H,m),4.30(1H,m),6.64(1H,dd,J=1. 8,3.5Hz),7.01(1H,d,J=9.1Hz),7.02(1H,brs),7.17(1H,m),7.79(1H,dd,J=2.4,9.1Hz),7.82(1H,brs),8.37( 1H,s),8.41(1H,m),9.76(1H,d,J=8.2Hz),11.51(1H,s) API-ES:405(M+H)⁺ |
| 60 | 6 | NMR:1.51-1.54(2H,m),2.12-2.14(2H,m),3.48-3.54(2H,m),4.29-4.33(3H,m),6.65(1H,m),7.02(1H,brs),7. 17-7.19(1H,m),7.79(1H,brs),8.38(1H,s),8.48(1H,d,J=1.4Hz),8.56(1H,d,J=1.4Hz),9.75(1H,d,J=8.1Hz) ,11.52(1H,s). API-ES:363(M+H)+,385(M+Na)⁺ |
| 61 | 6 | NMR:1.44-1.46(2H,m),2.06-2.09(2H,m),3.42-3.47(2H,m),4.29-4.36(3H,m),6.62-6.63(1H,m),7.01(1H,br s),7.17(1H,m),7.78(1H,brs),8.37(1H,s),8.46(2H,s ),9.75(1H,d,J=8.2Hz),11.51(1H,s). API-ES:416(M+H)⁺ |
| 62 | 6 | NMR:1.51-1.53(2H,m),2.13-2.15(2H,m),3.51-3.56(2H,m),4.35(3H,m),6.66(1H,m),7.01(1H,brs),7.17-7. 19(1H,m),7.41(1H,d,J=9.7Hz),7.79(1H,brs),7.86(1 H,d,J=9.7Hz),8.38(1H,s),9.76(1H,d,J=8.2Hz),11.5 2(1H,s). API-ES:363(M+H)⁺,385(M+Na)⁺ |
| 63 | 6 | NMR:1.55-1.62(2H,m),2.13-2.16(2H,m),3.52-3.57(2H,m),3.87-3.90(2H,m),4.31(1H,m),6.63(1H,dd,J=1. 4,3.5Hz),7.04(1H,brs),7.17-7.19(1H,m),7.81(1H,b rs),8.04(1H,s),8.38(1H,s),9.76(1H,d,J=8.2Hz),11 .53(1H,s). API-ES:368 (M+H)⁺,390(M+Na)⁺ |
| 64 | 6 | NMR:1.43-1.51(2H,m),2.09-2.11(2H,m),3.41-3.47(2H,m),3.79(3H,s),4.21-4.22(2H,m),4.30-4.33(1H,m) ,6.63-6.64(1H,m),6.93(1H,d,J=9.0Hz),7.00(1H,brs ),7.16-7.18(1H,m),7.79(1H,brs),7.95(1H,dd,J=2.4,9.0Hz),8.37(1H,s),8.66(1H,d,J=2.1Hz),9.75(1H,d ,J=7.0Hz),11.51(1H,s). API-ES:395(M+H)⁺,417(M+Na)⁺ |
| 65 | 6 | NMR:0.90(3H,d,J=6.8Hz),1.75-1.89(2H,m),2.15-2.21(1H,m),3.59-3.66(1H,m),3.73-3.78(2H,m),3.85-3. 90(1H,m),4.38-4.44(1H,m),6.61-6.62(1H,m),7.00(1 H,d,J=9.2Hz),7.15-7.17(1H,m),7.83(1H,dd,J=2.4,9.1Hz),8.40(1H,s),8.47(1H,d,J=2.2Hz),10.02(1H,d, J=8.6Hz),11.50(1H,brs) ESI-MS:376(M+H)⁺ |
| 66 | 6 | NMR:0.91(3H,d,J=7.0Hz),1.79-1.93(2H,m),2.21-2.27(1H,m),3.72-3.98(4H,m),4.42-4.46(1H,m),6.62-6. 64(1H,m),7.02(1H,br),7.16-7.18(1H,m),7.42(1H,d, J=9.7Hz),7.80(1H,br),7.85(1H,d,J=9.7Hz),8.40(1H ,s),10.03(1H,d,J=8.6Hz),11.51(1H,brs) ESI-MS:377(M+H)⁺ |
| 67 | 6 | NMR:1.06(3H,d,J=7.1Hz),1.75-1.91(2H,m),3.3-3.39(2H,m),3.42(3H,s),4.23-4.30(1H,m),4.53-4.61(1H, m),4.81-4.88(2H,m),7.02(1H,d,J=9.1Hz),7.82(1H,d d,J=2.3,9.1Hz),8.23(1H,s),8.27(1H,s),8.44(1H,d, J=2.3Hz),13.08(1H,brs) EST-MS:411(M+Na)⁺ |
| 68 | 6 | NMR:1.45-1.57(1H,m),1.63-2.07(5H,m),2.17-2.23(1H,m),2.77(3H,d,J=4.9Hz),2.81-2.87(2H,m),5.33-5. 40(1H,m),6.87(1H,brs),7.05-7.07(1H,m),7.38(1H,b rs),7.41-7.43(1H,m),8.69(1H,s),8.97-9.01(1H,m), 10.78(1H,br),12.00(1H,brs) ESI-MS:406(M+Na)⁺ |
| 69 | 6 | NMR:1.06(3H,d,J=7.2Hz),1.74-1.89(2H,m),3.40(2H,m),4.23-4.30(1H,m),4.53-4.59(1H,m),4.76-4.90(2H ,m),7.21(1H,d,J=9.1Hz),7.82(1H,dd,J=2.3,9.1Hz), 8.03(1H,s),8.30(1H,s),8.45(1H,d,J=2.3Hz),11.13( 1H,br),13.02(1H,br) ESI-MS:397(M+Na)⁺ |
| 70 | 6 | NMR:0.67(3H,d,J=7.2Hz),1.41-2.31(9H,m),4.71-4.78(1H,m),6.70-6.74(1H,m),7.34-7.39(1H,m),8.49(1H ,s),10.88(1H,brs),11.92(1H,brs) ESI-MS:271(M+H)⁺ |
| 71 | 6 | NMR:0.75(3H,d,J=6.8Hz),1.75-2.53(3H,m),3.55-3.64(1H,m),3.99-4.06(1H,m),4.34-4.51(2H,m),4.93-4. 98(1H,m),6.83-6.87(1H,m),7.15(1H,d,J=9.6Hz),7.3 4-7.38(1H,m),7.71(1H,d,J=9.6Hz),8.46(1H,s),10.6 4(1H,s),10.91(1H,brs) ESI-MS:375(M+H)⁺ |
| 72 | 6 | NMR:0.78(3H,d,J=7.2Hz),1.78-2.52(3H,m),3.60-3.68(1H,m),4.00-4.12(2H,m),4.30(1H,dd,J=6.4,13.6Hz ),4.85-4.90(1H,m),6.79-6.84(2H,m),7.33-7.38(1H, m),7.72(1H,dd,J=2.4,9.2Hz),8.35(1H,d,J=2.4Hz),8 .47(1H,s),10.74(1H,brs),11.92(1H,brs) ESI-MS:374(M+H)⁺ |
| 73 | 6 | NMR:1.05(3H,d,J=6.8Hz),1.69-1.78(1H,m),2.05-2.17(1H,m),2.47-2.57(1H,m),3.53-3.64(1H,m),4.29-4. 80(4H,m),6.05(2H,brs),6.52-6.65(1H,m),7.30-7.34 (1H,m),7.53(1H,d,J=9.6Hz),7.90(1H,d,J=9.6Hz),8. 16(1H,s),11.48(1H,brs) ESI-MS:374(M+H)⁺ |
| 74 | 6 | NMR:0.77(3H,d,J=6.8Hz),1.78-2.56(3H,m),2.76(3H,d,J=5.2Hz),3.57-3.66(1H,m),4.04-4.16(2H,m),4.42 (1H,dd,J=5.6,144.0Hz),5.03-5.09(1H,m),6.80(1H,d, J=9.2Hz),6.90-6.93(1H,m),7.44(1H,t,J=2.4Hz),7.6 8(1H,dd,J=2.4,9.2Hz),8.26-8.29(1H,m),8.74(1H,s) ,8.96-9.01(1H,m),10.45(1H,brs),12.06(1H,brs) ESI-MS:458(M+H)⁺ |
| 75 | 6 | NMR:0.74(3H,d,J=7.2Hz),1.75-2.57(3H,m),3.28(3H,s),3.35-3.64((5H,m),4.01-4.09(1H,m),4.35-4.66(2H ,m),5.11-5.17(1H,m),6.94-6.98(1H,m),7.15(1H,d,J=9.6Hz),7.44-7.47(1H,m),7.66(1H,d,J=9.6Hz),8.73 (1H,s),8.92(1H,t,J=6.0Hz),10.48(1H,s),12.05(1H, brs) ESI-MS:503(M+H)⁺ |
| 76 | 6 | NMR:0.96(3H,d,J=6.8Hz),1.48-1.73(2H,m),2.12-2.23(1H,m),3.07-3.42(2H,m),4.33-4.55(3H,m),6.63(1H ,d,J=8.8Hz),6.69-6.72(1H,m),6.97(1H,brs),7.19-7.24(1H,m),7.63(1H,dd,J=2.4,8.8Hz),7.74(1H,b.rs), 8.25(1H,d,J=2.4Hz),8.34(1H,s),10.0(1H,brs),11.6 9(1H,brs) ESI-MS:376(M+H)⁺ |
| 77 | 6 | NMR:0.76(3H,d,J=6.8Hz),1.78-1.90(1H,m),2.02-2.24(1H,m),2.47-2.588(1H,m),2.73(3H,d,J=4.8Hz),3.50 -3.62(1H,m),4.03-4.14(1H,m),4.20-4.38(1H,m),4.5 0-4.62(1H,m),5.09-5.15(1H,m),6.75-6.88(1H,m),6.92-6.98(1H,m),7.42-7.48(1H,m),8.02-8.12(1H,m),8 .73(1H,s),8.70-8.80(1H,m),8.88-8.98(1H,m),10.41 (1H,s),12.06(1H,s) ESI-MS:500(M+Na)⁺ |
| 78 | 6 | NMR:0.74(3H,d,J=6.8Hz),1.14-1.25(6H,m),1.72-1.91(1H,m),2.10-2.30(1H,m),3.52-3.65(1H,m),3.90-4. 10(2H,m),4.30-4.70(2H,m),5.10-5.20(1H,m),6.93-7.00(1H,m),7.08-7.20(1H,m),7.40-7.50(1H,m),7.65( 1H,d,J=9.7Hz),8.74(1H,s),8.81(1H,d,J=8.4Hz),10. 41(1H,s),12.05(1H,s) ESI-MS:509(M+Na)⁺ |
| 79 | 6 | NMR:0.22-0.29(2H,m),0.40-0.48(2H,m),1.04(3H,d,J=6.1Hz),1.02-1.11(1H,m),1.74-1.90(1H,m),2.04-2. 20(1H,m),2.40-2.55(1H,m),3.05-3.15(2H,m),3.55-3 .68(1H,m),4.02-4.16(2H,m),4.37-4.47(1H,m),5.02-5.10(1H,m),6.81(1H,d,J=9.2Hz),6.90-6.94(1H,m),7 .42-7.46(1H,m),7.65-7.74(1H,m),8.28(1H,d,J=2.2H z),8.75(1H,s),9.07-9.16(1H,m),10.42-10.54(1H,br s),12.06(1H,s) ESI-MS:498(M+H)⁺ |
| 80 | 6 | NMR:1.12-1.26(3H,m),1.92-2.28(2H,m),3.19(3H,s),3.17-3.55(4H,m),3.93-4.13(3H,m),4.77-4.90(1H,m) ,6.68-6.72(1H,m),6.90-7.20(2H,m),7.67-7.90(1H,b rs),8.74(1H,s),9.76(1H,d,J=8.7Hz),11.50(1H,s) ESI-MS:362(M+H)⁺ |
| 81 | 6 | NMR:1.04(3H,d,J=6.1Hz),1.76-1.86(1H,m),2.08-2.20(1H,m),2.47-2.58(1H,m),3.32(3H,s),3.30-3.43(2H ,m),4.00-4.12(2H,m),4.30-4.62(2H,m),5.09-5.16(1 H,m),6.92-6.96(1H,m),7.08-7.16(1H,m),7.41-7.46(1H,m),7.67(1H,d,J=9.6Hz),8.74(1H,s),9.70-9.96(1 H,brs),12.04(1H,s) ESI-MS:468(M+Na)⁺ |
| 82 | 6 | NMR:1.57-1.71(1H,m),2.12-2.26(1H,m),3.67-4.00(3H,m),4.20-4.32(1H,m),4.40-4.77(2H,m),6.63-6.69( 1H,m),6.995-7.24(3H,m),7.70-7.95(2H,m),8.40(1H,s ),8.52(1H,d,J=2.2Hz),9.96(1H,d,J=8.3Hz),11.56(1 H,s) ESI-MS:380(M+H)⁺ |
| 83 | 6 | NMR:2.03-2.13(1H,m),2.26-2.38(1H,m),4.14-4.23(1H,m),4.40-4.49(1H,m),5.37-5.46(1H,m),6.69-7.23( 6H,m),7.60-7.95(1H,brs),8.42(1H,s),9.43(1H,d,J= 8.2Hz),11.58(1H,s) ESI-MS:327(M+H)⁺ |
| 84 | 6 | NMR:0.75(3H,d,J=6.8Hz),1.78-1.90(1H,m),2.20-2.37(1H,m),2.42-2.55(1H,m),2.76(3H,d,J=4.8Hz),3.52 -3.62(1H,m),3.86-4.01(2H,m),4.19-4.30(1H,m),5.1 3-5.20(1H,m),6.94-6.98(1H,m),7.42-7.48(1H,m),7.85(1H,s),8.73(1H,s),8.93-9.03(1H,m),10.60-10.70 (1H,brs),12.02-12.11(1H,brs) ESI-MS:486(M+Na)⁺ |
| 85 | 6 | NMR:1.01(3H,d,J=6.8Hz),1.60-1.80(2H,m),2.21-2.69(2H,m),2.58(3H,s),3.14-3.47(2H,m),4.50-4.68(2H ,m),6.77(1H,d,J=3.7Hz),6.86-6.94(1H,m),7.26-7.30(1H,m),7.38(1H,d,J=9.3Hz),7.49(1H,d,J=9.3Hz),8 .57(1H,s),11.66-11.72(1H,brs) ESI-MS:416(M+H)⁺ |
| 86 | 6 | NMR:1.88-2.02(2H,m),2.12-2.24(2H,m),3.26-3.62(2H,m),3.90-4.00(1H,m),4.16-4.26(1H,m),4.73-4.83( 1H,m),6.81-6.85(1H,m),7.36-7.40(1H,m),8.01(1H,s),8.50(1H,s),10.98-11.13(1H,brs),11.92-12.00(1H ,brs) ESI-MS:366(M+H)⁺ |
| 87 | 6 | NMR:1.74-2.00(2H,m),2.11-2.37(2H,m),3.12-3.22(1H,m),3.41-3.52(1H,m),4.50-4.61(1H,m),4.64-4.88( 2H,m),6.74-6.79(1H,m),7.32-7.37(1H,m),7.43(1H,d,J=9.7Hz),7.86(1H,d,J=9.7Hz),8.50(1H,s),10.90-1 1.16(1H,brs),11.93(1H,s) ESI-MS:361(M+H)⁺ |
| 88 | 6 | NMR:2.06-3.85(6H,m),3.17(3H,s),4.32-4.48(1H,m),4.90-5.00(1H,m),6.62-6.72(2H,m),6.97-7.20(2H,m) ,7.70-7.90(2H,m),8.40(1H,s),8.48(1H,s),9.86(1H, d,J=9.4Hz),11.51(1H,brs) ESI-MS:392(M+H)⁺ |
| 89 | 6 | NMR:1.42-2.06(8H,m),2.70-3.90(2H,brs),4.13-4.30(1H,m),4.80-5.00(1H,m),6.52-6.59(1H,m),6.97-7.2 2(2H,m),7.68-7.92(1H,m),8.38(1H,s),10.00(1H,d,J =8.8Hz),11.63(1H,s) ESI-MS:299(M+Na)⁺ |
| 90 | 6 | NMR:1.74-1.87(1H,m),2.01-2.07(1H,m),3.51-3.62(1H,m),3.76(1H,dd,J=14.0,40.5Hz),3.97-4.02(1H,m), 4.32-4.42(1H,m),4.45-4.57(1H,m),5.06(1H,d,J=49. 0Hz),6.65-6.68(1H,m),6.98-7.16(1H,brs),7.20-7.22(1H,m),7.64-7.96(1H,brs),8.02(1H,s),8.40(1H,s) ,9.88(1H,d,J=9.0Hz),11.6(1H,s) ESI-MS:384(M-H)⁻ |
| 91 | 6 | NMR:1.67-1.74(1H,m),2.02-2.09(1H,m),3.30-3.43(1H,m),3.60(1H,dd,J=14.8,40.0Hz),4.48-4.67(2H,m), 4.85-4.98(1H,m),5.06(1H,d,J=49.2Hz),6.68-6.72(1H,m),6.82-7.14(1H,brs),7.20-7.23(1H,m),7.51(1H, d,J=9.8Hz),7.81(1H,d,J=9.8Hz),7.62-7.90(1H,brs),8.39(1H,s),9.86(1H,d,J=8.8Hz),11.6(lH,s) ESI-MS:422(M-H)⁻ |
| 92 | 6 | ESI-MS:398(M+H)⁺ |
| 93 | 6 | NMR:1.61-1.73(1H,m),1.98-2.05(1H,m),3.24-3.57(2H,m),4.44-4.59(2H,m),4.79-5.08(2H,m),6.66-6.68(1H,m),6.94-7.13(2H,m),7.19-7.22(1H,m),7.70-7.92 (2H,m),8.39(1H,s),8.48-8.50(1H,m),9.84-9.87(1H, m),11.57(1H,brs) API-ES:380(M+H)⁺. [α]_{D}²⁷-7.63(c 0.99, DMF) |
| 94 | 6 | NMR:1.61-1.73(1H,m),1.98-2.05(1H,m),3.24-3.57(2H,m),4.44-4.59(2H,m),4.79-5.08(2H,m),6.66-6.68( 1H,m),6.94-7.13(2H,m),7.19-7.22(1H,m),7.70-7.92(2H,m),8.39(1H,s),8.48-8.50(1H,m),9.84-9.87(1H, m),11.57(1H,brs) API-ES:380(M+H)⁺. [α]_{D}²⁷+7.40(c 1.02, DMF) |
| 95 | 6 | NMR:1.66-1.78(1H,m),2.02-2.10(1H,m),3.34-3.71(2H,m),4.49-4.72(2H,m),4.87-5.14(2H,m),6.67-6.71(1H,m),7.03(1H,brs),7.20-7.24(1H,m),7.47(1H,d,J=9.6Hz),7.79(1H,brs),7.89(1H,d,J=9.6Hz),8.39(1H, s),9.86(1H,d,J=9.2Hz),11.57(1H,brs) ESI-MS:381(M+H)⁺ [α]_{D}²⁶-10.0(c 0.47, DMF) |
| 96 | 6 | NMR:1.66-1.78(1H,m),2.02-2.10(1H,m),3.34-3.71(2H,m),4.49-4.72(2H,m),4.87-5.14(2H,m),6.67-6.71( 1H,m),7.03(1H,brs),7.20-7.24(1H,m),7.47(1H,d,J=9.6Hz),7.79(1H,brs),7.89(1H,d,J=9.6Hz),8.39(1H, s),9.86(1H,d,J=9.2Hz),11.57(1H,brs) ESI-MS:381(M+H)⁺ [α]_{D}²⁴+13.9(c 0.50, DMF) |
| 97 | 6 | NMR:1.71-1.81(1H,m),1.96-2.03(1H,m),3.21-3.28(1H,m),3.36-3.51(1H,m),3.96-4.02(1H,m),4.27-4.36( 1H,m),4.41-4.55(1H,m),4.92-5.07(1H,m),6.65-6.67 (1H,m),7.04(1H,br),7.07(2H,d,J=9.1Hz),7.20-7.22(1H,m),7.57(2H,d,J=9.1Hz),7.80(1H,br),8.39(1H,s ),9.89(1H,d,J=8.6Hz),11.59(1H,brs) ESI-MS:379(M+H)⁺ [α]_{D}^{3.6} +2.2 (c 0.50, DMF) |
| 98 | 6 | NMR:1.88-1.96 (1H, m), 3.00-3.13 (1H, m), 3.26-3.36 (1H, m), 3.50-3.67 (1H, m), 4.68-5.06 (4H, m), 6.34-6.38 (1H, m), 7.12 (1H, d, J = 9.2 Hz), 7.32-7.36 (1H, m), 7.91 (1H, dd, J = 9.2, 2.4 Hz), 7.93 (1H, s), 8.53-8.55 (1H, s), 11.0 (1H, brs), 11.52 (1H, brs) ESI-MS:376(M-H)⁻ |
| 99 | 7 | NMR:1.58-1.88(8H,m),2.05-2.13(4H,m),3.28(3H,s),3.37-3.50(4H,m),4.90-4.99(1H,m),6.84-6.88(1H,m) ,7.43-7.47(1H,m),8.68(1H,s),8.94(1H,t,J=5.6Hz), 10.85(1H,brs),12,04(1H,brs) ESI-MS:399(M+H)⁺ |
| 100 | 7 | NMR:0.61(3H,t,J=7.5Hz),0.96-1.27(2H,m),1.40-2.20(7H,m),2.77(3H,d,J=4.8Hz),3.30(2H,d,J=7.5Hz),4 .98-5.07(1H,m),6.81-6.84(1H,m),7.42-7.46(1H,m), 8.70(1H,s),8.95-9.05(1H,m),10.80-10.90(1H,brs), 11.98-12.08(1H,brs) ESI-MS:369(M+H)⁺ |
| 101 | 7 | NMR:0.61(3H,t,J=7.4Hz),0.96-1.30(2H,m),1.40-3.52(3H,m),3.28(3H,s),4.98-5.07(1H,m),6.81-6.87(1 H,m),7.41-7.47(1H,m),8.70(1H,s),8.90-9.00(1H,m) ,10.88-10.95(1H,brs),12.00-12.10(1H,brs) ESI-MS:413(M+H)⁺ |
| 102 | 7 | NMR:1.64-1.95(4H,m),1.97-2.26(4H,m),2.77(3H,d,J=4.8Hz),5.27-5.38(1H,m),6.92(1H,d,J=3.0Hz),7.41 -7.46(1H,m),8.67(1H,s),8.94-9.04(1H,m),10.78-10 .90(1H,brs),11.96-12.10(1H,brs) ESI-MS:327(M+H)⁺ |
| 103 | 7 | NMR:1.58-1.90(8H,m),2.05-2.15(4H,m),2.77(3H,d,J =4.8Hz),4.89-4.98(1H,m),6.85-6.90(1H,m),7.42-7. 47(1H,m),8.68(1H,s),8.94-9.02(1H,m),10.79-10.90 (1H,brs),12.00-12.08(1H,brs) ESI-MS:355(M+H)⁺ |
| 104 | 7 | NMR:0.65(3H,d,J=7.0Hz),1.40-2.05(7H,m),2.20-2.36(2H,m),2.77(3H,d,J=4.8Hz),4.90-4.98(1H,m),6.80 -6.85(1H,m),7.40-7.47(1H,m),8.69(1H,s),8.93-9.0 4(1H,m),10.83(1H,s),11.98-12.07(1H,brs) ESI-MS:355(M+H)⁺ |
| 105 | 7 | NMR:0.65(3H,d,J=7.0Hz),1.18(6H,d,J=6.6Hz),1.41-2.05(7H,m),2.19-2.37(2H,m),3.95-4.10(1H,m),4.90 -5.00(1H,m),6.80-6.85(1H,m),7.41-7.48(1H,m),8.7 1(1H,s),8.84(1H,d,J=12.3Hz),10.84(1H,s),12.02(1 H,s) ESI-MS:405(M+Na)⁺ |
| 106 | 8 | NMR:0.85(3H,d,J=6.9Hz),1.70-1.80(2H,m),2.07-2.18(1H,m),2.85(3H,s),3.20-3.35(4H,m),4.09(2H,s),5 .21-5.30(1H,m),7.15(1H,brs),7.80(1H,brs),8.07(1 H,s),8.47(1H,s),9.81(1H,d,J=9.2Hz),12.8(1H,brs) ESI-MS:371(M+H)⁺ |
| 107 | 8 | NMR:0.87(3H,d,J=6.9Hz),1.70-1.79(2H,m),2.04-2.12(1H,m),2.74(6H,s),3.00-3.24(4H,m),5.20-5.31(1H,m ),7.00-7.22(1H,brs),7.71-7.99(1H,brs),8.06(1H,s) ,8.47(1H,s),9.81(1H,d,J=9.2Hz),12.8(1H,brs) ESI-MS:346(M+H)⁺ |
| 108 | 8 | NMR:0.77(3H,d,J=6.8Hz),1.76-1.84(1H,m),1.96-2.06(1H,m),2.31-2.40(1H,m),2.67(6H,s),3.07-3.17(1H,m ),3.33-3.42(1H,m),3.46(1H,dd,J=3.6,13.2Hz),3.69( 1H,dd,J=7.2,13.2Hz),4.79-4.84(1H,m),6.72-6.'77(1H ,m),7.35-7.39(1H,m),8.49(1H,s),10.83(1H,brs),11. 92(1H,brs) ESI-MS:343(M+H)⁺ |
| 109 | 8 | NMR:0.77(3H,d,J=7.2Hz),1.73-2.45(3H,m),2.64(6H,s),3.05-3.13(1H,m),3.28(3H,s),3.38-3.50(5H,m),3.7 3-3.80(1H,m),4.98-5.03(1H,m),6.85-6.88(1H,m),7.4 4-7.47(1H,m),8.72(1H,s),8.96(1H,t,J=6.0Hz),10.86 (1H,brs),12.06(1H,brs) ESI-MS:471(M+H)⁺ |
| 110 | 8 | NMR:1.01(3H,d,J=7.2Hz),1.63-2.54(3H,m),2.65-2.73 (2H,m),3.11-3.22(1H,m),3.52-3.61(2H,m),3.95-4.67 (4H,m),6.40-6.43(1H,m),6.98-7.01(1H,m),7.41-7.44 (1H,m),7.91(1H,s),10.82(1H,brs),11.59(1H,brs) ESI-MS:368(M+H)⁺ |
| 111 | 8 | NMR:0.70(3H,d,J=7.0Hz),1.67-1.82(1H,m),1.91-2.05 (1H,m),2.36-2.48(1H,m),2.77(3H,d,J=4.8Hz),3.30-3 .43(1H,m),3.55-3.68(1H,m),3.72-3.82(1H,m),3.91-4 .06(3H,m),4.88-4.97(1H,m),6.89(1H,d,J=3.0Hz),7.0 8-7.18(1H,m),7.40-7.47(1H,m),8.74(1H,s),8.94-9.0 5(1H,m),10.68(1H,s),12.05(1H,s) ESI-MS:460(M+Na)⁺ |
| 112 | 8 | NMR:0.61(3H,d,J=6.3Hz),1.58-1.70(1H,m),2.21-2.42 (2H,m),2.77(3H,d,J=4.8Hz),3.15-3.30(1H,m),3.51-3 .60(1H,m),3.75-3.85(1H,m),3.93-4.02(1H,m),4.21(2 H,d,J=17.8Hz),4.35(2H,d,J=17.8Hz),5.05-5.12(1H,m ),6.93-6.98(1H,m),7.42-7.48(1H,m),8.72(1H,s),8.9 2-9.00(1H,m),11.03(1H,s),12.02-12.10(1H,brs) ESI-MS:499(M+Na)⁺ |
| 113 | 8 | NMR:0.77(3H,d,J=6.9Hz),1.70-1.82(1H,m),2.10-2.20 (1H,m),2.30-2.45(1H,m),2.64(6H,s),2.77(3H,d,J=4. 8Hz),3.05-3.15(1H,m),3.40-3.50(2H,m),3.72-3.82(1 H,m),4.95-5.05(1H,m),6.85-6.90(1H,m),7.42-7.49(1 H,m),8.72(1H,s),8.95-9.05(1H,m),10.72-10.85(1H,m ),12.06(1H,s) ESI-MS:449(M+Na)⁺ |
| 114 | 8 | NMR:0.71(3H,d,J=6.8Hz),1.65-1.75(1H,m),2.15-2.27(1H,m),2.30-2.42(1H,m),2.73(3H,s),2.77(3H,d,J= 4.8Hz),3.10-3.20(1H,m),3.52-3.58(1H,m),3.61-3.7 0(1H,m),3.82-3.92(1H,m),3.96-4.10(2H,m),5.00-5.07(1H,m),6.88-6.95(1H,m),7.42-7.47(1H,m),8.72(1 H,s),8.96-9.05(1H,m),10.85(1H,s),12.06(1H,s) ESI-MS:474(M+Na)⁺ |
| 115 | 8 | NMR:0.23-0.28(2H,m),0.40-0.50(2H,m),1.04(3H,d,J=6.1Hz),1.00-1.10(1H,m),1.62-1.80(1H,m),2.02-2. 16(1H,m),2.30-2.45(1H,m),3.07-4.07(9H,m),4.07-4 .15(1H,m),4.20-4.30(1H,m),4.92-5.00(1H,m),6.90-6.95(1H,m),7.42-7.48(1H,m),8.73(1H,s),9.07-9.15 (1H,m),10.85-10.95(1H,brs),12.04-12.12(1H,brs) ESI-MS:526(M+Na)⁺ |
| 116 | 8 | NMR:0.22-0.30(2H,m),0.40-0.49(2H,m),1.04(3H,d,J=6Hz),1.00-2.53(8H,m),3.07-3.15(2H,m),3.27-2.58 (2H,m),4.00-4.20(2H,m),4.96-5.05(1H,m),5.08-5.1 5(1H,m),5.58-5.67(1H,m),6.97-7.03(1H,m),7.40-7.50(1H,m),8.74(1H,m),9.08-9.17(1H,m),11.26(1H,s) ,12.05(1H,s) ESI-MS:565(M+Na)⁺ |
| 117 | 8 | NMR:0.72(3H,d,J=6.8Hz),1.18(6H,d,J=6.6Hz),1.67-1.74(1H,m),2.12-2.29(1H,m),2.31-2.42(1H,m),2.72 (3H,s),3.10-3.21(1H,m),3.52-3.70(2H,m),3.82-3.9 2(1H,m),3.95-4.10(3H,m),4.99-5.04(1H,m),6.88-6.93(1H,m),7.42-7.49(1H,m),8.73(1H,s),8.80-8.86(1 H,m),10.80-10.90(1H,brs),12.03-12.13(1H,brs) ESI-MS:502(M+Na)⁺ |
| 118 | 8 | NMR:0.23-0.30(2H,m),0.40-0.49(2H,m),1.04(3H,d,J=6.0Hz),1.68-1.77(1H,m),2.14-2.42(2H,m),2.72(3H ,s),3.04-3.20(3H,m),3.50-3.70(2H,m),3.82-3.92(1 H,m),3.95-4.11(2H,m),5.00-5.07(1H,m),6.55(1H,s) ,6.88-6.92(1H,m),7.42-7.48(1H,m),8.73(1H,s),9.0 7-9.16(1H,m),10.80-10.92(1H,brs),12.00-12.10(1H ,brs) ⁺ ESI-MS:492(M+H)⁺ |
| 119 | 8 | NMR:0.72(3H,d,J=6.9Hz),1.70-1.82(1H,m),2.03-2.18(1H,m),2.14(3H,s),2.32-2.48(1H,m),2.71(3H,s),3 .14-3.24(1H,m),3.51-3.64(2H,m),3.78-3.90(1H,m), 4.00-4.10(2H,m),4.95-5.02(1H,m),6.82-6.88(1H,m) ,7.38-7.44(1H,m),8.72-8.80(1H,brs),10.42-10.55( 1H,brs),11.96-12.05(1H,brs) ESI-MS:431(M+Na)⁺ |
| 120 | 8 | NMR:0.80(3H,d,J=6.2Hz),1.20-1.50(2H,m),1.55-3.40(11H,m),2.77(3H,d,J=4.9Hz),3.42-3.90(3H,m),4.9 5-5.05(1H,m),6.88-6.94(1H,m),7.42-7.50(1H,m),8. 72(1H,s),8.95-9.05(1H,m),10.79-10.87(1H,brs),12 .02-12.10(1H,brs) |
| 121 | 18 | NMR:1.62-3.90(14H,m),4.60-4.71(1H,m),6.87-6.93(1H,m),7.37-7.42(1H,m),8.48(1H,s),10.88-11.12(1H ,brs),11.92(1H,s) ESI-MS:391(M+H)⁺ |
| 122 | 8 | NMR:1.03(3H,d,J=7.0Hz),1.64-3.38(8H,m),3.60-3.7 7(1H,m),3.93-4.04(1H,m),4.33-4.71(2H,m),4.94-5. 06(2H,m),6.37-6.43(1H,m),7.41-7.48(1H,m),7.91-7 .94(1H,m),10.88(1H,s),11.56-11.66(1H,brs) ESI-MS:441(M+Na)⁺ |
| 123 | 8 | ESI-MS:419(M+H)⁺ |
| 124 | 8 | NMR:1.00(3H,d,J=7.1Hz),1.68-1.76(1H,m),1.96-2.0 8(1H,m),2.32-2.53(6H,m),3.20-3.31(1H,m),3.40-3. 53(4H,m),3.73-3.82(1H,m),4.26-4.37(1H,m),4.52-4 .61(1H,m),6.45-6.50(1H,m),7.41-7.47(1H,m),7.91( 1H,s),10.83(1H,s),11.58-11.64(1H,brs) ESI-MS:397(M+H)⁺ |
| 125 | 8 | NMR:0.95-1.02(3H,m),1.60-4.66(13H,m),5.12-5.38( 1H,m),6.40-6.48(1H,m),6.94-7.10(1H,m),7.20-7.31 (1H,m),7.40-7.48(1H,m),7.89-7.96(1H,m),10.82(1H ,m),11.53-11.64(1H,m) ESI-MS:430(M+H)⁺ |
| 126 | 8 | NMR:0.98(3H,d,J=7.1Hz),1.32-1.47(2H,m),1.64-3.7 3(17H,m),4.18-4.30(1H,m),4.50-4.59(1H,m),6.41-6 .47(1H,m),7.41-7.46(1H,m),7.91(1H,s),10.79(1H,s ),11.58(1H,brs) ESI-MS:452(M+H)⁺ |
| 127 | 8 | NMR:0.99(3H,d,J=7.2Hz),1.60-1.65(1H,m),1.84-1.9 3(1H,m),2.42-2.47(1H,m),2.74(6H,s),3.05-3.14(1H,m),3.35-3.42(1H,m),3.53-3.60(1H,m),4.56-4.66(1 H,m),4.83-4.89(1H,m),8.01(1H,s);8.33(1H,s),11.0 9(1H,br),13.00(1H,br) ESI-MS:366(M+Na)⁺ |
| 128 | 10 | NMR:0.98(3H,d,J=7.1Hz),1.66-1.75(1H,m),1.91-2.0 2(1H,m),3.32(3H,s),2.41-4.49(10H,m),6.49-6.54(1 H,m),7.41-7.45(1H,m),7.91(1H,s),10.85(1H,s),11. 61(1H,s) ESI-MS:396(M+Na)⁺ |
| 129 | 12 | NMR:0.60(3H,t,J=7.4Hz),1.02-2.60(11H,m),4.72-4.80(1H,m),5.59(1H,s),6.55(1H,s),6.63-6.69(1H,m), 7.27-7.32(1H,m),8.54(1H,s),11.70-11.80(1H,brs) ESI-MS:284(M+H)⁺ |
| 130 | 14 | NMR:1.52-1.56(2H,m),1.99-2.02(2H,m),2.27-2.32(2H,m),2.68(2H,m),3.57(2H,s),3.99(1H,m),6.49(1H,d d,J=1.8,3.6Hz),6.81(1H,brs),7.12-7.13(1H,m),7.55(1H,t,J=7.6Hz),7.68-7.76(4H,m),8.36(1H,s),9.71 (1H,d,J=7.0Hz),11.46(1H,s). APT-ES:375(M+H)+,397(M+Na)+ |
| 131 | 15 | NMR:1.19-1.82(10H,m),3.42(3H,s),4.20-4.30(1H,m) ,6.79(1H,d,J=3.5Hz),7.52(1H,d,J=3.5Hz),8.45(1H, s),12.2(1H,brs) ESI-MS:293(M+Na)+ |
| 132 | 15 | NMR(CDCl3)δ:1.47-2.07(14H,m),3.54(3H,s),4.60-4.69(1H,m),6.69(1H,d,J=3.6Hz),7.37(1H,d,J=3.6Hz), 8.72(1H,s),10.4(1H,s) ESI-MS:299(M+H)+ |
| 133 | 15 | NMR(CDCl3)δ:1.46-2.40(14H,m),4.13(3H,s),4.77-4.85(1H,m),6.74(1H,d,J=3.4Hz),7.31(1H,d,J=3.4Hz), 8.57(1H,s),10.3(1H,s) ESI-MS:299(M+H)+ |
| 134 | 15 | NMR:1.55-2.20(14H,m),3.34(3H,s),3.74-3.76(2H,m),4.45-4.49(2H,m),4.81-4.86(1H,m),6.76-6.77(1H,m ),7.43-7.44(1H,m),8.50(1H,s),12.1(1H,brs) EST-MS:343(M+H)+ |

The compounds in A1 to A6 shown in the following Table can be produced using the corresponding starting materials in a similar manner to that of the above-mentioned Preparations and Examples.

**Table 7**

| No | Structure | No | Structure |
|---|---|---|---|
| A1 | 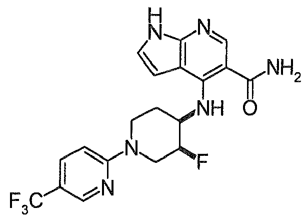 | A2 | 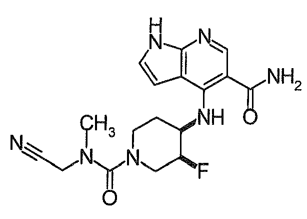 |
| A3 | 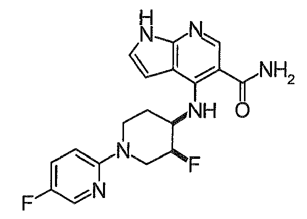 | A4 | 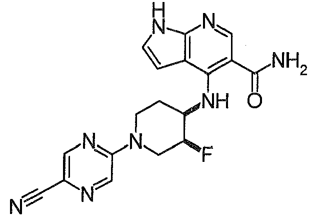 |
| A5 | 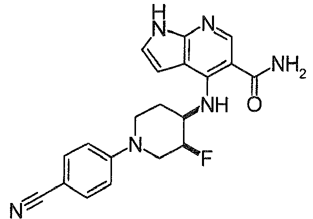 | A6 | 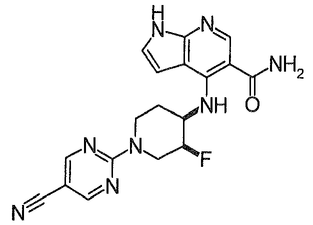 |

## Claims

1. A condensed pyridine compound represented by the following formula (I): 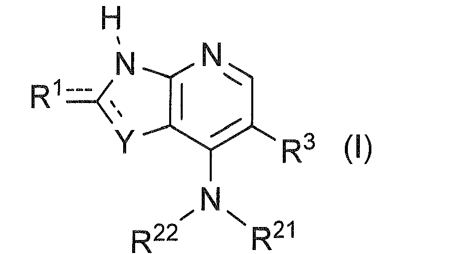 [wherein
R¹ is -H or =O;
R³ is carbamoyl or oxadiazolyl,
each of which may be substituted with C₁-C₆ alkyl;
R²¹ is -H or may be bonded with R³ via a certain functional group to form divalent groups represented by the following formulas (IA), (IB), (IC) or (ID): 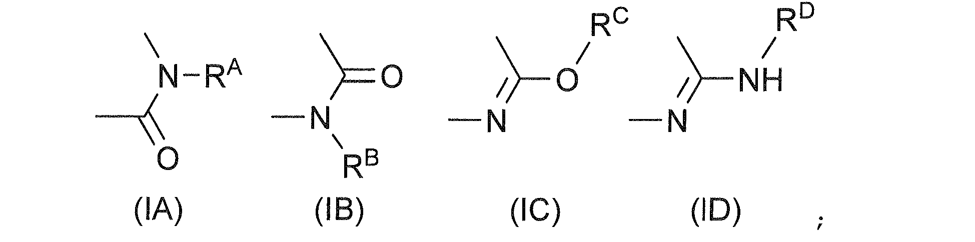
R^{A} is -H or C₁-C₆ alkyl;
R^{B} is -H or C₁-C₆ alkyl;
R^{c} is -H, C₁-C₆ alkyl or (C₁-C₆ alkyl)-O-(C₁-C₆ alkyl,) ;
R^{D} is -H, -C(=O)-(C₁-C₆ alkyl,), -C(=O)-(C₁-C₆ alkyl)-O-(C₁-C₆ alkyl) , -C(=O)C (=O) NH- (C₁-C₆ alkyl) , -C (=O) C(=O) NH- (C₁-C₆ alkyl)-O-(C₁-C₆ alkyl) or -C(=O)O-(alkyl);
R²² is 5- to 7-membered nitrogen-containing heterocycloalkyl, C₃-C₉ cycloalkyl, benzopyranyl or benzyl,
each of which may be substituted with one or more identical or different group(s) selected from the group consisting of R^{v};
R^{v} is halogen, C₁-C₆ alkyl, -O-(C₁-C₆ alkyl), aryl, 5- to 6- membered nitrogen-containing heteroaryl, benzyl, carbamoyl, -(C=O)- (C₁-C₆ alkyl), -C(=O)-(5- to 6- membered heteroaryl), -C(=O)-(5- to 6-membered nitrogen-containing heterocycloalkyl), -C(=O)-carbamoyl, -C(=O)C(=O)-(5- to 6-membered nitrogen-containing heterocycloalkyl), -C(=O)O-(C₁-C₆ alkyl) ;
each of which may be substituted with one or more identical or different group(s) selected from the group consisting of R^{w};
R^{w} is halogen, -CN, -CF₃, -OH, =O, -NO₂, carbamoyl, oxime, C₁-C₆ alkyl, -(C₁-C₆ alkyl)-CN, -(C₁-C₆ alkyl)-OH, -C(=O)O-(C₁-C₆ alkyl), -O-(C₁-C₆ alkyl), -O-(C₁-C₆ alkyl)-CN, 2-pyrroridinone-1-yl or phenyl, wherein phenyl may be substituted with halogen;
Y is N, CH or CH₂; and
--- is a single bond or a double bond,
wherein the one is single bond, the other is double bond; or pharmaceutically acceptable salts thereof, or prodrug thereof.

2. A compound of claim 1 having the following formula (II): 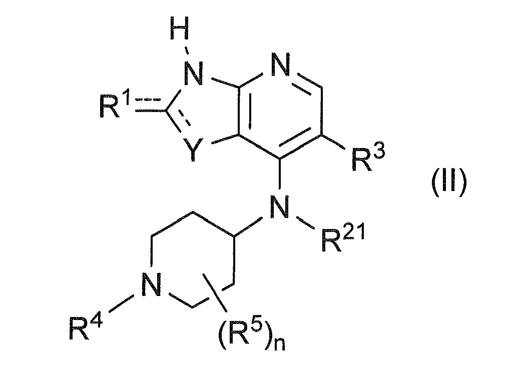 wherein
R¹ is -H or =O;
R³ is carbamoyl or oxadiazolyl,
each of which may be substituted with C₁-C₆ alkyl;
R²¹ is -H or may be bonded with R³ via a certain functional group to form divalent groups represented by the following formula (IA): 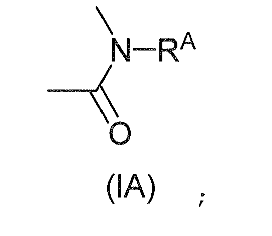
R^{A} is -H or C₁-C₆ alkyl;
R⁴ is aryl, 5- to 6- membered heteroaryl, benzyl, carbamoyl, -C(=O)-(5-to 6- membered heteroaryl), -C(=O)-(5- to 6-membered nitrogen-containing heterocycloalkyl), -C (=O) -carbamoyl, -C (=O) C (=O) - (5- to 6- membered nitrogen-containing heterocycloalkyl), -C (=O) O- (C₁-C₆ alkyl),
each of which may be substituted with one or more identical or different group (s) selected from the group consisting of R⁴¹;
R⁴¹ is halogen, -CN, -CF₃, -OH, =O, -NO₂, carbamoyl, oxime, C₁-C₆ alkyl, -(C₁-C₆ alkyl) -CN, - (C₁-C₆ alkyl) -OH, -C(=O)O-(C₁-C₆ alkyl) , -O-(C₁-C₆ alkyl) , -O-(C₁-C₆ alkyl)-CN, 2-pyrroridinone-1-yl or phenyl, wherein phenyl may be substituted with halogen;
R⁵ is halogen or -O-(C₁-C₆ alkyl);
n is an integer from 1 to 6;
Y is N, CH or CH₂; and
- is a single bond or a double bond,
wherein the one is single bond, the other is double bond; or pharmaceutically acceptable salts thereof, or prodrug thereof.

3. A compound of claim 2, wherein:
R⁴ is aryl or 5- to 6-membered nitrogen-containing heteroaryl, or -C (=O)O- (C₁-C₆ alkyl), each of which may be substituted with one or more identical or different group(s) selected from the group consisting of R⁴¹; and
R⁴¹ is halogen, -CN, -CF₃, -CH₂OH, -CONH₂, -C(=O)O-(C₁-C₆ alkyl) or -NO₂;
or pharmaceutically acceptable salts thereof, or prodrug thereof.

4. A compound of claim 3, wherein:
R⁵ is halogen;
or pharmaceutically acceptable salts thereof, or prodrug thereof.

5. A compound of claim 4, wherein:
R³ is -CONH₂ or -C(=O)NH-(C₁-C₆ alkyl);
Y is CH; and
R¹ is -H;
or pharmaceutically acceptable salts thereof, or prodrug thereof.

6. A compound of claim 5, which is :
(1) rel-4-{[(3R,4S)-1-(6-Cyanopyridazin-3-yl)-3-fluoropiperidin-4-yl]aminol-1H-pyrrolo[2,3-b]pyridine-5-carboxamide,
(2) rel-4-{[(3R,4S)-1-(5-Cyanopyridin-2-yl)-3-fluoropiperidin-4-yl]aminol-1H-pyrrolo[2,3-blpyridine-5-carboxamide,
(3) Ethyl rel-(3R,4S)-4-[(5-carbamoyl-1H-pyrrolo[2,3-b]pyridin-4-yl)amino]-3-methoxypiperidine-1-carboxylate,
(4) rel-4-{[(3R,4R)-1-(5-Cyanopyridin-2-yl)-3-fluoropiperidin-4-yl]amino}-1H-pyrrolo[2,3-b]pyridine-5-carboxamide,
(5) rel-4-{[(3R,4S)-1-(5-Cyanopyridin-2-yl)-3-methoxypiperidin-4-yl]aminol-1H-pyrrolo[2,3-b]pyridine-5-carboxamide,
(6) rel-4-{[(3R,4S)-1-(5-Cyano-1,3-thiazol-2-yl)-3-fluoropiperidin-4-yl]aminol-1H-pyrrolo[2,3-b]pyridine-5-carboxamide,
(7) rel-4-({(3R,4S)-3-Fluoro-1-[6-(trifluoromethyl)pyridazin-3-yl]piperidin-4-yl}amino)-1H-pyrrolo[2,3-b]pyridine-5-carboxamide,
(8) 4-{[1-(5-Cyanopyridin-2-yl)-3,3-difluoropiperidin-4-yl]amino}-1H-pyrrolo[2,3-b]pyridine-5-carboxamide,
(9) rel-6-[(3R,4S)-3-Fluoro-4-(2-oxo-3,6-dihydroimidazo[4,5-d]pyrrolo[2,3-b]pyridin-1(2H)-yl)piperidin-1-y1]nicotinonitrile,
(10) 4-{[(3S,4R)-1-(5-Cyanopyridin-2-yl)-3-fluoropiperidin-4-yl]amino}-1H-pyrrolo[2,3-b]pyridine-5-carboxamide,
(11) 4-{[(3S,4R)-1-(6-Cyanopyridazin-3-yl)-3-fluoropiperidin-4-yl]amino}-1H-pyrrolo[2,3-b]pyridine-5-carboxamide,
(12) 4-{[(3R,4S)-1-(6-Cyanopyridazin-3-yl)-3-fluoropiperidin-4-yl]amino}-1H-pyrrolo[2,3-b]pyridine-5-carboxamide,
(13) 4-{[(3R,4S)-1-(4-Cyanophenyl)-3-fluoropiperidin-4-yllaminol-1H-pyrrolo[2,3-b]pyridine-5-carboxamide, and
(14) rel-6-[(3R,4S)-3-Fluoro-4-(2-oxo-3,6-dihydroimidazo[4,5-d]pyrrolo[2,3-b]pyridin-1(2H)-yl)piperidin-1-yl]nicotinonitrile
or pharmaceutically acceptable salts thereof, or prodrug thereof.

7. A compound of claim 1 having the following formula (III): 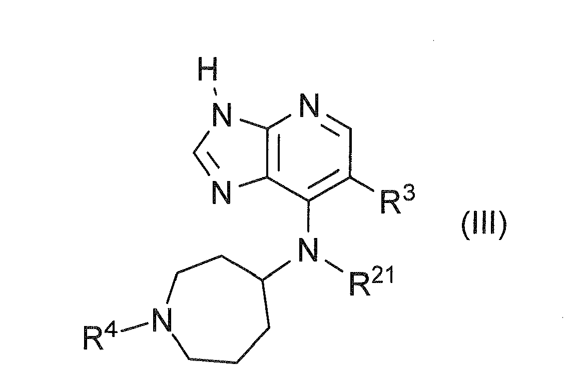 wherein
R³ is carbamoyl or oxadiazolyl,
each of which may be substituted with C₁-C₆ alkyl;
R²¹ is -H or may be bonded with R³ via a certain functional group to form divalent groups represented by the following formula (IA): 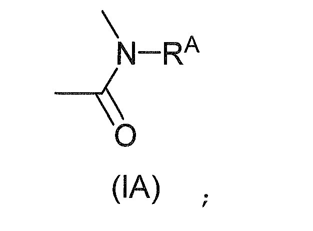
R^{A} is -H or C₁-C₆ alkyl;
R⁴ is aryl, 5- to 6- membered heteroaryl, benzyl, carbamoyl, -(C=O)-(C₁-C₆ alkyl), -C(=O)-(5- to 6- membered heteroaryl), -C(=O)-(5- to 6- membered nitrogen-containing heterocycloalkyl), -C(=O)-carbamoyl, -C(=O)C(=O)-(5- to 6- membered nitrogen-containing heterocycloalkyl), -C(=O)O-(C₁-C₆ alkyl), each of which may be substituted with one or more identical or different group (s) selected from the group consisting of R⁴¹_{;} and
R⁴¹ is halogen, -CN, -CF₃, -OH, =O, -NO₂, carbamoyl, oxime, C₁-C₆ alkyl, -(C₁-C₆ alkyl)-CN, -(C₁-C₆ alkyl)-OH, -C(=O)O-(C₁-C₆ alkyl), -O-(C₁-C₆ alkyl), -O-(C₁-C₆ alkyl)-CN, 2-pyrroridinone-1-yl or phenyl,
wherein phenyl may be substituted with halogen; or pharmaceutically acceptable salts thereof, or prodrug thereof.

8. A compound of claim 7, wherein:
R³ is -CONH₂ or -C(=O)NH-(C₁-C₆ alkyl) ;
or pharmaceutically acceptable salts thereof, or prodrug thereof.

9. A compound of claim 8, which is
7-{[1-(5-Cyanopyridin-2-yl)azepan-4-yl]amino}-3H-imidazo[4,5-b]pyridine-6-carboxamide,
or pharmaceutically acceptable salts thereof, or prodrug thereof.

10. A compound of claim 1 having the following formula (IV): 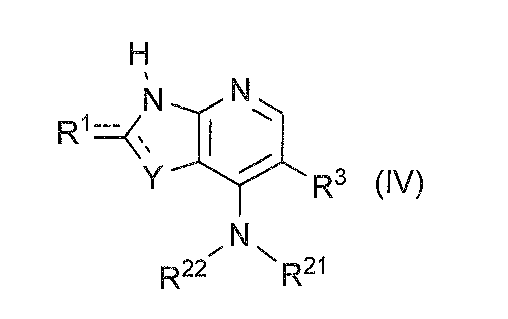 wherein
R¹ is -H or =O;
R²¹ is bonded with R³ via a certain functional group to form divalent groups represented by the following formula (IB), (IC) or (ID) : 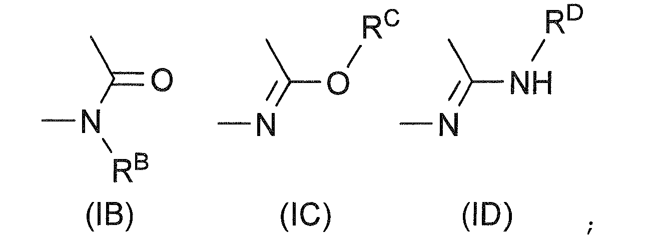
R^{B} is -H or C₁-C₆ alkyl;
R^{C} is -H, C₁-C₆ alkyl or (C₁-C₆ alkyl)-O-(C₁-C₆ alkyl);
R^{D} is -H, -C(=O)-(C₁-C₆ alkyl), -C(=O)-(C₁-C₆ alkyl)-O-(C₁-C₆ alkyl) , -C(=O)C(=O)NH-(C₁-C₆ alkyl), -C(=O)C(=O)NH-(C₁-C₆ alkyl)-O-(C₁-C₆ alkyl) or -C(=O)O-(alkyl);
R²² is 5- to 7-membered nitrogen-containing heterocycloalkyl, C₃-C₉ cycloalkyl, or benzyl,
each of which may be substituted with one or more identical or different group(s) selected from the group consisting of R^{V1};
R^{V1} is halogen, C₁-C₆ alkyl, -O-(C₁-C₆ alkyl), aryl, 5- to 6- membered heteroaryl, -(C=O)- (C₁-C₆ alkyl), -(C=O)-(5- to 6- membered nitrogen-containing heterocycloalkyl), -(C=O)O- (C₁-C₆ alkyl), or carbamoyl, each of which may be substituted with one or more identical or different group (s) selected from the group consisting of R^{W1};
R^{W1} is halogen, -CN, -CF₃, -OH, =O, -NO₂, carbamoyl, oxime, C₁-C₆ alkyl, -(C₁-C₆ alkyl)-CN, -(C₁-C₆ alkyl)-OH, -C(=O)O-(C₁-C₆ alkyl), -O-(C₁-C₆ alkyl), -O-(C₁-C₆ alkyl)-CN, 2-pyrroridinone-1-yl or phenyl, wherein phenyl may be substituted with halogen;
X is N, CH or CH₂; and
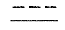 is a single bond or a double bond,
wherein the one is single bond, the other is double bond; or pharmaceutically acceptable salts thereof, or prodrug thereof.

11. A compound of claim 10, wherein
Y is CH;
R¹ is -H;
R²¹ and R³ form a group of represented by the formula (ID);
R^{D} is -H or -C(=O) C (=O)NH- (C₁-C₆ alkyl) ; and
R²² is 5- to 7-membered nitrogen-containing heterocycloalkyl or C₃-C₉ cycloalkyl,
each of which may be substituted with one or more identical or different group(s) selected from the group consisting of R^{V1};
or pharmaceutically acceptable salts thereof, or prodrug thereof.

12. A compound of claim 11,
which is selected from the group consisting of:
(1) rel-(2R,4R)-4-fluoro-1-{[(3S,4S)-4-methyl-3-(2-oxo-3,6-dihydroimidazo[4,5-d]pyrrolo[2,3-b]pyridin-1(2H)-yl)piperidin-1-yl]carbonyl}pyrrolidine-2-carbonitrile,
(2)rel-3-[(3R,4R)-3-(3-aminopyrazolo[3,4-d]pyrrolo[2,3-b]pyridin-1(6H)-yl)-4-methylpiperidin-1-yl]-3-oxopropanenitrile,
(3)rel-3-[(3R,4R)-4-methyl-3-(3-oxo-3,6-dihydropyrazolo[3,4-d]pyrrolo[2,3-b]pyridin-1(2H)-yl)piperidin-1-yl]-3-oxopropanenitrile,
(4)rel-N-{1-[(3R,4R)-1-(cyanoacetyl)-4-methylpiperidin-3-yl]-1,6-dihydropyrazolo[3,4-d]pyrrolo[2,3-b]pyridin-3-yl}-N'-(cyclopropylmethyl)ethanediamide,
(5)rel-6-[(3R,4R)-3-(3-aminopyrazolo[3,4-d]pyrrolo[2,3-b]pyridin-1(6H)-yl)-4-methylpiperidin-1-yl]pyridazine-3-carbonitrile,
(6)rel-N-{1-[(3R,4R)-1-(5-cyanopyridin-2-yl)-4-methylpiperidin-3-yl]-1,6-dihydropyrazolo[3,4-d]pyrrolo[2,3-b]pyridin-3-yl}-N'-(cyclopropylmethyl)ethanediamide,
(7)rel-N-(1-{(3R,4R)-1-[(cyanomethyl)(methyl)carbamoyl]-4-methylpiperidin-3-y1}-1,6-dihydropyrazolo[3,4-d]pyrrolo[2,3-b]pyridin-3-yl)-N'-isopropylethanediamide, and
(8)rel-N-(1-{(3R,4R)-1-[(cyanomethyl)(methyl)carbamoyl]-4-methylpiperidin-3-yl}-1,6-dihydropyrazolo[3,4-d]pyrrolo[2,3-b]pyridin-3-yl)-N'-(cyclopropylmethyl)ethanediamide, or pharmaceutically acceptable salts thereof, or prodrug thereof.

13. The compound according to any one of claims 1 to 12, said compound being used as a medicament.

14. The compound according to any one of claims 1 to 12, said compound being used to treat and/or prevent diseases including rejection during organ/tissue transplantation, autoimmune diseases, multiple sclerosis, rheumatoid arthritis, psoriasis, asthma, atopy, tumors, plasmacytic myeloma and leukemia in human beings or animals.

15. A medicament containing the compound according to any one of claims 1 to 12 as an active ingredient.

16. A medicament containing the compound according to any one of claims 1 to 12 as an active ingredient, with a pharmaceutically acceptable carrier or excipient.

17. A janus kinase 3 (JAK3) inhibitor comprising the compound according to any one of claims 1 to 12.

18. A method for treating and/or preventing diseases including rejection during organ/tissue transplantation, autoimmune diseases, multiple sclerosis, rheumatoid arthritis, psoriasis, asthma, atopy, tumors, plasmacytic myeloma and leukemia in human beings or animals, said method comprising administration of the compound according to any one of claims 1 to 12 to human beings or animals.

19. A use of the compound according to any one of claims 1 to 12 to treat and/or prevent diseases including rejection during organ/tissue transplantation, autoimmune diseases, multiple sclerosis, rheumatoid arthritis, psoriasis, asthma, atopy, tumors, plasmacytic myeloma and leukemia in human beings or animals.

20. A product comprising a pharmaceutical composition containing the compound according to any one of claims 1 to 12 and a written notice regarding the pharmaceutical composition, said written notice stating that the compound (I) can be or should be used to treat and/or prevent diseases including rejection during organ/tissue transplantation, autoimmune diseases, multiple sclerosis, rheumatoid arthritis, psoriasis, asthma, atopy, tumors, plasmacytic myeloma and leukemia in human beings or animals.
